(19) Europäisches Patentamt
European Patent Office
Office européen des brevets

(11) **EP 1 271 386 B1**

(12) **EUROPEAN PATENT SPECIFICATION**

(45) Date of publication and mention of the grant of the patent:
**10.08.2005 Bulletin 2005/32**

(51) Int Cl.⁷: **G06F 19/00**

(21) Application number: **02254358.1**

(22) Date of filing: **21.06.2002**

(54) **Apparatus and process for assessing medical facilities**

Vorrichtung und Verfahren zur Auswertung der medizinischen Einrichtungen

Dispositif et procédé d'évaluation des services médicaux

(84) Designated Contracting States:
**AT BE CH CY DE DK ES FI FR GB GR IE IT LI LU MC NL PT SE TR**

(30) Priority: **22.06.2001 US 887220**

(43) Date of publication of application:
**02.01.2003 Bulletin 2003/01**

(73) Proprietor: **Gambro Lundia AB**
**22 643 Lund (SE)**

(72) Inventors:
- **Bosche, Juan P. M.D.**
  **NW, Washington D.C. 20008 (US)**
- **Hegbrant, Maria Alquist M.D.**
  **237 91 Bjarred (SE)**

(74) Representative: **Barlow, Roy James et al**
**J.A. KEMP & CO.**
**14, South Square**
**Gray's Inn**
**London WC1R 5JJ (GB)**

(56) References cited:
**US-A- 5 365 425**
**US-A- 5 652 842**
**US-A- 5 706 441**
**US-A- 5 724 379**
**US-A1- 2001 032 195**
**US-B1- 6 223 164**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to an apparatus and process for analysing data representing technical measurements in order to assess the operations of medical facilities, such as facilities providing blood purification services such as dialysis.

US patent 5, 364, 425 describes a method and a system for evaluating effectiveness of service among a set of treatment facilities by modeling the factors of quality, cost and access in such a manner as to provide a holistic description of the effectiveness of medical treatment data from a variety of computerized databases and incorporating patient perceptions of medical care through the use of surveys. The method comprises gathering data quantifying quality, cost and access performance characteristics of each of the treatment facilities; displaying the quality, cost and access performance characteristics simultaneously on a graph (three dimensional cube using a separate axis for the quality, cost and access performance characteristics) to indicate strong and weak quality, cost and access performance characteristics; and identifying strong and weak quality, cost and access performance characteristics of each of the treatment facilities.

US patent 5, 724, 379 describes a method for comparing health care services from different providers with a computer-aided system using outpatient an inpatient claims data, bases containing indicators of clinical conditions, such as age, gender, diagnoses, and procedures used, and including comparison criteria such as indicia of quality. The diseases of the patients are grouped by clinical complexity, and the extent of the systematic relationships between the clinical complexity groups and the comparison criteria are analyzed, preferably by regression analysis.

Many people require replacement or supplementation of their natural renal function in order to remove excess fluid or fluids containing dissolved waste products from their blood for various reasons, including illness, injury or surgery. Several procedures known for this purpose are dialysis, hemodialysis, hemofiltration, hemodiafiltration ultrafiltration; and plasmapherisis. The specific procedure employed depends upon the needs of the particular patient. For example, dialysis is used to remove soluble waste and solvent from blood; hemofiltration is used to remove plasma water from blood; hemodiafiltration is used to remove both unwanted solute (soluble waste) and plasma water from blood; ultrafiltration is a variation of hemofiltration; and plasmapherisis is used to remove blood plasma by means of a plasmapherisis filter. Because the replacement of renal function may affect nutrition, erythropoiesis, calcium-phosphorus balance and solvent and solute clearance from the patient, it is beneficial if the procedure is controlled specifically for the particular patient's needs. The accurate control of the rate of removal of intravascular fluid volume is also useful to maintain proper fluid balance in the patient and minimize hypertension and hypotension.

Because hemodialysis should be performed frequently, it is important to keep the cost of each treatment as low as possible. Further, it is desirable to minimize the amount of blood outside a patient's body in the extracorporeal circuit, thereby minimizing the stress on the patient as well as minimizing the potential for trauma to the blood. Bubble traps typically retain a relatively large volume of blood during normal operation. Blood trauma also can occur at an air-blood interface such as are found in typical bubble traps and drip chambers. It is advantageous to reduce the need to add anticoagulant to the blood, and decrease the physical stress on the patient.

In dialysis clinics for hemodialysis, large numbers of patients are treated simultaneously. Each patient is connected to a dialysis machine comprising a monitor, which prepares the dialysis solution and administers the solution to a dialyzer, which is connected to the patient.

In the past, setup, monitoring and adjusting of the process and machine have been labor intensive. Assembling and priming of the extracorporeal blood treatment apparatus, can be especially time consuming. Manual systems are labor intensive because of the need for personnel frequently to monitor the patient and equipment, as well as to adjust fluid flow rates based upon visual observation. During operation of some extracorporeal blood treatment machines, operators can only adjust the rate of removal of body fluid from the secondary chamber by raising or lowering the height of a container collecting the matter from the secondary chamber. Changing the height of the collection container effectively modifies the pressure across the semipermeable membrane, increasing or decreasing the rate at which body fluid passes from the blood across the membrane. To maintain the rates, the height of the collection container requires frequent monitoring so that appropriate adjustments can be made. The connection of some manual extracorporeal treatment systems to patients can also be labor intensive. An arterial catheter connection should also be monitored, as it is susceptible to disconnection and any such disconnection can result in significant blood loss.

In the real world, the quality, effectiveness, efficiency and cost of treatments are not absolutely identical but vary somewhat per treatment for an individual patient, as well as for all the patients in the same clinic or other medical facility. These variations are attributable to a number of technical factors, e.g. different conditions and needs of the human body of the patient on different days, different moods and physical conditions for medical personnel administering the treatment, different medical personnel, different temperatures, different humidity, different equipment, different compositions of dialysis fluids, varying costs in supplies and equipment, changing overhead costs, such as for electricity, heat, air conditioning, rent, etc. These variations also differ from medical facility to medical facility depending on the size, location, staff and equipment at the medical facilities.

Further, these technical parameters can be adjusted in most cases. It would therefore be desirable to provide a computer implemented method and an apparatus for carrying out this method to determine how the technical parameters should be adjusted in order to move closer to a predetermined goal, e.g. increased profit or happier patients.

A helpful process is provided for improving operations of clinics and other medical facilities to enhance care and treatment of patients. Advantageously, the process has achieved unexpected surprisingly good results. While the process can be used with many types of treatment, it is particularly useful for extracorporeal blood treatment for patients requiring blood purification, such as hemodialysis, dialysis, ultrafiltration, hemofiltration, hemodiafiltration, plasmapherisis, and/or apherisis. Furthermore, while the treatment (Rx) can be administered in the home of the patient, the process is particularly beneficial when the treatment is administered at one or more medical treatment facilities, such as at clinics, hospitals, centers for medical treatment, patient treatment facilities of health care providers, and/or doctor's offices (i.e. offices of physicians).

In order to improve operations at the clinics and other medical facilities and enhance the care and treatment of patients being administered the treatment, a set (series or array) of factors are selected which comprise criteria and variables affecting the performance of the treatment of the patients in the medical treatment facilities. Such factors can comprise: the effectiveness of each treatment per patient, the efficiency of each treatment per patient, the frequency of the treatments for each patient, costs of each treatment per patient, the total costs of the treatments for each patient, variations of effectiveness of the treatments for each patient, variations of efficiency of the treatments for each patient, variations of costs of the treatments for each patient, demographics of each facility including the geographical location of each facility, metropolitan statistical area of each facility defining an urban MSA, ownership of each facility, type of ownership of each facility including company owned and joint venture facilities, length of service of each facility, hospitalization of patients, division, employee turnover (T/O) at each facility, type of treatment per patient, duration (months) of treatments per patient, percentage of patients having the treatment as a primary cure for their ailments, race of patients, ethnic background of patients, hemoglobin per patient, albumen per patient, catheter usage per patient, equipment usage per patient, noncompliance per patient, iron supplement usage per patient, epogen usage per patient, crude mortality rate (CMR) of patients in the facility, average months on dialysis (MOD) per patient, temperature conditions during treatment, humidity conditions during treatment, type of equipment and supplies, composition of dialysis fluids, modified charleson comorbidity index (MCCI), costs of equipment and supplies per treatment, dialyzer costs per treatment, costs for sterilizing dialysis equipment for the treatments, savings and costs for reuse of equipment for the treatment, labor costs per treatment, overhead per facility, percentage of patients covered by commercial insurance, reimbursement of medicare for the treatments, reimbursements from a government agency for the treatment, and reimbursement from insurance companies for the treatments.

In the process, the data comprising the set of factors are inputted into a central processing unit (CPU), such as a: microprocessor, computer, main frame computer, server computer, desktop computer, workstation computer, laptop computer, notebook computer, palm pilot-type computer, computer chip, integrated circuit, electronic controller, network, internet, or global communications network.

Advantageously, the data can be statistically analyzed and compared with the CPU to compute a sigma comprising a standard deviation around a mean of the data to determine the performance of the process. Desirably, the financial results of the process for treatments of the patients at the facilities can be electronically calculated with the CPU. Such financial results can include: earnings, operating income, gross income, net income, gross margin, net margin, profits, EBITA comprising earnings before interest, taxes and amortization and EBITDA comprising earnings before interest, taxes, depreciation and amortization.

In one preferred process, the effectiveness and efficiency of each treatment per patient are measured. The frequency of treatments for each patient and the costs per treatment per patient are determined. Also, the total costs of the treatments for each patient are calculated, as well as the variations of effectiveness and efficiency of the treatments for each patient. The demographics of each facility are identified, such as the geographical location of each facility. Data concerning the preceding are then entered (inputted) and stored in the CPU and statistically analyzed with the CPU to compute a sigma comprising a standard deviation around a mean of the data to determine the performance of the process. Supplemental data, such as facility data, patient data, and other cost data, as described above, can also be entered into the CPU. The preceding data, analysis, correlations and comparisons can be retrieved from the CPU.

Preferably, the process is operated and performed at about one sigma. It was unexpectedly and surprisingly found that costs, such as labor costs for more time for patient monitoring and attentiveness by medical personnel with appropriate equipment to achieve enhanced patient care also attains greater profit, EBITA and EBITDA for the process.

In the preferred treatment to which the process relates, blood is removed from the patient; the removed blood is treated externally to the patient by removing matter or molecules from the blood, and the treated blood is returned to the patient. Most preferably, the treatment comprises hemodialysis or dialysis. The effectiveness of each treatment can be measured according to the mathematical model KT/V wherein K = clearance, T = time of the treatment, and V = body distribution volume of urea or creatine. Advantageously, the effectiveness of the treatments can be statistically analyzed with the CPU to calculate a standard deviation for KT/V for each patient, a standard deviation for inter patient KT/V, and/or a standard deviation for intra patient KT/V standard deviation, in order to help determine the effectiveness of the treatment.

Specifically, each patient can be treated by: preparing the patient for the treatment; preparing a dialysis fluid for the patient; injecting an injector consisting of a needle or a catheter into the patient; removing blood from the patient through the needle or catheter via tubing connected to a monitor (the monitor comprising a dialysis machine with a dialyzer cartridge having a filter) (the catheter can be a multi-use or single use catheter); passing the removed blood through a semipermeable membrane; pumping the dialyzer fluid from the monitor through the semipermeable membrane; and returning the treated blood which has passed through the semipermeable membrane to the patient via the needle or catheter and the tubing. The treatment can be monitored by the monitor (dialysis machine) and/or medical personnel. After the treated blood is returned to the patient, the needle or catheter is, or may be, removed from the patient by medical personnel. Thereafter, the monitor can be cleaned by disinfecting, sterilizing and/or sanitized with heat or a chemical disinfectant. The used tubing and injector comprising the used needle or used catheter are normally discarded (disposed) in compliance with governmental regulations. The used cartridge can also be discarded in an environmentally and medically safe manner as required by U.S. regulations or the used cartridge can be cleaned (disinfected, sterilized or sanitized) for reuse, such as is customary in Europe.

The invention will be further described by way of example only, with reference to the accompanying drawings, in which:-

Figure 1 is a graph comprising a histogram of the Kt/V distribution count for three months for patients in clinics in France;

Figure 2 is a graph comprising a histogram of the Kt/V distribution count for three months for patients in a clinic located in the USA;

Figure 3 is a graph comprising a histogram of the Kt/V distribution count for three months for patients in clinics in Bari, Italy;

Figure 4 is a graph comprising a histogram of the Kt/V distribution count for three months for patients in clinics in Spain;

Figure 5 is a graph comprising a histogram of the Kt/V distribution count for three months for patients in clinics in Hungary;

Figure 6 is a graph of the overall clinic standard deviation (SD) for delivering treatments as specified;

Figure 7 is a graph of inter patient variation;

Figure 8 is a graph of intra patient variation;

Figure 9 is a graph of statistical analysis a graph of statistical analysis for patients and clinics for patients and clinics in Spain;

Figure 10 is a graph of statistical analysis a graph of statistical analysis for patients and clinics for patients and clinics in Hungary;

Figure 11 is a graph of statistical analysis a graph of statistical analysis for patients and clinics for patients and clinics in Bari, Italy;

Figure 12 is a graph of statistical analysis a graph of statistical analysis for patients and clinics for patients at a clinic in the USA;

Figure 13 is a graph of statistical analysis at a sigma level of three for Kt/V for patients at clinics in the USA;

Figure 14 is a graph of the percentage of treatment variations per clinic per country;

Figure 15 is a graph of the graph of the operational level and variations (defects) per 100 treatments (Rxs);

Figure 16 is a graph of the percentage EBITDA to the overall clinic standard deviation (SD) for patients and clinics in Argentina;

Figure 17 is a graph of the operating income to the overall clinic standard deviation (SD) for patients and clinics in Argentina;

Figure 18 is a graph of the gross margin to the overall clinic standard deviation (SD) for patients and clinics in Argentina;

Figure 19 is a graph of the percentage EBITDA to the overall clinic standard deviation (SD) for patients and clinics in Spain;

Figure 20 is a graph of the operating income to the overall clinic standard deviation (SD) for patients and clinics in Spain;

Figure 21 is a graph of the gross margin to the overall clinic standard deviation (SD) for patients and clinics in Spain;

Figure 22 is a graph of the percentage EBITDA to the overall clinic standard deviation (SD) for patients and clinics in Italy;

Figure 23 is a graph of the operating income to the overall clinic standard deviation (SD) for patients and clinics in Italy;

Figure 24 is a graph of the percentage EBITDA to the overall clinic standard deviation (SD) for patients and clinics in Argentina, Spain, Italy, Portugal and Hungary;
Figure 25 is a graph of the operating income to the overall clinic standard deviation (SD) for patients and clinics in Argentina, Spain, Italy and Portugal;
Figure 26 is a graph of the gross margin to the overall clinic standard deviation (SD) for patients and clinics in Argentina, Spain, Italy and Portugal;
Figure 27 is a graph of the percentage EBITDA to the overall clinic standard deviation (SD) including reimbursement for patients and clinics in the USA;
Figure 28 is a graph of the percentage EBITDA to the overall clinic standard deviation (SD) for patients in 41 clinics in the world and for 22 clinics in the USA;
Figure 29 is a graph of the total process time;
Figure 30 is a graph of the treatment step time;
Figure 31 is a graph of the disinfection step time;
Figure 32 is a graph illustrating the process, treatment and other steps time;
Figure 33 is a graph of the total process staff time;
Figure 34 is a graph of the staff treatment step time;
Figure 35 is a graph illustrating the total process, treatment and other steps time;
Figure 36 is a graph illustrating the median and ideal staff times;
Figure 37 is a graph of the total process time for patients and clinics in the USA when dialyzers are reused ;
Figure 38 is a graph comparing the total process time for patients and clinics in which new dialyzers are used (no reuse) versus those clinics in the USA when used dialyzers are reused;
Figure 39 is a graph comparing the medium staff time for patients and clinics in the USA in which new dialyzers are used (no reuse) versus those clinics in the USA when used dialyzers are reused;
Figure 40 is another graph comparing the medium staff time for patients and clinics in the USA in which new dialyzers are used (no reuse) versus those clinics in the USA when used dialyzers are reused;
Figure 41 is graph comparing the shortest total process staff times for patients and clinics in the USA in which new dialyzers are used (no reuse) versus those clinics in the USA when used dialyzers are reused;
Figure 42 is process flow chart mapping treatment procedures;
Figure 43 is a flow charting mapping variables for the process; and
Figure 44 is a histogram and box and whisker plot of EBITDA per treatment for patients in clinics in the USA.

In order to set the invention in context, a description will now be given of the typical blood treatment process that is carried out in the medical facilities. This description will make it apparent as to how the technical parameters manipulated in the apparatus of the present invention are related to the blood treatment process.

Extracorporeal blood treatment comprises removing blood from a patient, treating the blood externally to the patient, and returning the treated blood to the patient. Extracorporeal blood treatment is typically used to extract undesirable matter or molecules from the patient's blood and/or add desirable matter or molecules to the blood. Extracorporeal blood treatment is used with patients unable to effectively remove matter from their blood, such as when a patient has suffered temporary or permanent kidney failure. These patients and other patients may undergo extracorporeal blood treatment to add or remove matter to their blood to maintain an acid/base balance or to remove excess body fluids, for example.

Extracorporeal blood treatment is typically accomplished by removing the blood from the patient in a continuous flow, introducing the blood into a primary chamber of a filtration unit where the blood is allowed to flow past a semipermeable membrane. The semipermeable membrane selectively allows matter in the blood to cross the membrane from the primary chamber into a secondary chamber and also selectively allows matter in the secondary chamber to cross the membrane into the blood in the primary chamber, depending on the type of treatment.

A number of different types of extracorporeal blood treatments can be performed. In ultrafiltration (UF) treatment, undesirable matter is removed from the blood by convection across the membrane into the secondary chamber. In a hemofiltration (HF) treatment, the blood flows past the semipermeable membrane as in UP and desirable matter is added to the blood, typically by dispensing a fluid into the treated blood either before or after it passes through the filtration unit and before it is returned to the patient. In a hemodialysis (HD) treatment, a secondary fluid containing desirable matter is introduced into the secondary chamber of the filtration unit. Undesirable matter from the blood crosses the semipermeable membrane into the secondary fluid and desirable matter from the secondary fluid may cross the membrane into the blood. In a hemodiafiltration (HDF) treatment, blood and secondary fluid exchange matter as in HD, and, in addition, matter is added to the blood, typically by dispensing a fluid into the treated blood before its return to the patient as in HF. To perform one of these extracorporeal blood treatments, blood should usually be continuously removed from either a vein or artery of the patient.

Each type of extracorporeal blood treatment can been conducted with a separate system because of the unique combination of fluids, flow rates, pressures and other parameters associated with each of the treatments. So, for example, manual systems used to perform HD on arterial blood rely on the arterial blood pressure to cause blood to flow past the membrane and be treated. Because a natural flow cannot be achieved when using venous blood, these systems cannot perform HD on venous blood and a separate machine or pump is required to establish a blood flow from a venous blood source and cause the venous blood to pass through the filtration unit and return the treated blood to a venous return point. Extracorporeal blood treatment systems can include monitoring the treatment to detect operational conditions placing the patient at risk. Such conditions include leaking of blood at connection points of the blood flow lines to and from the extracorporeal blood treatment machine, clotting of blood in the semipermeable membrane, depletion of fluids in the containers of matter required for treatment, filling of the containers collecting matter during treatment, and existence of dangerously high or low pressures of blood or fluids during the treatment.

The purpose of dialysis is to replace the excretory function of the kidneys by artificial means to remove excess fluid and unwanted solutes from the body. During a hemodialysis (HD) treatment, the patient's blood is circulated outside the body through an artificial kidney, the dialyzer. In principle, a dialyzer contains two chambers separated by a membrane, one of them perfused by the blood and other by a special dialysis fluid. The membrane is semipermeable, thereby permitting the passage of water and solutes up to a certain size. The extracorporeal circulation is controlled by a dialysis machine, which also prepares the dialysis fluid.

When hemodialysis treatment starts, the patient's blood contains excess fluid and waste products. To remove the fluid, a pressure gradient is applied across the membrane in the dialyzer. This forces water to leave the blood, penetrate the membrane and enter the dialysis fluid by the process of ultrafiltration. The amount of fluid ultrafiltered during the entire treatment session should correspond to excess volume. As the dialysis fluid is free from waste products, a concentration gradient is created across the membrane. This makes the waste products move by diffusion from the blood, through the membrane and into the dialysis fluid. The result of hemodialysis treatment is that the volume of the blood is adjusted and that waste products are removed from it. The two processes of fluid removal (ultrafiltration) and solute removal (diffusion) normally occur simultaneously. However, since they are controlled by different parameters we are describing them separately.

In hemodialysis, solutes are removed from blood mainly by diffusion. The driving force for the diffusive transport of the solutes across the dialysis membrane is the concentration gradient between blood and dialysis fluid. The blood flow ($Q_B$) brings waste products into the dialyzer, and the dialysis fluid flow ($Q_o$) carries them away. By this continuous transport of solutes to and from the dialyzer, a large concentration gradient is maintained across the membrane. The larger the concentration gradient, the faster the removal of waste products from the blood. Blood and dialysis fluid normally flow in opposite directions (counter-current flow). This is the most efficient way to create a continuous concentration gradient along the whole length of the dialyzer.

Small solutes with a molecular weight below 300, such as the waste products urea (MW 60) and creatinine (MW 113), easily move across the dialysis membrane. Since the membrane offers little resistance to these solutes, increasing the flow rates directly increases their transport across the membrane. Higher blood flow brings more solutes to the membrane surface, and higher dialysis fluid flow carries them away faster. The removal of small solutes is mainly flow-dependent. The smaller the solute, the greater is the impact of the flow rate. Larger solutes do not diffuse across the membrane as easily. Since the main resistance to transfer of these solutes is in the membrane, increasing the flow rates has little effect. The removal of larger solutes is also mainly membrane-dependent. The larger the solute, the greater is the impact of the membrane. In general, the thinner the membrane, the less resistance it offers.

As discussed above, the solute removal rate by diffusion in hemodialysis is controlled by: blood flow rate, $Q_B$; dialysis fluid flow rate, $Q_o$; concentration gradient between blood and dialysis fluid; and dialyzer characteristics, such as membrane type, thickness and surface area.

The clearance (K) of a substance is the volume of blood from which the substance is completely removed per unit of time (normally in ml/min). K = excretion rate/blood concentration. Clearance is most easily measured in the dialysis unit at an ultrafiltration rate of zero. Under this condition, the incoming blood flow ($Q_{Bin}$) is the same as the outgoing blood flow ($Q_{Bout}$). The clearance formula can be simplified.

$$K = \frac{Q_{Bin} \text{ x } (C_{Bin} - C_{Bout})}{C_{Bin}}$$

To measure the clearance of a dialyzer for a certain substance, e.g. creatinine, we need to know the blood flow and the creatinine concentration in the blood circuit before and after the dialyzer. The blood flow can be read from the machine display (monitor). The creatinine concentration is analysed from blood samples taken from the blood lines before and after the dialyzer.

The term dialyzer is commonly used rather than "artificial kidney". As previously indicated, the dialyzer is a device through which blood and dialysis fluid flow, separated by a semipermeable membrane. Some dialyzers are so small that they can be it held in a hand. Basic types of dialyzers include: the plate and the hollow fiber dialysis.

The most essential quality of the dialyzer is the performance, i.e. the efficiency with which it purifies the blood. A further concern is its compatibility, i.e. that the contact between the blood and the foreign materials of the dialyzer does not evoke any clinically important adverse reactions.

For the removal of small solutes from blood, e.g. urea and creatinine, diffusion is by far the most efficient transport principle. With increasing size, however, molecules move more slowly and hence the diffusive transport is reduced. For solutes with a molecular weight of several thousand, convective transport is more important than diffusion. When solute removal is quantified as clearance, the combined effects of diffusion and convection are measured. To describe the solute removal properties of a dialyzer, we often use the following substances to measure their clearance at different blood flow rates and constant dialysis fluid flow rate, normally 500 ml/min. The clearance figures can be measured using an aqueous test solution in the blood circuit. In the clinical situation with blood in the dialyzer the clearance is somewhat lower.

Urea (MW 60), is an end product of protein metabolism. Creatinine (MW 113) is a breakdown product of muscle metabolism. Urea and creatinine are small molecules which easily diffuse across the membrane. Accordingly their clearance is high. Their removal is markedly flow dependent, i.e. it increases greatly with increased blood flow rate.

Phosphate (MW 96-97) is an important substance in the body, but in uremia it accumulates and the excess must be removed. It is a small solute but in dialysis it behaves like a larger one. This is because it attracts water and binds to proteins, forming large aggregates that do not easily pass the membrane. Consequently, the clearance value is lower than could be expected considering the molecular weight alone.

Vitamin $B_{12}$ (MW 1 355) is not a uremic toxin and therefore not really important to remove. It is, however, used as a market for so-called "middle molecules", a non-specific range of solutes of intermediate molecular weight suspected of including uremic toxins. Removing molecules of the size of vitamin $B_{12}$ is a membrane dependent process and an increase in blood flow rate only marginally affects the removal. To increase the removal of such molecules, more permeable membranes, larger surface area and/or convective transport is needed.

Inulin (MW ca 5 000) is a synthetic carbohydrate sometimes used as a marker for larger solutes. One solute of particular interest in renal therapy is a protein known as $B_2$ microglobulin, $B_2$m (MW 11 800). It accumulates in the body in renal failure, a process which may eventually lead to dialysis-related amyloidosis. For most low-flux membranes, the $B_2$m permeability is practically zero which results in no removal by dialysis. High-flux membranes are mostly more permeable to large solutes and are thereby capable of removing $B_2$m. For such a large solute, the transport across the membrane is mainly convective. The amount removed then depends on the sieving coefficient of the membrane and the ultrafiltered volume. For some membranes, adsorption is also an important mechanism for $B_2$m removal.

The internal parts of a dialyzer are in direct contact with the blood. It is important that the dialyer is sterile, i. e., that it contains no living microorganisms. A common way to sterilize medical disposables is by using the bactericidal gas ethylene oxide, EtO. This method is considered safe and economical. The environmental problems of EtO have been solved by the use of a mixture of 10% EtO in carbon dioxide, which, after use, is transformed into harmless waste in a cleaning process. EtO gas is able to penetrate all areas of the dialyzer, even if it is packaged before sterilization. Afterwards, it is placed in quarantine for a certain period of time, normally 1-2 weeks, during which deaeration takes place. It has been shown that despite the deaeration, some EtO residuals may still be left in the dialyzer for a long time, mainly in the potting material (polyurethane) of hollow fiber dialyers. In a sensitised patient, the small amount of EtO that may escape from the dialyzer into the blood during the treatment can be enough to cause an allergic reaction. For plate dialyzers, the risk of such EtO associated hypersensitivity is considerably lower, as they contain no potting material and thus retain less EtO. Hypersensitivity reactions toward EtO sterilized materials are very rare.

Sterilization by gamma radiation is also easily performed, even for pre-packed dialyzers. However, high energy of the radiation has been reported to induce the formation of reactive chemical species or to cause breakdown of polymer materials. To minimize these effects, the dialyzer is often filled with water before gamma sterilization.

Steam sterilization (autoclaving) is performed at high temperature (>121°C) and high pressure. Since no chemicals are employed, this process is non-toxic and permits immediate product release. It is considered to be more complicated and expensive than EtO sterilization. Many membranes and dialyzer materials are not resistant to high temperatures. Thus, steam sterilization may destroy them or modify their performance.

In a typical dialysis unit, the majority of beds are occupied by chronic patients. The organization is based upon a weekly schedule where the same patients are treated three times a week, e.g. Monday-Wednesday-Friday or Tuesday-Thursday-Saturday. A treatment normally takes between 3.5 and 5 hours. Accordingly, a clinic can perform two or even three shifts a day. In most hospitals, the dialysis unit is part of the clinic of nephrology. Independent centers can be located away from the large hospitals. A physician (doctor) has the medical responsibility and prescribes the treatment for the patient, as well as follows up the clinical outcome. A supervising nurse can have responsibility for management duties at the clinic. The delivery of the treatment can be carried out by nurses under supervision of a physician.

The dialysis machine (monitor) is normally in standby mode after a disinfection procedure. When the monitor is first activated and connected to the dialysis fluid, the dialysis fluid is allowed to flow through the fluid circuit to steady state conditions in order to reach stable conductivity and temperature. The dialyzer and the blood lines are then attached to the machine. The arterial end of the blood line is connected to a bag of saline solution which can be mounted on an infusion pole. The venous end of the blood line can be connected to a waste bag. Filling and rinsing of the dialyzer and blood lines can occur next, which is sometimes referred to as the "priming" procedure. The fluid circuit is also connect to the dialyzer for counter-current flow. This can be done before or after the initiation of the priming, depending on the instructions for the specific dialyzer used and the routines of the clinic. During this procedure the blood lines and the dialyzer are filled with saline, followed by a rinse with 1000-1500 ml of additional saline to remove air and residuals. Air bubbles can induce clotting and also obstruct the blood path in the dialyzer which would result in reduced surface area. Residuals are small particles e.g. from the polymer materials, glycerol which can be used as a stabilizer in the membrane or EtO from the sterilizing process. When the dialyzer and the dialysis machine (monitor) are ready for the patient, the system can be in a ready mode with saline in the blood lines and dialysis fluid flowing through the fluid circuit.

Preparation of the dialysis solution can be accomplished by mixing two dialysis concentrates to produce a desired composition and concentration. The dialysis concentrate can be an A-concentrate, which consists of acetic acid, sodium chloride, potassium chloride, calcium chloride and magnesium chloride, and a B-concentrate, consisting of bicarbonate. These concentrates are diluted with water. The water should be sterile or treated so that it contains as few impurities as possible, and can be prepared by the so-called RO (reverse osmosis) process.

One type of extracorporeal blood processing is an apheresis procedure in which blood is removed from a donor or patient, directed to a blood component separation device (e.g., centrifuge), and separated into various blood component types (e.g., red blood cells, white blood cells, platelets, plasma) for collection or therapeutic purposes. One or more of these blood component types are collected (e.g., for therapeutic purposes), while the remainder are returned to the donor or patient.

There now follows a description of the factors that have been found to be important and which are used in preferred embodiments of the invention to assess the performance of the medical facility.

A number of factors affect the commercial viability of an apheresis system. One factor relates to the operator of the system, specifically the time and/or expertise required of an individual to prepare and operate the apheresis system. Reducing the time required by the operator to load and unload the disposables, as well as the complexity of these actions, can increase productivity and/or reduce the potential for operator error. Moreover, reducing the dependency of the system on the operator can lead to reductions in operator errors.

Donor-related factors can also impact the commercial viability of an apheresis system and include donor convenience and donor comfort. Donors typically have only a certain amount of time which may be committed to visiting a blood component collection facility for a donation. Consequently, once at the collection facility the amount of the donor's time which is actually spent collecting blood components is another factor which should be considered. This also relates to donor comfort in that many view the actual collection procedure as being somewhat discomforting in that at least one and sometimes two access needles are in the donor throughout the procedure.

Performance-related factors affect the commercial viability of a treatment system. Performance may be judged in terms of the "collection efficiency" of the system, which may in turn reduce the amount of treatment time and thus increase patient convenience. The efficiency of a system can be gauged in a variety of ways, such as by the amount or rate of a particular blood component type which is treated or passes through the system. Performance can also be evaluated based upon the effect which the procedure has on the various blood component types. It is also desirable to minimize the adverse effects on the blood component types as a result of the procedure.

In dialysis, it is important to use some method to quantify the extent of dialysis which is administered to a patient. One method is referred to as "Urea Kinetic Modeling" (UKM). This method is based upon measurement of the level of urea in the blood, both before and after each treatment. These values are then employed in a theoretical model, which describes how the level of urea in the blood is changed during dialysis. In this model it is assumed that the degree of purification of the blood in the dialyzer is given by clearance K (including the remaining function of the kidneys), and that this leads to a similarly large concentration (c) of urea in the whole distribution volume (V) of same in the body. If one thus neglects the production of urea in the body, as well as the change in fluid volume during dialysis, one can then after a treatment time (T) arrive at a concentration (C).

The coefficient KT/V is normally utilized as a measure of the administered dose of dialysis and can, in the above model, be calculated from the concentrations of urea both before and after the dialysis treatment has taken place. This model can be corrected for the production of urea by measuring the concentration at the start of the next dialysis, and can then also provide a measure of the urea production, which is an indirect measurement of the patient's protein intake.

There are numerous patients on dialysis in the United States and other countries. Most of them dialyze in hemodialysis centers (clinics). Some are on home peritoneal dialysis with less on home hemodialysis. In-center hemodialysis can be performed three times per week for between two and four hours. Four times per week dialysis sessions are typically used only with patients with severe intolerance to three times weekly dialysis, mostly related to cardiovascular instability. Home hemodialysis is usually performed three times weekly.

A prerequisite for hemodialysis (HD) treatment is the possibility to lead a portion of the patient's blood through an extracorporeal circuit, i.e. outside the body. For this a good vascular access to the blood stream is needed. The best and most widely used vascular access for chronic HD treatment is the arterio-venous fistula. If a peripheral artery is "short circuited" and directly connected to a vein, this vein will develop thick walls as the internal pressure and flow increase; it is arterialised. The thick walls of the vessel permit repeated punctures with large bore needles. The blood flow in the vessel - the "fistula" - is now substantial, up to 1000 ml/min. From a good fistula it should be possible to obtain an extracorporeal flow of up to 400 ml/min without any problems for the patient. The most common place for a fistula is in the forearm, where one of the two arteries to the hand is surgically connected to a superficial vein. A maturation period of four weeks or more is needed for the arterialization process. An AV fistula can in favorable cases function well for 10-15 years. However, many patients encounter problems, e.g. constriction of the fistula by gradual hardening and narrowing of the walls (stenosis) or obstructions by blood clots (thrombosis). In many cases reconstructive surgery or the creation of a new fistula in another limb is necessary. In some cases, the patient's blood vessels are so fragile that an AV-fistula cannot be created. Then a synthetic graft may be used to form a connection between an artery and a vein, which can be punctured just like a natural fistula but that has a shorter life span. For acute treatment, a temporary access is created by the insertion of catheters into deep-lying veins. The catheters can be placed in, for example, the groin or the neck. In the latter position it can remain for a prolonged period of time and serve as a permanent access, when no other alternatives are available.

Normally, two identical fistula needles are used in hemodialysis, one for the arterial blood line and one for the venous. The use of large bore needles can be very stressful for the patient and the personnel. On the other hand, small needles may limit the treatment efficiency as they do not allow high blood flow rates to be reached. In order to reduce the number of needle punctures, single-needle dialysis is sometimes used.

An arteriovenous fistula is the most commonly used method of creating blood access for hemodialysis. For each dialysis session, the fistula can be punctured with large bore needles to deliver blood into, and return blood from, the artificial kidney (dialyzer). The punctures with these large bore needles can be painful, even with the use of anesthetics. It is natural that the patients would like to have punctures done as infrequently as possible. Also, there is a general perception that frequent punctures are detrimental to the fistula longevity. Three times weekly dialysis schedule seems to be a reasonable compromise.

To prevent the blood from clotting in the extracorporeal circuit an anticoagulant is needed, most commonly heparin. The anticoagulant can be administered intravenously prior to and during the treatment. Often, the patient's coagulation capacity is monitored in terms of clotting time, i.e. the time it takes before the blood clots. During the treatment, the clotting time should preferably be prolonged by 50-100%. A loading dose is normally given directly through one of the fistula needles prior to the start of dialysis. When the treatment starts, additional heparin may need to be administered.

When arriving at the clinic or other medical treatment facility, the patient is weighed and recorded. The fluid volume to be removed, the UF volume, is calculated from the weight gain since the last treatment, to which is added the volume of the drink consumed during the session as well as of any infusions.

When the UF-volume is set, the dialysis machine (monitor) can calculate the required UF rate by considering the treatment time, normally standardized between 3.5 and 5 hours. The length of the treatment is a compromise between practical and social considerations and the physiological limits for the removal rate of fluid and solutes. To achieve an efficient solute removal, the blood flow rate, $Q_B$, should be kept high. Care must be taken to ensure that the fistula can give the chosen blood flow without collapsing. A further problem is that at higher $Q_B$, recirculation may occur in the fistula, i.e. the "cleaned" blood may take a shortcut and re-enter the arterial line instead of flowing back into the body. This leads to reduced solute removal.

To accomplish a satisfactory dialysis treatment, two things have to be achieved: adequate removal of excess fluid and adequate removal of unwanted solutes. Although it is not known which substances cause uremia, it has been shown that efficient removal of urea is correlated with successful clinical results.

The simplest way to follow the urea removal is to analyze and compare the blood urea concentrations before (pre-) and after (post-) dialysis. The index Kt/V is widely used for treatment planning and follow-up. The expression consists of the clearance urea (K), the treatment time (t) and the volume of body water (V). The recommended Kt/V for an adequate hemodialysis session is much discussed; at present a minimum of 1.2 is recommended. However, the Kt/V index is just a tool to understand the relationship between patient size, clearance and treatment time.

One contribution to the well-being of renal failure patients is the introduction of erythropietin, EPO. This hormone is produced by the kidneys and controls the production of red blood cells in the bone marrow. EPO can be manufactured by means of genetic engineering. In most forms of renal failure, EPO production is impaired and this results in anemia. This means that the number of red blood cells and the bood concentration of hemoglobin are below normal. The hematocrit is a measure of the volume fraction of red blood cells. A hematocrit of 45% which is typical for a healthy subject, means the 45% of the volume of the blood consists of red blood cells. Before the introduction of EPO, dialysis patients usually had hematocrits of 20-25%, and frequent blood transfusions were required to maintain even these subnormal levels. Today, with EPO, hematocrit levels are generally above 30% and often the target level is 35%. The tendency in many places is to increase it even further, to physiological levels. EPO administration can follow different strategies. It can be given to the patients intravenously through the venous needle at the conclusion of each treatment. It can also be administered as a subcutaneous injection, given in the thigh or the stomach, usually performed one to three times a week in connection with dialysis. Since the cost of EPO is high, the latter method is often preferred, as it requires a smaller dose. To benefit from the EPO treatment, the patient also needs administration of iron.

It is often desirable to reduce the treatment time. The benefit for the patient is that he spends less time at the clinic, while the benefit for the clinic is to be able to use the facililty more efficiently, fitting in more than two sessions per day. In order to reduce the dialysis time, the fluid and solute removal rates can be increased so that the same degree of blood purification is achieved.

High-efficiency dialysis is normally defined as a method where the rate of solute removal is higher than is standard therapy. This is accomplished by a combination of high blood and dialysis fluid flows and dialyzers with large surface areas. For the fluid removal, the physiology of the patient sets the limit. A machine with volume control is necessary in order to carefully control the ultrafiltration. There is always a risk that the fluid overload cannot be removed in the shorter period of time, resulting in overhydration and subsequent hypertension.

Methods based on convective solute removal using highly permeable membranes can be useful. Compared to "classical" HD, i.e. a standard low-flux treatment based mainly of diffusive transport of solutes, these convective therapies have two main benefits: superior removal of large solutes, e.g. $B_2$m, and improved blood pressure stability.

Hemofiltration, HF, is an important convective therapy. No dialysis fluid is used. Instead a very large volume of fluid is ultrafiltered from the patient's blood - up to 80-100 litres- depending on the HF mode. Substitution fluid, the composition of which is close to that of dialysis fluid, is continuously infused into the blood circuit. The volume of the substitution fluid is adjusted to be somewhat smaller than the UF volume, the difference corresponds to the desired weight loss. Hemodiafiltration, HDF, is a hybrid between HF and HD, combining diffusion and convection. The major drawback of convective therapies is the large cost of the substitution fluid which needs to be sterile and is supplied in large, heavy bags. Some modern HF machines can produce non-pyrogenic fluid on-line, which significantly reduces the cost. High-flux dialysis, i.e. hemodialysis with a high-flux filter, is sometimes also referred to as a convective therapy, although the convective transport is limited compared to HDF and HF.

Existing hemodialysis systems consist fundamentally of two halves; one comprising the extracorporeal blood circuit (the blood flow path) and the other comprising the dialysate circuit or flow path. Typically, the entire blood circuit is disposable and comprises: (a) an arterial and venous fistula needle, (b) an arterial (inflow) and venous (outflow) blood line, (c) a hemodialyzer, (d) physiologic priming solutions (saline) with infusion set, and (e) an anticoagulant (heparin or citrate). The arterial needle accesses blood from the patient's fistula and is connected to the arterial blood tubing set, which conveys blood to the dialyzer. The arterial line can comprise: a pumping segment with interfaces to a blood pump (rotary or peristaltic) on the hemodialysis machine, pressure monitoring chambers including tubing which interfaces to pressure transducers on the machine to monitor the pressure pre-pump and/or post pump, inlet ports for saline and anticoagulant, and one or more injection sites fer drawing blood or injecting drugs.

The hemodialyzer can comprise a case which encloses a semi-permeable membrane. The blood is circulated on one side of the membrane while dialysis solution is circulated on the other, so that the two never come into direct contact. Uremic toxins diffuse out of the blood and into the dialysis solution owing to the concentration gradient. Excess water in the patent's blood enters the dialysate as a result of a pressure gradient. The membrane can be made from cellulose or synthetic polymers.

The venous line and needle carry the newly dialyzed blood away from the dialyzer and back into the patient's circulatory system via a puncture site slightly closer to the heart than the arterial needle site. The venous set is comprised of a pressure monitoring chamber with tubing leading to another pressure transducer in the machine, injection sites, and a segment of tubing which interfaces to an air detection assembly in the machine in order to prevent air emboli during treatment.

The set flow rate for the dialysis fluid through the dialyzer can be 500 ml/min, although higher flow rates, up to 1000 ml/min, are sometimes used in high-efficiency dialysis. Dialysis fluid is prepared, i.e. mixed, heated and degassed, during the course of the treatment. Normally, each individual dialysis machine (monitor) produces the fluid form liquid concentrate by continuous dilution with purified water. In some dialysis units, the fluid production is accomplished by a central system which distributes the fluid to all the machine in the clinic. The water entering the dialysis machine can be first heating to between 36 °C and 40 °C before it is mixed with the dialysis concentrate. The temperature regulation includes a second temperature measurement before the fluid enters the dialyzer. Underheated fluid can cause the patient to feel discomfort from chilling, but normally no medical damage is done. On the other hand, overheated fluid can be harmful to the patient at temperatures above 41 °C, this will cause damage for example to the blood protein. Incoming water into the dialysis machine (monitor) contains large amounts of dissolved air which should be removed. The air bubbles, which are released when the fluid is exposed to negative pressures, may sometimes distort the flow and conductivity measurements, in some cases also affect the dialyzer performance. Degassing can be performed by the dialysis machine, for example, after the mixing step. The fluid is exposed to a high negative pressure and the released air is then allowed to escape in a degassing chamber located after the flow pump.

Even if the incoming water in the dialysis unit as well as the concentrates used are of a high microbiological quality, bacteria may still enter the system. Therefore, disinfection of the fluid circuit is useful to prevent excessive bacterial growth in the dialysis machine. Proper disinfection after each treatment is achieved with heat at 90-95 °C or chemicals such as peracetic acid, hypochlorite or formaldehyde. The most highly recommended procedure is to use heat disinfection after each treatment, complemented by a weekly chemical disinfection. After chemical disinfection and before initiating a treatment, a final rinse can be performed. Test kits are available for analysis of residual disinfectants in the dialysis machine after rinsing.

When using bicarbonate dialysis fluid, some precipitation of calcium carbonate can occur in the fluid circuit. This requires decalcification such as with a citric acid solution. Dialysate from the dialyzer contains organic matter from the patient which can also precipitate in the fluid circuit. A regular alkaline cleaning such as with hypochlorite can be used to clean such organic matter.

Dialysis solution can be prepared continuously on-line in present-day machines by combining: (1) water which has first been purified by a separate water treatment system and (2) liquid concentrates of electrolytes. Dialysate concentrates have evolved from a single formulation which contained acetate as the physiologic buffering agent for the correction of circulatory acidosis to two containers where bicarbonate replaces acetate as the buffering agent, and should be kept separate due to its chemical incompatibility with calcium and magnesium. Two proportioning pumps are often used, the first to mix the bicarbonate concentrate with water and the second to proportion this mixture with the concentrated electrolytes to achieve the final physiologically compatible solution.

The dialysis machine monitors the pressure at the blood inlet and outlet sides of the dialyzer (by way of the pressure transducers connected to the blood sets) as well as in the dialysate circuit. The dialysis system can calculate the transmembrane pressure, such as with a microprocessors or other CPU to determine the amount of water transmission through the membrane. Dialysis machines can also measure the amount of dialysis solution entering and dialysate leaving the dialyzer, which allows the calculation of net water removal from the patient (ultrafiltration). By electronically comparing the amount of water entering or leaving the blood with the transmembrane pressure, the dialysis system is able to actively control the water removed from the patient to a desired target previously programmed into the system. When low-water-transmission cellulosic membranes are employed, negative pressure is usually generated on the dialysate side of the membrane by the machine in order to accomplish sufficient water removal. Because suction may be applied to the dialysate as it transits the dialyzer, it should be first be placed under a greater vacuum in a degassing chamber so that air bubbles are not generated within the dialyzer that would cause errors in the calculation of ultrafiltration by the flow sensors and also reduce the efficiency of the dialyzer. When high-water-transmission, synthetic membranes are used, it is frequently desirable to apply positive pressure on the dialysate side to control the rate of ultrafiltration.

Dialysate fluids provide dialysate for the dialysis. For the concentrate of electrolytes no preparation may be necessary; a hose from the dialysis machine is simply inserted into a jug or other container just as it comes from the manufacturer. The bicarbonate, however, is most often bought as a powder because of its instability in solution, and should be mixed in a jug or other container with purified water. When the concentrates are ready, the dialysis machine is activated so that the temperature and conductivity have time to come into their effective operating ranges.

After, the dialysis fluid is prepared, other components of the extracorporeal blood circuit can be unpacked, connected together using aseptic technique and secured to the dialysis machine by matching the respective components to their hardware interfaces. The air can be purged from the circuit by connecting sterile normal saline to the arterial tubing set via an intravenous (IV) administration set, and thereafter activating the blood pump on the dialysis machine. Agitation of the dialyzer is frequently necessary to completely purge air. Some practitioners are able to both prime the circuit and rinse the blood back at the end of treatment with a single one liter bag of saline, but most often, two one-liter bags are required.

If the dialyzer is being reused, a chemical sterilizing solution may be present in the dialyzer instead of air, and this must first be rinsed out. Once the bulk of the disinfectant and air are primed out of the circuit, the arterial and venous blood lines are usually connected together to form a closed loop. Thereafter, the enclosed solution is recirculated countercurrently to the dialysate, thus causing any remaining contaminants to dialyze across the membrane, into the dialysate, and down the drain. Before the patent can be connected to this primed extracorporeal circuit, the priming fluid can be manually tested for residual sterilizing chemicals (e.g. formaldehyde) by a calorimetric chemical assay to assure they are at safe levels.

Arterial and venous needles can be placed in the patient's blood access site. The pump can be started to cause the priming solution to the displaced into a drain container. When blood approaches the venous tubing set, the pump is stopped. The set is connected to the venous needle, and the pump speed is gradually or rapidly elevated to the prescribed value. Blood flow rates of 175-450 ml/min are typical, being limited by the needle size and access anatomy. The faster the blood flow rate, the faster the dialysis procedure can be accomplished, thereby benefitting both patient and physician (as long as water removal rates are tolerable). However, the needles, which in the past have in part determined the allowable blood flow rate, are typically 14-17 gauge, and are already pushing the tolerance of most patients.

The patient can be then dialyzed for a period specified by the nephrologist or other medical personnel. The patient can be monitored for pulse, temperature, and blood pressure, and the functions of the machine are also noted and recorded on the patient's chart. Monitoring the patient, especially for blood pressure, is useful because, a significant number of dialysis patients have very fragile cardiovascular systems some hemodialysis procedures result in hypotensive episodes owing to the rapidity of removing in 2-4 hours the fluid which has been accumulated over 2-3 days. Most of the patients have prodromal symptoms before hypotensive episodes but sometimes the episode occurs suddenly, without warning and patient "crashes", losing consciousness. Most of the "crashes" happen during the second half of hemodialysis sessions. The standard treatment for such blood pressure "crashes" is for medical personnel to open up the IV administration line connecting the saline bag to the arterial blood set and to infuse the saline in order to improve the patient's circulatory volume and bring the pressure back up. Slower ultrafiltration helps to reduce incidence of crashes. The "crashes" are less frequent if controlled ultrafiltration machines are used.

Clinically, three dialyses per week are associated with rapid changes in body fluid compartments and in concentrations of all dialyzable solutes. These changes are a major cause of side effects. Many patients have enormous difficulties achieving a dry body weight if they accumulate three, four, or more kilograms of fluid between dialyses. Some patients, especially with heart failure, poorly tolerate even a two kilogram fluid weight gain; they are short of breath before dialysis, have muscle cramps and hypotension during dialysis, and feel washed out and are extremely weak, needing several hours to equilibrate and become functional. Serum concentration of highly toxic potassium frequently reaches dangerous levels (more than seven mEq/L), particularly preceding the first dialysis after a longer interval (e.g. weekend). Calcium and pH can be too low before dialysis or too high after dialysis in many patients. In some hemodialysis units, these patients are placed on a four times weekly dialysis schedule.

Another common occurrence during hemodialysis happens when the arterial needle engages (sucks up) against the interior wall of the blood vessel either because of the suction generated by the blood pump or because the patient changes his/her arm position. This creates excessive negative pressure in the pre-pump segment of the arterial line which, if monitored by the dialysis machine, will trip an alarm and shut off the blood pump until someone repositions the needle and/or arm and restarts the pump. This, of course, wastes time and lengthens the procedure. If the pre-pump pressure is not monitored, then as suction increases, the blood flow rate diminishes dramatically and the amount of dialysis expected does not occur.

At the end of the treatment, the arterial needle is removed, the saline line opened, and the pump started in order to flush the blood remaining in the extracorporeal circuit back to the patient. Many patients are anemic (the kidneys control the production of new red cells) and, therefore, retrieval of as much blood as possible is important. Since flow is always in the same arterial to venous direction through the circuit, the arterial needle can be inserted into the saline bag so the few inches of tubing between the needle and the saline infusion port will also be flushed.

When the blood is mostly out of the extracorporeal circuit, the venous needle can be removed from the patient, and a compress can be applied to the puncture site until it clots, which can be 10-20 minutes depending on the size of the needles and the degree of systemic anticoagulation at the end of the treatment. Afterwards, the needles and blood lines can be discarded in biohazard containers as these components are rarely reused.

In the USA, most dialyzers are reused. There are numerous procedures for reusing dialyzers both manually and automatically. In centers (clinics), special machines for simultaneous multiple dialyzer regeneration can be used. Generally the steps are as follows: (a) Water at high flow rates flush of blood compartment; (b) Water is forced through the membrane in dialysate compartment to the blood compartment direction (reverse ultrafiltration); (c) Residual blood and protein are removed by flushing blood compartment with bleach and/or peroxide; (d) Further water flush; (e) The remaining fiber bundle volume or the ultrafiltration rate can be measured as an indicator of remaining dialyzer efficiency; (f) The dialyzer is cleaned with a chemical sterilant such as formaldehyde, peracetic acid, or glutaraldehyde.; and (g) The preceding steps can be documented to minimize the chance of using a reused dialyzer on a different patient. Regeneration of dialyzers and lines can be performed on or in association with the dialysis machine. After treatment, the dialysis machine plumbing should be periodically cleaned and disinfected.

Whereas the normal human kidneys function continuously to produce seamless, gradual changes in total body fluid volume and metabolic waste levels, three times weekly dialysis schedules can produce fluctuations which can cause considerable stress on the patient's systems and may affect their prognosis.

Home hemodialysis is convenient but has many disadvantages: (a) Current equipment is big, complicated, intimidating, and difficult to operate, requiring a very long time for training. Also, both partner and patient must be trained and this represents a major expense to the medial provider; (b) Complication of equipment engenders reliability issues. If a hemodialysis system breaks down in a patient's home, no dialysis is possible until it is repaired; (c) It is currently very difficult for home hemo patients to travel since the present systems are in no way portable; (d) If the bicarbonate component of the dialysate is used in powdered form, it must be mixed and inspected by the patient; (e) Supplies require a large storage space; (f) There is a high initial investment in the dialysis equipment, the water treatment system, and their instillation with low utilization (one patient only) compared to in-center use where the systems are used on many patients; (g) There is little or no possibility for reuse of supplies, providing less economic incentive to the medical sponsor; (h) A partner is required to insert fistula needles and monitor emergencies; (i) Considerable time is involved for setup, priming, tear down, and cleaning; and (j) The water treatment system should also be cleaned/disinfected periodically.

One type of hemodialysis machine is manufactured and marketed under the tradename GAMBRO AK 200 ULTRA by Gambro Lundia AB of Sweden and is adapted to perform hemodialysis, hemodiafiltration or hemofiltration treatments.

The dialysis machine can prepare a dialysis solution e.g. a dialysis fluid comprising sodium, bicarbonate, potassium, calcium, magnesium, chloride and acetate ions in suitable concentrations, as well as possibly glucose and other ions, all dissolved in water. The concentrations of the ions in the dialysis solution are generally complementary to their concentrations in blood, where the optimum is the normal concentration of these ions in blood. Therefore, if the concentration of an ion is increased in the blood over its normal concentration, the ion concentration in the dialysis solution can be decreased in relation to the normal concentration. The pH of the solution can be adjusted to about 7.1-7.4.

During hemodialysis treatment, the solution comprising a dialysis fluid is used to attain dialysis in a dialyzer. The dialyzer can be divided into two chambers by a semi-permeable membrane. Blood which is to be treated passes over one side of the membrane while the dialysis solution prepared by the dialysis machine passes over the other side. Diffusion of ions can occur through the membrane in order to condition the blood and to at least partially replace the function of the kidneys. Also, a quantity of liquid can be removed from the blood since the patient is unable to get rid of surplus liquid in the normal manner. This removed liquid flowing through the membrane is sometimes referred to as the ultrafiltrate flow.

During hemodiafiltration, the ultrafiltrate flow is increased above that which is necessary to restore the liquid balance of the patient. As replacement, an infusion solution can be added to the blood in order to permit such increased ultrafiltration flow.

During hemofiltration, substantially no dialysis takes place, but instead the blood is filtered, whereby a portion of the ultrafiltration volume is added to the blood as an infusion solution. The difference between the ultrafiltration volume and the added substitution volume constitutes the volume of liquid which is removed from the patient in order to restore the liquid balance. The infusion solution may be added upstream of the dialyser or hemofilter in a process referred to as "pre-infusion", or downstream of the dialyzer or hemofilter in a process referred to as "post-infusion".

In order to effect the infusion flow, the dialysis machine can include an infusion pump which can be connected to an outlet for infusion solution on the dialysis machine. The infusion solution is normally the same as the dialysis solution. The infusion solution passes through the infusion pump, and a sterile filter, and is then fed to the patient's blood. The infusion pump can be a so-called peristaltic pump.

Prior to treatment, the dialysis machine can be connected to a tube set, the constituent components of which must be filled with liquid so that all air is expelled. This normally takes place in a priming step during which sterile sodium chloride solution is fed into the various tubes and components of the tube set. The conduit system of the dialysis machine can also be filled with dialysis solution.

During priming of the infusion circuit, a dedicated deaeration conduit from the sterile filter can be used in order that the filter can be completely filled with priming liquid so that air is expelled. Medical personnel often use forceps or a tube clamp, which can be placed on the normal output conduit from the sterile filter to block the flow therethrough. Subsequently, a tube clamp can be opened in the deaeration conduit from the sterile filter. After deaeration of the sterile filter, medical personnel can removes the forceps from the output conduit or open the tube clamp, as well as close the tube clamp on the deaeration conduit and the priming continues.

The infusion pump can be a peristaltic pump which is driven at a predetermined speed or number of revolutions so that a desired infusion flow or volume is attained. Peristaltic pumps are sensitive to the pressure at the inlet. It is desirable to calibrate the pump for the particular treatment and/or to check whether the desired infusion volume has actually being attained at a particular rotational speed of the peristaltic pump.

Extracorporeal dialysis is based on the contact of the blood to be purified with a dialysate through a semi-permeable membrane. In order to obtain efficient exchanges, the blood and dialysate should be efficiently processed. The speed of diffusion, or of dialysis, depends on the difference of concentrations between the blood and the dialysate. This is obtained by creating an extra-corporeal circulation

The artificial kidney or dialyzer eliminates water and of the waste produced by the body by contacting the blood with a liquid which is the dialysate or a dialysis bath. The blood and the dialysate are separated from one and other only by means of a thin artificial organic membrane, of cellophane, cuprophane, polysulfone, polypropylene, polyamide, polyacetate, copolymer of acrylonitrile, etc. The membrane can be prepared in different ways, e.g. in the form of plates, as compartments which are piled over one and other, or in the form of coils, as a single compartment which is spirally wounded about an axis or finally as capillaries consisting of an assembly of very fine hollow fibres. There are three types of dialysis which are often delivered sterile for single use. In the coiled kidneys, of the type where two sheets of dialysing membrane are spirally wound in a parallel fashion about an axis, the blood circulates inside the envelope defined by the two sheets of the membrane and the dialysate on the outside. The coiled kidney is actually the less utilized. Plate kidneys can all disposed corresponding to an assembly of 15 to 20 sheets of membrane mounted as an arrangement of plates in a sandwich type fashion so as to provide inserted blood compartments and dialysate compartments. The blood circulates from top to bottom between the sheets while the dialysate circulates in opposite direction. Hollow fibre kidney contains very little blood; it offers a very weak resistance to the flow of blood. Instead of sheets of membrane in roller or plate arrangement, the dialysing surface is formed in the case of thousands of tiny hollow fibres. The blood passes from top to bottom and the dialysate passes around in the other direction.

Dialysate is a mixture of water and electrolytes, and the concentration of the electrolytes in the dialysate is provided so that the composition of the electrolytes in the blood is normal at the end of the operation. For hemodialysis uses, sterile water should be used. Hemodialysis equipment can be provided with a water treatment consisting of softeners, demineralizers, osmosers, filters and a container for storing water.

Hemodialysis systems generally comprise a console or cabinet which provides both the blood and dialysate handling and processing functions that are necessary for hemodialysis. The membrane dialyzer can be positioned in proximity to the console, along with blood conduit set containing arterial and venous conduits, as well as blood pump segment of the arterial conduit. Inside the console, the dialysate handling and processing equipment can include a proportioning system to mix a dialysis concentrate with water to form the dialysate. The console can also include: temperature gauges, conductivity meters, a dialysis solution pump, and other components for the processing, heating, monitoring, and pumping of dialysate through the membrane dialyzer unit. A pair of tubular dialysate conduits can be provided for connecting the membrane dialyzer with a supply of dialysate. The console can also include the blood handling and processing equipment comprising typically a blood pump, heparin pump, air bubble detector, line clamp, and blood pressure monitors and alarms. This equipment acts upon and/or communicates with the blood pathway inner lumens of the arterial and venous blood conduit sets.

The arterial and venous blood conduit sets can be connected to the membrane dialyzer, as well as being connected to the patient, to provide an extracorporeal blood flow circuit between the membrane dialyzer and the patient. The membrane dialyzer can be carried on the console during the hemodialysis procedure. The arterial and venous blood conduit sets typically include long tubing. Such long tubing creates a significant extracorporeal blood volume that can cause a strain on the patient's vascular system. Any reduction in extracorporeal blood volume without an attendant rise in pressure drop can result in a significant reduction in hypotension of other hemodynamic problems currently endemic to hemodialysis. Usually, the arterial and venous blood conduit sets for hemodialysis are disposed of after one use, or a few uses at most. The cost of the sets represents a significant percentage of the cost of dialysis.

Advances have been made in the treatment of end stage renal disease. Through dialysis with artificial kidneys it is possible to keep patients alive, and to permit them to lead relatively normal lives, even after loss of kidney function. One form of dialysis is hemodialysis, where the patient's blood is removed from the patient's body, anticoagulated, circulated by an extracorporeal tubing circuit through an artificial kidney, or dialyzer, to remove toxic substances, such as urea and creatinine, as well as excess fluid, and returned to the patient. Hemodialysis is typically performed on a patient about every three days.

The extracorporeal tubing circuit typically comprises cannulae for drawing blood from and returning blood to a patient, the dialyzer and a blood tubing set. The blood tubing set typically comprises a plurality of sections of medical tubing, bubble traps or drip chambers (collectively "bubble traps" herein), pressure monitoring sites, air bubble detection sites, access sites connectors, clamps, peristaltic pump headers and accessories of various sorts. Pressure monitoring sites can be disposable pressure pods which transmit the pressure of blood, or another fluid, to a pressure sensor while simultaneously isolating the blood or other fluid from the pressure sensor. Access sites can be disposable septa and associated housing for sampling the patient's blood or adding medication. Access sites can require two hands to operate, one to hold the site steady, another to operate a sampling or injection syringe.

As previously discussed, extracorporeal fluid treatment typically involves the removal of a body fluid from a patient, treatment of the fluid externally to the patient, and return of the treated fluid to the patient. Blood is one body fluid for which conventional extracorporeal techniques have been developed. Using these techniques, blood is treated to extract materials from the blood and/or add materials to augment the blood prior to return of the treated blood to the patient. More particularly, extracorporeal blood treatment can be accomplished by removing the blood from the patient in a continuous flow and introducing the blood into a chamber containing a filtration unit, wherein the blood is conducted past a semi-permeable membrane. The semi-permeable membrane selectively allows material in the blood to pass through the membrane for removal from the blood and/or allows material to pass through the semi-permeable membrane to the blood, to or from a fluid separately flowing past the semi-permeable membrane of the filtration unit. After passage of materials to and from the blood, the treated blood is discharged from the filtration device for return to the patient. The material which has been removed from the blood is separately discharged from the filtration unit.

One exemplary extracorporeal blood treatment is hemodialysis. In hemodialysis treatment, treated water can be provided to a hemodialysis machine and mixed therein with a predetermined amount of one or more solutes or concentrates to form a dialysate. The water can be heated in the hemodialysis machine before and/or after addition of solutes and/or concentrates, typically to a body temperature. Fresh dialysate can be conducted into a filtration device or dialyzer of the hemodialysis machine. Once in the dialyzer, the dialysate flows past a side of a semi-permeable membrane, typically in a counter-current direction to that of the blood from a patient flowing in the dialyzer on an opposite side of the membrane. Waste matter, typically organic molecular ions, plasma and water, is transferred from the blood to the dialysate due to osmotic, diffusive and convective action. In ultrafiltration, excess fluid can be removed from the blood by establishing a pressure differential across the membrane that pulls the excess fluid from the blood across the membrane and combines it with the dialysate in the dialyzer. Dialysate discharged from the filtration device, sometimes referred to as spent dialysate, can be conducted past a heat exchanger where heat from the spent dialysate can be transferred to the treated water being provided to the hemodialysis machine on the "fresh" side. Thereafter, the spent dialysate can be conducted to a drain line for collection, analysis and discharge.

A single hemodialysis machine generally does not operate continuously, but rather is used to treat blood in discrete treatment sessions, usually with different patients. The equipment can be idle between treatments and may accumulate deposits in the flowpath. Further, spent dialysate may contain molecules or material which can accumulate in the flowpath after the dialyzer, which can provide a nutrient source for bacterial growth and accumulation therein. The use of such equipment for different patients, the need to prevent patient pyrogenic reactions due to bacterial endotoxin, and the possible accumulation of dirt or other unsterile substances in the equipment make periodic cleaning and disinfection of the equipment desirable.

Cleaning of equipment can be accomplished by rinsing the affected portions of the flowpath with bleach solution or other disinfectant. Chemical and/or heat disinfection are methods commonly used to disinfect the non-disposable portions of hemodialysis equipment. Chemical disinfection techniques include the use of chemicals such as formaldehyde, bleach, peracetic acid or other disinfectant solutions through the non-disposable portions of such equipment. Chemical disinfection techniques often require medical personnel to add, remove and/or dispose of the chemical disinfectant, while disconnecting the hemofiltration device and other components from the dialysate equipment. In disinfecting equipment with chemicals, care must be taken to completely flush the chemicals, from the portion of the flowpath in which the dialysate is prepared and through which fresh dialysate flows during treatment, to avoid any possibility of delivering the chemical to the patient through the membrane of the dialyzer. There may also be environmental concerns or regulations which restrict the discharge of the disinfecting chemicals to public waste disposal facilities. Proper container disposal is important.

Heat disinfection of extracorporeal blood treatment systems can also be useful. Heat disinfection of the fluid pathway of extracorporeal blood treatment systems is performed by circulating a fluid such as water, sterile water, or a disinfection solution throughout all such pathways of the equipment for a sufficiently long period of time, typically 15 minutes or more, at a sufficiently high temperature, typically from 80-125 degrees C. One way in which such disinfection is achieved is by conducting the solution (a) through the pathway which ordinarily receives treated water, (b) into the portions of the flowpath in which fresh dialysate is prepared with heated water and in which fresh dialysate flows during treatment, (c) bypassing the dialyzer, (d) into the portions of the flowpath in which spent dialysate flows during treatment, and (e) through the drain or discharge line to exit the machine. This technique is sometimes referred to as a single path once-through heat disinfection method. Single path once-through heat disinfection discharges the heated fluid through the drain line to the drain. As a result, fluid continuously added to the system must be heated to temperature, which consumes additional power and results in additional cost. This problem has been minimized in some hemodialysis systems by routing all or a portion of the heated fluid from the part of the flowpath in which spent dialysate flows during treatment back, to the part which receives treated water, or to the dialysate preparation portion, thereby conserving heat and reducing the power required to maintain adequate disinfection temperature. These techniques are sometimes referred to herein as single path heat disinfection methods with recirculation. Although single path heat disinfection with recirculation reduces the power consumed by the disinfection process, it creates a new problem. The heated fluid, in passing through the spent dialysate line, may pick up material in the line deposited by spent dialysate during prior treatments. Such material may be subsequently carried into the dialysate preparation line and fresh dialysate lines. This creates a possibility of contamination which may potentially be passed on to patients subsequently treated with the equipment.

Single path heat disinfection methods may exhibit heat loss between a point in the dialysate preparation line where the solution is heated and the drain line where solution is discharged from the system. Such heat loss results in decreasing temperature from the dialysate preparation portion of the flowpath to the drain line which may result in incomplete disinfection of the flowpath approaching the drain line. The disinfection fluid in the dialysate preparation portion is sometimes heated to a temperature substantially above 90 degree C., for example at about 125 degree C., so that the resulting fluid temperature gradient from the fluid in the dialysate preparation portion to the fluid in the drain line results in a low temperature of closer to 90 degree C. However, this technique can sometimes cause damage to equipment which may not be designed for repeated operation at such elevated temperatures or can require the use of material capable of withstanding elevated temperatures.

The process of the invention improves operations of clinics and other medical facilities to enhance care and treatment of patients by assessing the performance of the facilities using an analysis of several technical features. While the process can be used with many types of treatment, it is particularly useful for extracorporeal blood treatment for patients requiring blood purification, such as ultrafiltration, hemofiltration, hemodiafiltration, plasmapherisis, apherisis, and especially hemodialysis and dialysis. Furthermore, while the treatment (Rx) can be administered in the home of the patient, the process is particularly useful when the treatment is administered at one or more medical treatment facilities, such as at clinics, hospitals, centers for medical treatment, patient treatment facilities of health care providers, and/or doctor's offices (i.e. offices of physicians).

As previously indicated, each patient can be treated by: preparing the patient for the treatment; preparing a dialysis fluid for the patient; injecting an injector consisting of a needle or a catheter into the patient; removing blood from the patient through the needle or catheter via tubing connected to a monitor (the monitor comprising a dialysis machine with a dialyzer cartridge having a filter) (the catheter can be multi-use or single use); passing the removed blood through a semipermeable membrane; pumping the dialyzer fluid from the monitor through the semipermeable membrane; and returning the treated blood which has passed through the semipermeable membrane to the patient via the needle or catheter and the tubing. The treatment can be monitored by the monitor (dialysis machine) and/or medical personnel. After the treated blood is returned to the patient, the needle or catheter is removed from the patient by medical personnel. Thereafter, the monitor can be cleaned by disinfecting, sterilizing and/or sanitized with heat or a chemical disinfectant. The used tubing and injector comprising the used needle or used catheter are discarded (disposed) in compliance with governmental regulations. The used cartridge can also be discarded in an environmentally and medically safe manner as required by U.S. regulations or the used cartridge can be cleaned (disinfected, sterilized or sanitized) for reuse, such as is customary in Europe.

Measurements, Calculations and Statistical Analysis

The treatment for each patient and medical facility can be mapped, charted and recorded.

In order to improve operations at the clinics and other medical facilities and enhance the care and treatment of patients being administered the treatment, the effectiveness and efficiency of each treatment for each patient are measured. The effectiveness of each treatment can be measured according to the mathematical model KT/V wherein K = clearance, T = time of the treatment, and V = body distribution volume of urea or creatine. Desirably, the efficiency of each treatment is measured by determining the time for each treatment of each patient.

In order to further improve operations at the clinics and other medical facilities and enhance the care and treatment of patients being administered the treatment, the frequency of treatments per patient and the costs of each of the treatments per patient are determined. The total costs of the treatment for each patient are calculated.

A set, series or array of factors which comprise criteria and variables affecting the performance of the treatment of the patients in the medical treatment facilities can be entered (inputted) into a central processing unit (CPU). The factors can include: the measured effectiveness and efficiency of the treatments per patient, the frequency of treatment per patient, the costs of the treatment per patient, the variations in effectiveness, efficiency and costs of the treatments per patient, the patient characteristics, and the demographics of each facility, as well as other patient data, facility data, and cost data.

The CPU can be a: microprocessor, computer, main frame computer, server computer, desktop computer, workstation computer, laptop computer, notebook computer, palm pilot-type computer, computer chip, integrated circuit, electronic controller, network, internet, or global communications network.

Patient characteristics for each patient can be identified, such as by measuring the weight and height of each patient and determining the sex and age of each patient. Other patient data comprising patient information can be determined from each patient, such as: type of treatment per patient, duration (months) of treatments per patient, percentage of patients having the treatment as a primary cure for their ailments, race of patients, ethnic background of each patient, hemoglobin per patient, albumen per patient, catheter usage per patient, equipment usage per patient, temperature conditions during treatment, humidity conditions during treatment, type of equipment and supplies, composition of dialysis fluids, noncompliance per patient, iron supplement usage per patient, epogen usage per patient, crude mortality rate (CMR) of patients in the facility, average months on dialysis (MOD) per patient, and/or modified charleson comorbidity index (MCCI).

Demographics and facility data comprising demographic information can be identified for each facility. The facility data can further include: geographical location of each facility, metropolitan statistical area of each facility defining an urban MSA, ownership of each facility, type of ownership of each facility including company owned and joint venture facilities, length of service of each facility, hospitalization of patients, division, and/or employee turnover (T/O) at each facility.

Cost data comprising financial data can be identified and/or computed for each treatment and facility. Cost data can include: equipment costs per treatment, dialyzer costs per treatment, costs of supplies per treatment, costs for sterilizing dialysis equipment for the treatments, savings and costs for reuse of equipment for the treatment, labor costs per treatment, overhead per facility, percentage of patients covered by commercial insurance, reimbursement of Medicare for the treatments, reimbursements from government agencies for the treatments, and/or reimbursement from insurance companies for the treatments.

Variations (deviations) of effectiveness, efficiency, and costs of the treatments per patient, can be calculated with the CPU. Advantageously, the effectiveness of the treatments can be statistically analyzed with the CPU to calculate a standard deviation for KT/V for each patient, a standard deviation for inter patient KT/V, and/or a standard deviation for intra patient KT/V standard deviation, in order to help determine the effectiveness of the treatment.

Advantageously, the data can be statistically analyzed, compared, and correlated, such as with the CPU, to compute a sigma comprising a standard deviation around a mean of the data to determine the performance of the process. Desirably, the financial results of the process for the treatment of the patients at the facilities can be electronically calculated with the CPU. Such financial results can include: earnings, operating income, gross income, net income, gross margin, net margin, profits, EBITA comprising earnings before interest, taxes and amortization and EBITDA comprising earnings before interest, taxes, depreciation and amortization. The preceding data, analysis, correlations and comparisons can be retrieved from the CPU. Preferably, the process is operated and performed at about one sigma.

The process of the invention can improve profitability as well as quality of care. The process can provide: (a) global analysis of current clinic performance; (b) mapping and analysis of the dialysis process; and (c) research and development advantages for improving treatments, e.g. dialysis delivery.

A process can be defined as a series of steps and activities that take inputs, add value, and produce outputs (results). Instead of measuring something to see if it was good or bad (outcome), the measurement of the output can be an indicator of how well (effective) the process is performing. The quantitative measurement of the process effectivity for blood purification can be KT/V also referred to as (Kt/V) or (kt/v). The determination and evaluation of variation is a way to analyze the performance of the process. The variation measured statistically is the standard deviation (SD) around the mean, or sigma. The overall clinic variation can assess the ability of a given clinic or other medical facility to deliver the target Kt/V. Variation can be measured by calculating the variance or SD of a sample of the treatments performed during a given period.

Factors responsible for the overall clinic variation provide the ability of a clinic to deliver the same treatment to all patients (considering weight differences, etc). The inter patient variation is the ability of the clinic to deliver the same treatment to the same patient all the time. Such factors are technical and susceptible to being modified in a feedback loop.

The intra patient variation can be measured by taking a sample of patients from clinic (+/- 10-15% of population) and evaluating four or five consecutive treatments of each of those patients. The standard deviation (SD) or sigma of each of the treatment can be calculated using a statistical formula as shown below. The average of the standard deviations (SD) of the patients can also be calculated and compared.

Standard deviation can be calculated from the formula:

$$\sigma = \sqrt{\frac{\Sigma(X_i - \mu)^2}{N}}$$

where

$\sigma$ = sigma = standard deviation
$\mu$ = the mean
$X_i$ = values of the data (factors)
$N$ = number of data points (values) = population size

The following examples described studies, analysis, computations, comparisons and correlations of various clinics, patients and treatments. These examples shall not be regarded as restricting the scope of the process of the invention, as they are only examples of specific clinics, patients and treatment which could benefit from the process of the invention.

Example 1

Numerous patients in various clinics in different countries were studied as shown in Table 1.

Table 1:

| Clinics and Patients | | | |
|---|---|---|---|
| Country | Clinics | Patients | Total Patients |
| Argentina | 14 | 1059 | 1947 |
| Spain | 9 | 807 | 1761 |
| Italy | 8 | 419 | 692 |
| Portugal | 4 | 752 | 1192 |
| France | 3 | 722 | 926 |
| Sweden | 3 | 100 | 109 |
| Hungary | 6 | 476 | 617 |
| USA | 459 | all | all |

The histograms of the patients for the indicated countries in Example 1 are shown in Figures 1-5 and illustrate their Kt/V distribution for three (3) months.

Example 2

The mean (average) of the Kt/V and standard deviation of the patients and claims in Argentina, Spain, Italy, Portugal, France, Sweden and Hungary, as well as for 8 of the clinics in the USA, in Example 1 were calculated as shown in Table 2.

Table 2:

| Kt/V and clinic SD | | | |
|---|---|---|---|
| Country | Clinics | Kt/V (mean) | Overall Clinic SD (mean) |
| Argentina | 14 | 1.52 | .280 |
| Spain | 9 | 1.41 | .232 |
| Italy | 8 | 1.32 | .216 |
| Portugal | 4 | 1.41 | .253 |
| France | 3 | 1.53 | .327 |
| Sweden | 3 | 1.47 | .283 |
| Hungary | 6 | 1.22 | .194 |
| USA | 8 | 1.45 | .263 |

The main causes of the overall clinic variation were attributable to inter patient variation, e.g. differences in prescription of therapy, and intra patient variation, e.g. differences in process delivery: nursing, equipment; etc. The inter patient variations and intra patient variations are shown in Figures 7 and 8, respectfully.

The standard deviation for the patients and clinics in Example 2 are illustrated in Figures 9-13. The variations or percentage of treatments above or below a predetermined specified level per clinic per country are shown in Figure 14. The sigma operational level and variations for defects per 100 treatments (Rxs) are illustrated in Figure 15.

The percentage of earning before income, taxes, depreciation and amortization (EBITDA), operating income, gross margin, and overall standard deviation (SD) or sigma for the clinics of Examples 1 and 2 were calculated and compared. Figures 16-23 illustrate this comparison per country for Argentina, Spain and Italy, while Figures 24-26 illustrate a worldwide comparison for Argentina, Spain, Italy, and Portugal (Figure 24 also includes Hungary). Figure 27 further illustrates this comparison for the preceding countries in the world as well as the USA.

Reimbursement per treatment (Rx) for clinics in the USA are shown in Figure 28.

Time was measured for each treatment per patient and for preparing and disinfecting the dialysis machine (monitor) in Examples 1 and 2. The staff time for medical personnel was also measured for administering the treatment in the clinics of Examples 1 and 2, as well for disinfecting and reusing dialyzer cartridges, and for using new dialyzer cartridges (no reuse). Total times for completing the process were also calculated for the patients and clinics in Examples 1 and 2. The preceding time were compared and are shown in Figures 29-41.

The procedural steps for patient treatment, such as for dialysis, can be mapped, recorded, and analyzed. One such mapping is shown in the block flow diagram of Figure 42, where the patient enters the medical treatment facility (patient introduction) and is prepared for treatment, as described previously. Once the treatment is terminated and/or the patient departs, i.e. leaves the medical treatment facility, the used needle and tubing are disconnected from the dialysis machine (monitor undress) and discarded in compliance with government regulations. The monitor (dialysis machine) is then disinfected with heat or a chemical disinfectant. The dialyzer cartridge can be cleaned, e.g. disinfected, and reused (supplies reuse). New dialysis fluid is prepared for the monitor (monitor preparation) when the next patient arrives.

The following conclusions were determined based upon the preceding data, calculations, statistical analysis and comparisons. The delivery of the existing dialysis process could be better controlled for the patients and clinics in Examples 1 and 2. The process output is best at 1 sigma. Variability may have clinical consequences by reducing the overall dose of dialysis. The observed variability may have cost implications by inadequate use of resources, labor, supplies and overhead.

The percentage of earning before income, taxes, depreciation and amortization (EBITDA %) per treatment (Rx) can be affected by the following factors: joint venture, crude mortality, labor cost per Rx, average reimbursement, non compliant, average Kt/V, and the overall clinic standard deviation (SD). The sigma or standard deviation (SD) for the clinics can be effected by the following factors: EBITDA per Rx, labor cost per Rx, SMR, average Kt/V, and non compliance. Each 0.1 increase in overall clinic SD can result in a loss of about $10.00 per Rx to the EBITDA per Rx.

Further conclusions were also determined based upon the preceding data, calculations, statistical analysis and comparisons. The financial output of the process exhibits a wide variation globally. The financial variability correlates with the variability of the process. The model predicts significant improvements in profitability through process control.

The mapping and analysis of the dialysis process can provide a study of the source of variation and of the financial performance variation of the treatment per patient per clinic. Mapping of the dialysis delivery process demonstrates considerable variation on each step for both patient time and staff time. It also gives insight into the errors and their sources during the delivery of the treatment

It is important to have a close understanding of customer needs; disciplined use of facts and data, statistical analysis of data. Attention to management of medical personnel can also be important. It is also useful to further improve processes for enhanced patient treatment and increased profits for the business.

The preceding process, data, calculations, statistical analysis, and comparisons can also provide management with tools with which to evaluate both potential acquisitions and/or existing clinics or other medical facilities for their ability to improve financial and/or patient outcomes. It has been shown for the preceding that the stability and accuracy of dialysis treatment is related to financial and/or patient outcomes.

In Examples 1 and 2, the medical personnel, patients, and clinics comprising the population were selected so that clinics with adequate experience both in regard to the number of patients treated and the length of time at their company would be included. The time period was selected so that clinic-level variables would be more stable. The variables were selected so that they could assist in the explanation of clinic-level profitability. The population included 381 clinics in Examples 1 and 2 that met the following criteria: (1) provided hemodialysis for the entire 2000 calendar year; (2) had at least 30 patient-years (i.e. 30 patients *3 times a week *52 weeks = 4680 treatments); (3) had at least 1 dollar in hemodialysis revenue during the year; (4) were active and owned by Gambro Healthcare during the year; and (5) had either an EBITA per treatment within 3 standard deviations of the mean or an EBITA per treatment greater than 3 standard deviations from the mean that could be explained by extreme values in explanatory variables (e.g. 90 percent diabetic population). The reason for the last criterion was to exclude clinics who had unexpected revenues or costs which would give a distorted view of their profitability. For example, a clinic may have started dialysis January 1, 2000 and incurred start-up costs that would cause the EBITA per treatment for the year to be an inaccurate estimate of their profitability from an ongoing operational perspective. The time period was for the year 2000, from January 1 to December 31, 2000.

The variables in the dataset for Example 2 reflect a multidimensional group of demographic, process, clinical, and financial variables that describe not only clinic profitability but also the intermediate variables that are related to profitability (e.g. hospitalization and mortality). Mapping of variables and their intuitive relationship (denoted by -positive, -negative, or -undetermined) to profitability are shown in Figure 43. The Charleson Comorbidity Index was included as an acuity indicator for Figure 43. It has been found to predict patient outcomes and costs in dialysis patients and is computed by scoring various comorbidity/age combinations. A histogram and box and whisker plot of EBITDA per treatment for the USA clinics in Examples 1 and 2 are shown in Figure 44. Analysis of EBITDA per treatment shows that it has a normal distribution with large variation. Table 3 shows the summary of statistical analysis for all of the variables in Examples 1 and 2. Table 4 shows the correlation of the data, factors, computations and statistical analysis for Examples 1 and 2.

In the tables and figures, the following abbreviations and acronyms have the following meaning: (a) average MOD is average months on dialysis; (b) catheter only percent is the percentage of patients who have a catheter; (c) MCCI are the months of care in the clinic per patient; (d) average weekly EPO is the average weekly dose of erythropoietin, which is a hormone that stimulates red blood cell production; (e) SMR is the standard mortality ratio; (f) LSL is the lower specification limit; (g) USL is the upper specification limit; (h) PTH or pth is parathyroid harmone; (i) employee T/O is turnover of medical personnel (staff); (j) division is a division of the company; (k) pct of commercial is the percentage of patients covered by commercial insurance as compared to Medicare or government insurance; (1) CMR is the crude mortality rate; (m) MCCI is also the Modified Charleson Comorbidity Index; (n) Non-compliance and non-compliant refer to patients who do not follow their physician's instructions or the procedures recommended by the health care provider; (o) MSA is the metropolitan service area; (p) Mo. s. is the number of months the clinic has been providing hemodialysis treatments; (q) source denotes the original or source of the variation (The variation is either explained by the model or the residual, i.e. the difference between the observed and predicted, which is sometimes referred to as a model error); (r) DF is the degrees of freedom associated with model error (i.e. residual) and the regression sum of the square of squares (SSE and SSR); (s) MS is the mean squares which are computed by dividing the sums of squares by the respective degrees of freedom; (t) MSE is the mean square error which can provide an estimate of sigma squared, the variance of the error; and (u) Root MSE is the square root of the mean square error, which can estimate the standard deviation of the error.

Standard deviation is an established statistical formula and provides a measure of how widely data, factors or other values are dispersed (vary) from their average (the mean).

Table 3:

| Statistical Analysis | | | | | | |
|---|---|---|---|---|---|---|
| Variable | N | Mean | Std Dev | Sum | Minimum | Maximum |
| EBIDTA per treatment | 381 | 38.32008 | 28.74880 | 14600 | -40.11000 | 128.90000 |
| months the clinic has been giving dialysis with Gambro | 381 | 42.98688 | 27.68711 | 16378 | 14.00000 | 165.00000 |
| urban MSA (yes=1/no=0) | 381 | 0.80577 | 0.39612 | 307.00000 | 0 | 1.00000 |
| joint venture (yes=1/no=0) | 381 | 0.10761 | 0.31030 | 41.00000 | 0 | 1.00000 |
| number of treatments | 381 | 11119 | 5347 | 4236350 | 4699 | 34477 |
| crude mortality rate(100py) | 372 | 20.38110 | 7.72384 | 7582 | 2.90000 | 52.81000 |
| employee turnover percent | 380 | 26.03316 | 21.62447 | 9893 | 0 | 113.33000 |
| dialyzer cost per treatment | 381 | 6.33866 | 3.96925 | 2415 | 1.15000 | 18.05000 |
| labor cost per treatment | 381 | 60.09420 | 9.51131 | 22896 | 39.28000 | 111.86000 |
| average reimbursement | 381 | 152.64659 | 19.14448 | 58158 | 120.10000 | 244.62000 |
| average weekly epogen (1000s) | 381 | 15.10853 | 3.07374 | 5756 | 9.30164 | 30.38271 |
| iron supplement usage percent | 381 | 60.75525 | 14.45194 | 23148 | 8.39000 | 92.00000 |
| noncompliance percent | 381 | 1.73420 | 1.24559 | 660.73000 | 0.01000 | 7.61000 |
| hospitalization percent | 381 | 3.58475 | 1.28569 | 1366 | 0.28000 | 14.42000 |
| reuse percent | 381 | 51.27472 | 40.64969 | 19536 | 0 | 99.85000 |
| male percent | 381 | 51.90215 | 7.00987 | 19775 | 29.41000 | 70.74000 |
| white percent | 381 | 49.80614 | 29.88320 | 18976 | 0 | 100.00000 |
| average months on dialysis | 381 | 35.99814 | 8.85251 | 137.15 | 13.84000 | 82.30000 |
| modified charleson comorbidity index | 381 | 3.77144 | 0.35146 | 1437 | 2.87000 | 4.73000 |
| commercial percent | 381 | 15.80315 | 10.20760 | 6021 | 1.14000 | 58.25000 |

Table 3: (continued)

| Statistical Analysis | | | | | | |
|---|---|---|---|---|---|---|
| Variable | N | Mean | Std Dev | Sum | Minimum | Maximum |
| kt/v standard deviation | 381 | 0.27592 | 0.03576 | 105.12707 | 0.15338 | 0.39703 |
| kt/v inter-patient standard deviation | 381 | 0.21658 | 0.03791 | 82.51782 | 0.09593 | 0.35073 |
| kt/v intra-patient standard deviation | 381 | 0.18509 | 0.02351 | 70.51897 | 0.12361 | 0.24896 |
| kt/v average | 381 | 1.50810 | 0.09109 | 574.58702 | 1.23066 | 1.82377 |
| hemoglobin average | 381 | 11.47344 | 0.23330 | 4371 | 10.53000 | 12.22000 |
| pth average | 381 | 318.67656 | 94.70221 | 121416 | 117.75000 | 694.14000 |
| albumin average | 381 | 3.75333 | 0.07051 | 1430 | 3.24000 | 3.94000 |
| catheter only percent | 381 | 18.64262 | 9.73770 | 7103 | 2.76000 | 58.80000 |

## Table 4: Correlation of Variables

| Red values denote significant correlation with p <.05 | EBIDTA Per Trmt | Mo.s Giving Dialysis | Joint Venture | Nbr of Treatments | CMR (100py) | Turnover Percent | Dial Cost Per Trmt | Labor Cost Per Trmt | Avg Reimbursement | Average Weekly Epo | Iron Supplement Pct | Noncompliance Pct | Hospitalization Pct | Reuse Percent | Male Percent | White Percent | Average MOD | MCCI | Commercial Percent | Std Dev of KT/V | Catheter Only Pct |
|---|---|---|---|---|---|---|---|---|---|---|---|---|---|---|---|---|---|---|---|---|---|
| EBIDTA per Treatment | | .50 | .14 | .07 | .20 | 04 | .05 | .36 | .51 | .01 | .04 | -.21 | .03 | -.03 | -.12 | .17 | -.06 | .09 | -.03 | -.12 | .00 |
| Mo.s Giving Dialysis | .50 | | .11 | .22 | -.01 | .04 | .19 | -.22 | -.09 | .07 | .09 | .09 | -.01 | -.21 | .09 | -.05 | .18 | -.12 | -.08 | -.01 | -.02 |
| Joint Venture | .14 | .11 | | .09 | -.05. | .03 | .08 | .07 | .15 | .02 | .11 | .04 | .03 | -.12 | .03 | .07 | .00 | .00 | .04 | .02 | .00 |
| Number of Treatments | -.07 | .22 | .09 | | -.20 | .04 | .03 | .20 | .16 | -.03 | .09 | .18 | -.11 | .02 | -.02 | -.16 | .19 | -.13 | .04 | .05 | -.06 |
| CMR (100py) | .20 | -.01 | -.05 | -.20 | | .01 | -.03 | -.11 | .12 | .02 | -.09 | -.24 | .23 | .01 | .06 | .38 | -.23 | .45 | .04 | -.02 | .24 |
| Employee Turnover Pct | .04 | .04 | .03 | .04 | .01 | | -.06 | .16 | .05 | .13 | -.06 | .02 | -.06 | .07 | .02 | -.06 | .04 | -.09 | .04 | .03 | -.06 |
| Dialyzer Cost Per Treatment | -.05 | .19 | .08 | .03 | -.03 | -.06 | | -.06 | .11 | .30 | .00 | .11 | .16 | -.96 | .06 | -.10 | .03 | -.12 | -.10 | .00 | .07 |
| Labor Cost Per Treatment | -.36 | -.22 | .07 | .20 | -.11 | .16 | -.06 | | .23 | .07 | -.11 | .06 | -.01 | .12 | .14 | -.01 | .06 | .03 | .14 | .15 | -.03 |
| Avg Reimbursement | .51 | -.09 | .15 | .16 | .12 | .05 | .11 | .23 | | .02 | -.10 | -.17 | -.02 | -.12 | .03 | .19 | -.09 | .07 | .11 | .01 | .03 |
| Avg Weekly Epogen (1000) | .01 | .07 | .02 | -.03 | .02 | .13 | .30 | .07 | .02 | | -.03 | .24 | .33 | -.32 | -.02 | -.20 | -.03 | -.10 | -.05 | .08 | .11 |
| Iron Supplement Percent | .04 | .09 | .11 | .09 | -.09 | -.06 | .00 | -.11 | -.10 | -.03 | | .11 | -.05 | .00 | -.05 | -.10 | .09 | -.09 | -.02 | .03 | -.03 |
| Noncompliant Percent | -.21 | .09 | .04 | .18 | -.24 | .02 | .11 | .06 | -.17 | .24 | .11 | | -.09 | -.08 | -.08 | -.54 | .17 | -.59 | -.16 | .20 | .03 |
| Hospital Pct | .03 | -.01 | .03 | -.11 | .23 | -.06 | .16 | -.01 | -.02 | .33 | -.05 | -.09 | | -.15 | -.04 | -.11 | -.08 | -.01 | -.14 | .02 | .27 |
| Reuse Percent | -.03 | -.21 | -.12 | .02 | .01 | .07 | -.96 | .12 | -.12 | .32 | .00 | -.08 | -.15 | | -.05 | .08 | -.04 | .10 | .13 | .04 | -.04 |
| Male Percent | -.12 | .09 | .03 | -.02 | .06 | .02 | .06 | .14 | .03 | -.02 | -.05 | -.08 | -.04 | -.05 | | .20 | -.04 | .14 | .23 | .05 | .04 |
| White Percent | .17 | -.05 | .07 | -.16 | .38 | -.06 | -.10 | -.01 | .19 | -.20 | -.10 | -.54 | -.11 | .08 | .20 | | -.44 | .61 | .20 | .03 | .12 |
| Average MOD | -.06 | .18 | .00 | .19 | -.23 | .04 | .03 | .06 | -.09 | -.03 | .09 | .17 | -.08 | -.04 | -.04 | -.44 | | .39 | -.23 | -.05 | -.23 |
| MCCI | .09 | -.12 | .00 | -.13 | .45 | -.09 | -.12 | .03 | .07 | -.10 | -.09 | -.59 | -.01 | .10 | .14 | .61 | -.39 | | .16 | -.08 | .10 |
| Commercial Percent | -.03 | -.08 | -.04 | .04 | .04 | .04 | -.10 | .14 | .11 | -.05 | -.02 | -.16 | -.14 | .13 | .23 | .20 | .23 | .16 | | .11 | .03 |
| Std Dev of KT/V | -.12 | -.01 | -.02 | .05 | -.02 | .03 | .00 | .15 | .01 | .08 | .03 | .20 | .02 | .04 | .05 | .03 | -.05 | -.08 | .11 | | .03 |
| Catheter Only Percent | .00 | -.02 | .00 | -.06 | .24 | -.06 | .07 | -.03 | .03 | .11 | -.03 | .03 | .27 | -.04 | .04 | .12 | .23 | .10 | .03 | .03 | |

Note that some intermediate outcome (avg kt/v, avg alb, avg pth, avg hgb), division, and urban MSA variables are not included in this table because of size constraint.

Correlation analysis of Table 4 includes the following. The correlation coefficient (r) measures the strength of a relationship between two variables. It varies between -1 (perfect inverse relationship) and 1 (perfect positive relationship) and a small or 0 coefficient tells us that the two variables are not linearly related. If two variables X and Y are correlated it can mean one of four things: (1) X causes Y; (2) Y causes X; (3) a third factor or a group of factors, either directly or indirectly, causes X and/or Y; and (4) the correlation occurred by random chance but is not truly significant. Marginal effect is the effect of a particular explanatory variable while holding all other variables constant.

Regression analysis is the analysis of the relationship between one variable and another set of variables. The relationship is expressed as an equation that predicts a response variable (also called a dependent variable) from a function of regressor variables (also called independent variables, predictors, explanatory variables, factors, or drivers) and parameters. A regression model can be an excellent predictor of the response if the model is carefully formulated from a large sample, as has been done for the process.

Model 1: To consider a simple example first, EBITDA was modeled per treatment against the standard deviation of kt/v to determine if there is a significant relationship between process stability and financial outcomes, as shown in Table 5. The analysis shows that there is a significant relationship between the two variables (p = 0.0153) but that it is weak ($R^2$ = .02), signifying there are other variables that influence EBITDA per treatment besides stability of kt/v. We also found that each 0.1 increase in the standard deviation results in a loss of about $10.00 per treatment.

Table 5: EBITA per Treatment vs Standard Deviation of kt/v

The REG Procedure

Model: MODEL 1

Dependent Variable: EBITA_per_trtmt

Analysis of Variance

| Source | DF | Sum of Squares | Mean Square | F Value | Pr > F |
|---|---|---|---|---|---|
| Model | 1 | 4845.05281 | 4845.05281 | 5.4 | 0.0153 |
| Error | 379 | 309223 | 815.89067 | | |
| Corrected Total | 380 | 314068 | | | |

| | | | |
|---|---|---|---|
| Root MSE | 28.56380 | R-Square | 0.0154 |
| Dependent Mean | 38.32008 | Adj R-Sq | 0.0128 |
| Coeff Var | 74.54003 | | |

Parameter Estimates

| Variable | DF | Parameter Estimate | Standard Error | t Value | Pr > t |
|---|---|---|---|---|---|
| Intercept | 1 | 65.87184 | 11.40049 | 5.78 | <.0001 |
| sd_ktv | 1 | -99.85270 | 40.97571 | -2.44 | 0.0153 |

Interpreting $R^2$: $R^2$ is usually defined as the proportion of variance of the response that is predictable from (that can be explained by) the regressor variables. It may be easier to interpret the square root of (1 - $R^2$), which is approximately the factor by which the standard error of prediction is reduced by the introduction of the regressor variables.

Interpreting p-values: Probability values (p-values) do not necessarily measure the importance of a regressor especially in a nonexperimental setting. Therefore, p-values are relative in nonexperimental regression and should not be used as the only criterion for final selection into a model.

Interpreting Parameter Estimates: In an observational study, parameter estimates can be interpreted as the expected difference in the response of two observations that differ by one unit on the variable in question and that have the same values for all other regressors (i.e. the marginal effect of the explanatory variable).

Effect of Correlated Explanatory Variables: If the explanatory variables are correlated, it becomes difficult to disentangle the effects of one variable from another, and the parameter estimates may be highly dependent on which explanatory variables are used in the model. Two correlated variables may be nonsignificant when tested separately but highly significant when considered together and if two regressors have a correlation of 1.0, it is impossible to separate their effects.

Table 6 is a model of the standard deviation (SD) of Kt/V versus various clinical and demographic variables.

Table 6: Std dev of kt/v vs Various Clinical & Demographic Variables

The REG Procedure

Model: MODEL 1

Dependent Variable: sd_ktv

Analysis of Variance

| Source | DF | Sum of Squares | Mean Square | F Value | Pr > F |
|---|---|---|---|---|---|
| Model | 9 | 0.12000 | 0.01333 | 13.76 | <.0001 |
| Error | 370 | 0.35842 | 0.00096870 | | |
| Corrected Total | 379 | 0.47842 | | | |

| | | | |
|---|---|---|---|
| Root MSE | 0.03112 | R-Square | 0.2508 |
| Dependent Mean | 0.27570 | Adj R-Sq | 0.2326 |
| Coeff Var | 11.28925 | | |

Parameter Estimates

| Variable | DF | Parameter Estimate | Standard Error | t Value | Pr > t | Variance Inflation |
|---|---|---|---|---|---|---|
| Intercept | 1 | 0.02465 | 0.03774 | 0.65 | 0.5140 | 0 |
| N_C | 1 | -0.01279 | 0.00452 | -2.83 | 0.0049 | 1.78472 |
| S_E | 1 | -0.02442 | 0.00435 | -5.62 | <.0001 | 1.78285 |
| noncompliance_pct | 1 | 0.01014 | 0.00140 | 7.26 | <.0001 | 1.18778 |
| hospital_pct | 1 | 0.00427 | 0.00136 | 3.14 | 0.0018 | 1.19343 |
| male_pct | | 0.00061028 | 0.00024339 | 2.51 | 0.0126 | 1.13946 |
| commercial_pct | 1 | 0.00044919 | 0.00017423 | 2.58 | 0.0103 | 1.23439 |
| avg_ktv | 1 | 0.13177 | 0.02005 | 6.57 | <.0001 | 1.30698 |
| turnover_pct | 1 | 0.00013780 | 0.00007527 | 1.83 | 0.0679 | 1.03652 |
| MSA | 1 | -0.01094 | 0.00449 | -2.43 | 0.0154 | 1.24173 |

The following other variables were eligible to be entered into the model but were not found to significantly affect the stability of kt/v: months_giving_dial, treatments, mcci, cmr_100py_hd, white_pct, avg_mod, cath_pct, JV, iron_pct, and reuse_pct.

The regression model in Table 5 shows that there is a significant relationship between the standard deviation of kt/v with EBITA per treatment but the model does not take into account the correlation between other possible variables and EBITA per treatment or with the standard deviation of kt/v itself. Table 6 shows that the standard deviation of kt/v is related with division, percent of males, percent of commercial primary insurance, actual kt/v delivered, and other variables that are proxies for patient management.

Model 2 and 3: To gain a better understanding of what variables impact profitability, multivariate models were formed relating EBITDA per treatment with various clinical, demographic, and process variables (this model includes all variables except those from the General Ledger, albumin, PTH, and Hemoglobin). Model 2 is shown in Table 7 and excludes division variables. Model 3 is shown in Table 8 and includes division variables. The models in Tables 7 and 8 are useful for: examining potential acquisitions and new clinics and thus will need to include division variables; and examining existing clinics and thus will need to exclude division variables because we cannot affect the location of an existing clinic.

Table 7: EBITA per Treatment vs Process, Demographic, Clinical & Financial Variables (Excluding Geographic Variable -- Division)

The REG Procedure

Model: MODEL 2

Dependent Variable: EBITA_per_treatment

Analysis of Variance

| Source | DF | Sum of Squares | Mean Square | F Value | Pr > F |
|---|---|---|---|---|---|
| Model | 11 | 182054 | 16550 | 49.67 | <.0001 |
| Error | 359 | 119609 | 333.17363 | | |
| Corrected Total | 370 | 301664 | | | |

| | | | |
|---|---|---|---|
| Root MSE | 18.25304 | R-Square | 0.6035 |
| Dependent Mean | 38.24381 | Adj R-Sq | 0.5914 |
| Coeff Var | 47.72889 | | |

Parameter Estimates

| Variable | DF | Parameter Estimate | Standard Error | t Value | Pr > t | Variance Inflation |
|---|---|---|---|---|---|---|
| Intercept | 1 | 4.48594 | 17.23680 | 0.26 | 0.7948 | 0 |
| cmr_100py_hd | 1 | 6.47439 | 3.14840 | 2.06 | 0.0405 | 1.08501 |
| turnover_pct | 1 | 0.13015 | 0.04527 | 2.88 | 0.0043 | 1.04897 |
| dialyzer_cost_per_trtmt | 1 | -3.13018 | 0.82797 | -3.78 | 0.0002 | 12.07803 |
| labor_cost_per_trtmt | 1 | -1.50980 | 0.11108 | -13.59 | <.0001 | 1.25251 |
| avg_reimbursement | 1 | 1.02978 | 0.05697 | 18.08 | <.0001 | 1.16328 |
| avg_wkly_epo_1000 | 1 | 0.53626 | 0.33663 | 1.59 | 0.1120 | 1.18509 |
| iron_pct | 1 | 0.11975 | 0.06751 | 1.77 | 0.0769 | 1.05967 |
| male_pct | 1 | -0.24428 | 0.13945 | -1.75 | 0.0807 | 1.04044 |
| sd_ktv | 1 | -51.77912 | 27.32598 | | 0.0589 | 1.04909 |
| reuse_pct | 1 | -0.19868 | 0.08303 | -2.39 | 0.0172 | 12.67427 |

**The following other variables were eligible to be entered into the model but were not found to significantly affect the EBITDA per treatment: months_giving_dial, treatments, MSA, treatments, noncompliance_pct, hospital_pct, white_pct, avg_mod, mcci, commercial_pct, avg_ktv, and cath_pct.**

The preceding analysis shows that there is a significant relationship between the explanatory variables and EBITDA per treatment ($p < 0.0001$). Model 2 explains about 60 percent of the variation between clinics ($R^2 = .60$).

Interpretation of parameter estimates shows that: (a) being a joint venture - an increase of $6.47 per treatment;

(b) Having one more death per 100 patient years - an increase of $0.26 per treatment; (c) Having one more percent of employee turnover - an increase of $0.13 per treatment; (d) an increase in dialyzer cost per treatment of $1 - a loss of $3.13 per treatment; (e) an increase in labor cost per treatment of $1 - a loss of $1.51 per treatment; (f) an increase in average reimbursement of $1 - an increase of $1.03 per treatment; (g) an increase in average weekly epogen (in 1000s) - an increase of $0.54 per treatment; (h) having one more percent of iron supplement usage - an increase of $0.12 per treatment; (i) a 0.1 increase in the standard deviation of kt/v - a loss of $5.18 per treatment; and (j) having one more percent of reuse - a loss of $0.20 per treatment.

Table 8: EBITA per Treatment vs Process, Demographic, Clinical & Financial Variables (Including Geographic Variable -- Division)

The REG Procedure

Model: MODEL 3

Dependent Variable: EBITA_per_treatment

Analysis of Variance

| Source | DF | Sum of Squares | Mean Square | F Value | Pr > F |
|---|---|---|---|---|---|
| Model | 10 | 185763 | 18576 | 57.70 | <.0001 |
| Error | 360 | 115901 | 321.94730 | | |
| Corrected Total | 370 | 301664 | | | |

| | | | |
|---|---|---|---|
| Root MSE | 17.94289 | R-Square | 0.6158 |
| Dependent Mean | 38.24318 | Adj R-Sq | 0.6051 |
| Coeff Var | 46.91788 | | |

Parameter Estimates

| Variable | DF | Parameter Estimate | Standard Error | t Value | Pr > t | Variance Inflation |
|---|---|---|---|---|---|---|
| Intercept | 1 | -2.39259 | 12.60050 | -0.19 | 0.8495 | 0 |
| N-C | 1 | 13.37534 | 2.61069 | 5.12 | <.0001 | 1.72614 |
| S_E | 1 | 6.46338 | 2.55082 | 2.53 | 0.0117 | 1.80967 |
| JV | 1 | 6.83822 | 3.06409 | 2.23 | 0.0262 | 1.06351 |
| cmr_100py_hd | 1 | 0.25135 | 0.12768 | 1.97 | 0.0498 | 1.12039 |
| Turnover_pct | 1 | 0.11105 | 0.04497 | 2.47 | 0.0140 | 1.07125 |
| Dialyzer_cost_per_trmt | 1 | -1.32937 | 0.24451 | -5.44 | <.0001 | 1.09009 |
| labor_cost_per_trmt | 1 | -1.61179 | 0.11289 | -14.28 | <.0001 | 1.33876 |
| avg_reimbursement | 1 | 0.99683 | 0.05797 | 17.20 | <.0001 | 1.24652 |
| male_pct | 1 | -0.34219 | 0.13758 | -2.49 | 0.0133 | 1.04805 |
| cath_pct | 1 | -0.20277 | 0.09958 | -2.04 | 0.0425 | 1.08828 |

**The following other variables were eligible to be entered into the model but were not found to significantly affect the EBITDA per treatment: months_giving_dial, treatments, MSA, treatments,avg_wkly_epo_1000, noncompliance_pct, hospital_pct, white_pct, avg_mod, mcci, commercial_pct, avg_ktv, sd_ktv, and reuse_pct.**

The preceding analysis shows that there is a significant relationship between the explanatory variables and EBITDA per treatment ($p < 0.0001$) and that Model 3 explains about 62 percent of the variation between clinics ($R^2 = .62$)

Interpretation of the parameter estimates shows that: (a) being in the North Central division - an increase of $13.38 per treatment; (b) being in the South East division - an increase of $6.46 per treatment; (c) being in the West division - a loss of about $20.00 per treatment; (d) being a joint venture - an increase of $6.84 per treatment; (e) having one more death per 100 patient years - an increase of $0.25 per treatment; (f) having one more percent of employee turnover - an increase of $0.11 per treatment; (g) having dialyzer cost per treatment go up $1 - a loss of $1.33 per treatment; (h) having labor cost per treatment go up $1- a loss of $1.61 per treatment; (i) having the average reimbursement go up $1 - an increase of $1.00 per treatment; (j) having one more percent males - a loss of $0.34 per treatment; and (k) having one more percent of catheters only - a loss of $0.20 per treatment.

The analysis shows that there is a set of variables (division, joint venture, crude mortality, acuity, employee turnover, noncompliance, percent male, percent white, reimbursement, and dialyzer and labor cost) that can help predict clinic profitability stability and accuracy of delivering dialysis is also related to profitability. Furthermore, the process/ patient management variables were highly correlated, so that clinics or other medical treatment facilities that have inadequate nutrition management are more likely to have inadequate access, anemia, and adequacy management (and visa versa).

The invention also provides an apparatus which has means for inputting the various identified parameters and means for processing the parameters to provide the performance assessment. The apparatus also comprises output means for outputting the performance assessment. The apparatus preferably comprises a computer having a keyboard, mouse and monitor for these purposes.

The computer is preferably instructed by a computer program stored on a computer media such as a CD-ROM, floppy disk, RAM, ROM or hard drive.

## Claims

**1.** A computer implemented process for determining the performance of clinics and other medical facilities to enhance care and treatment of patients having to undergo repetitive extracorporeal blood treatments, said facility comprising a medical facility selected from the group consisting of a clinic, a hospital, a center for medical treatment, a patient treatment facility of a health care provider, and an office of a physician;

said process comprising the steps of:

measuring the effectiveness of each treatment per patient using a parameter indicative of blood purification;
measuring the efficiency of each treatment per patient by determining the time for each treatment of each patient;
determining the frequency of said treatments for each patient;
determining costs of said treatments per patient;
calculating total costs of said treatments for each patients;
calculating variations of effectiveness of said treatments for each patient;
calculating variations of efficiency of said treatments for each patient;
calculating variations of costs of said treatments for each patient;
identifying demographics of each facility including the geographical location of each facility;
inputting data into a central processing unit (CPU);
said data comprising said measured effectiveness and efficiency of said treatments per patient, said frequency of treatment per patient, said costs of said treatment per patient, said variations in effectiveness, efficiency and costs of said treatment per patient, said patient characteristics, and said demographics of each facility;
storing said data in said CPU;
retrieving data from said CPU; and
statistically analysing said data with said CPU to complete a sigma comprising a standard deviation around a mean of said data and correlating said data to determine the performance of said facility.

**2.** A process as in claim 1 wherein:

said facility is selected from the group consisting of a medical treatment facility and a home of a patient; and said medical treatment facility is selected from the group consisting of a clinic, a hospital, a center for medical treatment, a patient treatment facility of a health care provider, and an office of a physician.

**3.** A process as in claim 1 or 2, wherein the effectiveness of each treatment is measured according to the mathematical model KT/V wherein K = clearance, T = time of said treatment, V=body distribution volume of urea or creatinine, and the clearance of a substance being defined as the volume of blood from which the substance is completely removed by unit of time.

**4.** A process as in any one of the preceding claims wherein said statistically analysing further comprises calculating a standard deviation for said effectiveness of said treatment by statistical computation, selected from the group consisting of calculating a standard deviation for KT/V for each patient, calculating a standard deviation for inter patient KT/V, and calculating a standard deviation for intra patient KT/V.

**5.** A process as in any one of the preceding claims wherein:

said CPU is selected from the group consisting of a microprocessor, computer, main frame computer, server computer, desktop computer, workstation computer, notebook computer, laptop computer, notebook computer, palm pilot-type computer, computer chip, integrated circuit, electronic controller, network, internet, and global communications network.

**6.** A process as in any one of the preceding claims including:

electronically calculating financial results of said process for said treatment of said patients at said facilities with said CPU;
said financial results are selected from the group consisting of: earnings, operating income, gross income, net income, gross margin, net margin, profits, EBITA and EBITDA;
said EBITA comprising earnings before interest, taxes and amortization; and
said EBITDA comprising earnings before interest, taxes, depreciation and amortization.

**7.** A process as in any one of the preceding claims wherein:

said data further comprises supplemental data selected from the group consisting of facility data, patient data, and cost data;
said facility data comprising demographic information selected from the group consisting of: metropolitan statistical area of each facility defining an urban MSA, ownership of each facility, type of ownership of each facility including company owned and joint venture facilities, length of service of each facility, hospitalization of patients, division, and employee turnover (T/O) at each facility;
said patient data comprising patient information determined from each patient, selected from the group consisting of: type of treatment per patient, duration (months) of treatments per patient, percentage of patients having said treatment as a primary cure for their ailments, race of patients, ethnic background of each patient; haemoglobin per patient, albumen per patient, catheter usage per patient, equipment usage per patient, temperature conditions during treatment, humidity conditions during treatment, type of equipment and supplies, composition of dialysis fluids, non-compliance per patient, iron supplement usage per patient, epogen usage per patient, crude mortality rate (CMR) of patients in said facility, average months on dialysis (MOD) per patient, modified charleson comorbidity index (MCCI); and
said cost data comprising financial data selected from the group consisting of equipments costs per treatment, dialyzer costs per treatment, costs of supplies per treatment, costs for sterilizing dialysis equipment for said treatments, savings and costs for reuse of equipment for said treatment, labor costs per treatment, overhead per facility, percentage of patients covered by commercial insurance, reimbursement of medicare for said treatments, reimbursements from government agencies for said treatments, and reimbursements form insurance companies for said treatments.

**8.** A process as in any one of the preceding claims, including mapping of said process.

**9.** A process as in any one of the preceding claims, including operating said process at a standard deviation (sigma) of about one.

**10.** A process as in any one of the preceding claims, wherein said treatment is selected from the group consisting of hemodialysis and dialysis.

**11.** Apparatus for providing a measure of the performance of a medical facility which treats patients having to undergo repetitive extracorporeal blood treatements, said apparatus comprising:

input means adapted to permit the inputting of data comprising:

measured effectiveness and efficiency of said treatments per patient by using a parameter indicative of blood purification , frequency of treatment per patient, costs of said treatment per patient, variations in effectiveness, efficiency and costs of said treatment per patient, patient characteristics, and demographics of each facility;
storage means adapted to store said data;
processor means adapted to statistically analyse said stored data to compute a sigma comprising a standard deviation around a mean of said data and to correlate said data to determine the performance of said facility.

**12.** Apparatus according to claim 11, wherein said apparatus is selected from the group consisting of a microprocessor, computer, main frame computer, server computer, desktop computer, workstation computer, notebook computer, laptop computer, notebook computer, palm pilot-type computer, computer chip, integrated circuit, electronic controller, network, internet, and global communications network.

**13.** Apparatus according to claim 11 or 12, wherein the effectiveness of each treatment is measured according to the mathematical model KT/V wherein K = clearance, T = time of said treatment, and V=body distribution volume of urea or creatinine and further wherein said processor is arranged to calculate a standard deviation for said effectiveness of said treatment by statistical computation, selected from the group consisting of calculating a standard deviation for KT/V for each patient, calculating a standard deviation for inter patient KT/V, and calculating a standard deviation for intra patient KT/V.

**14.** Apparatus according to claim 13, wherein said processor is further arranged to perform the calculation KT/V to determine the effectiveness of each treatment.

**15.** A computer program which, when loaded on a computer, instructs the computer to perform the steps of the method of any one of claims 1 to 10.

**16.** The computer program of claim 15, embodied on a computer readable medium.

**Patentansprüche**

**1.** Computerimplementiertes Verfahren zum Bestimmen der Leistung von Kliniken oder anderen medizinischen Einrichtungen, um die Versorgung und die Behandlung von Patienten zu verbessern, die sich wiederholten extrakorporalen Blutbehandlungen unterziehen müssen, wobei die Einrichtung eine medizinische Einrichtung umfasst, die aus der Gruppe ausgewählt ist, die aus einer Klinik, einem Krankenhaus, einem medizinischen Behandlungszentrum, einer Patientenbehandlungseinrichtung eines Gesundheitsfürsorgeanbieters und aus einer Arztpraxis besteht;
wobei das Verfahren die folgenden Schritte umfasst:

Messen der Effektivität jeder Behandlung pro Patienten unter Verwendung eines die Blutreinigung angebenden Parameters;
Messen des Wirkungsgrades jeder Behandlung pro Patienten durch Bestimmen der Zeit für jede Behandlung jedes Patienten;
Bestimmen der Häufigkeit der Behandlungen für jeden Patienten;
Bestimmen der Kosten der Behandlungen pro Patienten;
Berechnen der Gesamtkosten der Behandlungen für jeden Patienten;
Berechnen von Effektivitätsschwankungen der Behandlungen für jeden Patienten;
Berechnen von Wirkungsgradschwankungen der Behandlungen für jeden Patienten;
Berechnen von Kostenschwankungen der Behandlungen für jeden Patienten;
Identifizieren der Demographie jeder Einrichtung einschließlich des geographischen Ortes jeder Einrichtung;
Eingeben von Daten in eine Zentraleinheit (CPU);

wobei die Daten die gemessene Effektivität und den gemessenen Wirkungsgrad der Behandlungen pro Patienten, die Behandlungshäufigkeit pro Patient, die Kosten der Behandlung pro Patient, die Effektivitäts-, Wirkungsgrad- und Kostenschwankungen der Behandlung pro Patienten, die Patienteneigenschaften und die Demographie jeder Einrichtung umfassen;
Speichern der Daten in der CPU;
Wiedergewinnen von Daten aus der CPU; und
statistisches Analysieren der Daten mit der CPU, um eine Summe (Sigma), die eine Standardabweichung um einen Mittelwert der Daten enthält, zu vervollständigen und um die Daten zu korrelieren, um die Leistung der Einrichtung zu bestimmen.

**2.** Verfahren nach Anspruch 1, bei dem

die Einrichtung aus der Gruppe ausgewählt ist, die aus einer medizinischen Behandlungseinrichtung und aus einem Haus eines Patienten besteht; und

die medizinische Behandlungseinrichtung aus der Gruppe ausgewählt ist, die aus einer Klinik, einem Krankenhaus, einem medizinischen Behandlungszentrum, einer Patientenbehandlungseinrichtung eines Gesundheitsfürsorgeanbieters und einer Arztpraxis besteht.

**3.** Verfahren nach Anspruch 1 oder 2, bei dem die Effektivität jeder Behandlung entsprechend dem mathematischen Modell (KT/V) gemessen wird, wobei K = Reinigungswirkung, T = Behandlungszeit, V = Körperverteilungsvolumen von Harnstoff oder Creatinin sind, wobei die Reinigungswirkung einer Substanz als das Blutvolumen pro Zeiteinheit, aus der die Substanz vollständig entfernt ist, definiert ist.

**4.** Verfahren nach einem der vorhergehenden Ansprüche, bei dem das statistische Analysieren ferner das Berechnen einer Standardabweichung für die Effektivität der Behandlung durch statische Berechnung umfasst, die aus der Gruppe ausgewählt ist, die aus dem Berechnen einer Standardabweichung von KT/V für jeden Patienten, dem Berechnen einer Standardabweichung von KT/V zwischen Patienten und dem Berechnen einer Standardabweichung von KT/V in einem Patienten besteht.

**5.** Verfahren nach einem der vorhergehenden Ansprüche, bei dem:

die CPU aus der Gruppe ausgewählt ist, die besteht aus einem Mikroprozessor, einem Computer, einem Großrechner, einem Server-Computer, einem Desktop-Computer, einem Workstation-Computer, einem Notebook-Computer, einem Laptop-Computer, einem Notebook-Computer, einem Computer des Palm Pilot-Typs, einem Computerchip, einer integrierten Schaltung, einer elektronischen Steuereinheit, einem Netz, dem Internet und einem globalen Kommunikationsnetz.

**6.** Verfahren nach einem der vorhergehenden Ansprüche, das umfasst:

elektronisches Berechnen finanzieller Resultate des Verfahrens für die Behandlung der Patienten in den Einrichtungen mit der CPU;

wobei die finanziellen Ergebnisse aus der Gruppe ausgewählt sind, die besteht aus: Einkommen, Betriebseinkommen, Bruttoeinkommen, Nettoeinkommen, Bruttoertrag, Nettobetrag, Gewinn, EBITA und EBITDA;

wobei EBITA Ergebnis vor Zinsen, Steuern und Amortisierung umfasst; und

wobei EBITDA Ergebnis vor Zinsen, Steuern, Abschreibung und Amortisierung umfasst.

**7.** Verfahren nach einem der vorhergehenden Ansprüche, bei dem:

die Daten ferner ergänzende Daten umfassen, die aus der Gruppe ausgewählt sind, die aus Einrichtungsdaten, Patientendaten und Kostendaten bestehen;

wobei die Einrichtungsdaten demographische Informationen umfassen, die aus der Gruppe ausgewählt sind, die besteht aus: statistischer städtischer Bereich jeder Einrichtung, die eine städtische MSA definiert, Eigentumsrecht jeder Einrichtung, Typ des Eigentumsrechts jeder Einrichtung einschließlich gesellschaftseigener Einrichtungen und Joint-Venture-Einrichtungen, Dienstdauer jeder Einrichtung, Hospitalisierungsgrad von Patienten, Abteilung sowie Angestelltenfluktuation (T/O) in jeder Einrichtung;

wobei die Patientendaten Patienteninformationen umfassen, die von jedem Patienten bestimmt werden und aus der Gruppe ausgewählt sind, die besteht aus: Behandlungstyp pro Patienten, Dauer (Monate) der Behandlungen pro Patienten, Prozentsatz von Patienten, bei denen die Behandlung die Hauptbehandlung für ihre Leiden ist, Rasse der Patienten, ethnischer Hintergrund jedes Patienten, Hämoglobin pro Patienten, Albumin pro Patienten, Katheternutzung pro Patienten, Gerätenutzung pro Patienten, Temperaturbedingungen während der Behandlung, Feuchtigkeitsbedingungen während der Behandlung, Typ der Geräte und Verabreichungen, Zusammensetzung von Dialysefluiden, Nichteinhaltung pro Patienten, Eisenverabreichungsnutzung pro Patienten, Epogen-Nutzung pro Patienten, grobe Sterblichkeitsrate (CMR) von Patienten in der Einrichtung, durchschnittliche Monate an der Dialyse (MOD) pro Patienten, modifizierter Charleson-Comorbiditäts-Index (MCCI); und

wobei die Kostendaten finanzielle. Daten umfassen, die aus der Gruppe ausgewählt sind, die besteht aus Gerätekosten pro Behandlung, Dialyseeinrichtungskosten pro Behandlung, Verabreichungskosten pro Behandlung, Kosten für die Sterilisierung der Dialysegeräte für die Behandlungen, Einsparungen und Kosten für die Wiederverwendung von Geräten für die Behandlung, Arbeitskosten pro Behandlung, Kosten pro Einrichtung, Prozentsatz der Patienten, die durch eine private Versicherung geschützt sind, Erstattung der Gesundheitsfürsorge für die Behandlungen, Erstattungen von Regierungsämtern für die Behandlungen und Erstattungen von Versicherungsgesellschaften für die Behandlungen.

**8.** Verfahren nach einem der vorhergehenden Ansprüche, das .die Abbildung (Mapping) des Verfahrens umfasst.

**9.** Verfahren nach einem der vorhergehenden Ansprüche, das das Betreiben des Verfahrens mit einer Standardabweichung (Sigma) von etwa eins umfasst.

**10.** Verfahren nach einem der vorhergehenden Ansprüche, bei dem die Behandlungen aus der Gruppe ausgewählt sind, die aus Hämodialyse und Dialyse besteht.

**11.** Vorrichtung für die Schaffung eines Maßes der Leistung einer medizinischen Einrichtung, die Patienten behandelt, die sich wiederholten extrakorporalen Blutbehandlungen unterziehen müssen, wobei die Vorrichtung umfasst:

Eingabemittel, die so beschaffen sind, dass sie die Eingabe von Daten ermöglichen, die ihrerseits umfassen:

gemessene Effektivität und gemessener Wirkungsgrad der Behandlungen pro Patienten unter Verwendung eines die Blutreinigung angebenden Parameters, Häufigkeit der Behandlung pro Patienten, Kosten der Behandlung pro Patienten, Effektivitäts-, Wirkungsgrad- und Kostenschwankungen der Behandlung pro Patienten, Patienteneigenschaften und Demographie jeder Einrichtung;
Speichermittel, die so beschaffen ist, dass sie die Daten speichert;
Prozessormittel, die so beschaffen sind, dass sie die gespeicherten Daten statistisch analysieren, um eine Summe (Sigma) zu berechnen, die eine Standardabweichung um einen Mittelwert der Daten enthält, und um die Daten zu korrelieren, um die Leistung der Einrichtung zu bestimmen.

**12.** Vorrichtung nach Anspruch 11, wobei die Vorrichtung aus der Gruppe ausgewählt ist, die besteht aus einem Mikroprozessor, einem Computer, einem Großrechner, einem Server-Computer, einem Desktop-Computer, einem Workstation-Computer, einem Notebook-Computer, einem Laptop-Computer, einem Notebook-Computer, einem Computer des Palm Pilot-Typs, einem Computerchip, einer integrierten Schaltung, einer elektronischen Steuereinheit, einem Netz, dem Internet und einem globalen Kommunikationsnetz.

**13.** Vorrichtung nach Anspruch 11 oder 12, bei der die Effektivität jeder Behandlung entsprechend dem mathematischen Modell KT/V gemessen wird, wobei K = Reinigungswirkung, T = Behandlungszeit und V = Körperverteilungsvolumen von Harnstoff oder Creatinin sind und bei der ferner der Prozessor so beschaffen ist, dass er eine Standardabweichung für die Effektivität der Behandlung durch statistische Berechnung berechnet, die aus der Gruppe ausgewählt ist, die aus dem Berechnen einer Standardabweichung von KT/V für jeden Patienten, dem Berechnen einer Standardabweichung von KT/V zwischen Patienten und dem Berechnen einer Standardabweichung von KT/V im selben Patienten besteht.

**14.** Vorrichtung nach Anspruch 13, bei der der Prozessor ferner so beschaffen ist, dass er die KT/V-Berechnung ausführt, um die Effektivität jeder Behandlung zu bestimmen.

**15.** Computerprogramm, das, wenn es in einen Computer geladen ist, den Computer anweist, die Schritte des Verfahrens nach einem der Ansprüche 1 bis 10 auszuführen.

**16.** Computerprogramm nach Anspruch 15, das in einem computerlesbaren Medium verkörpert ist.

## Revendications

**1.** Procédé implémenté par ordinateur pour déterminer les performances de cliniques et de d'autres installations médicales afin d'améliorer les soins et le traitement de patients devant subir des traitements sanguins extracorporels répétitifs, ladite installation comprenant une installation médicale sélectionnée dans le groupe consistant en une clinique, un hôpital, un centre de traitement médical, une installation de traitement de patient d'un fournisseur de soins de santé et un cabinet d'un médecin ;

ledit procédé comprenant les étapes consistant à :

mesurer l'efficacité de chaque traitement par patient en utilisant un paramètre indicatif d'une purification du sang ;
mesurer le rendement de chaque traitement par patient en déterminant le temps pour chaque traitement de chaque patient ;
déterminer la fréquence desdits traitements pour chaque patient ;
déterminer des coûts desdits traitements par patient ;
calculer des coûts totaux desdits traitements pour chaque patient ;
calculer des variations d'efficacité desdits traitements pour chaque patient ;
calculer des variations de rendement desdits traitements pour chaque patient ;
calculer des variations des coûts desdits traitements pour chaque patient ;
identifier des données démographiques pour chaque installation comprenant l'emplacement géographique de chaque installation ;
entrer des données dans une unité centrale (UC) ;

lesdites données comprenant ladite efficacité et ledit rendement mesurés desdits traitements par patient, ladite fréquence de traitement par patient, lesdits coûts dudit traitement par patient, lesdites variations d'efficacité, de rendement et de coûts dudit traitement par patient, lesdites caractéristiques des patients et lesdites données démographiques de chaque installation ;

mémoriser lesdites données dans ladite UC ;

extraire les données de ladite UC ; et

analyser statistiquement lesdites données avec l'UC pour réaliser un sigma comprenant un écart type autour d'une moyenne desdites données et corréler lesdites données afin de déterminer les performances de ladite installation.

**2.** Procédé selon la revendication 1, dans lequel :

ladite installation est sélectionnée dans le groupe consistant en une installation de traitement médical et un domicile d'un patient ; et
ladite installation de traitement médical est sélectionnée dans le groupe consistant en une clinique, un hôpital, un centre de traitement médical, une installation de traitement de patient d'un fournisseur de soins de santé et un cabinet d'un médecin.

**3.** Procédé selon la revendication 1 ou 2, dans lequel l'efficacité de chaque traitement est mesurée selon le modèle mathématique KT/V, où K = épuration, T = temps dudit traitement, V = volume de répartition dans le corps d'urée ou de créatinine et l'épuration d'une substance étant définie comme le volume de sang duquel la substance est complètement retirée par unité de temps.

**4.** Procédé selon l'une quelconque des revendications précédentes, dans lequel ladite analyse statistique comprend, en outre, le calcul d'un écart type pour ladite efficacité dudit traitement par un calcul statistique, sélectionné dans le groupe consistant en le calcul d'un écart type pour KT/V pour chaque patient, le calcul d'un écart type pour KT/V inter-patient et le calcul d'un écart type pour KT/V intra-patient.

**5.** Procédé selon l'une quelconque des revendications précédentes, dans lequel :

ladite CPU est sélectionnée dans le groupe consistant en un microprocesseur, un ordinateur, un ordinateur central, un ordinateur serveur, un ordinateur de bureau, un ordinateur de poste de travail, un ordinateur bloc-notes, un ordinateur portable, un ordinateur de type pilote de palm, une puce d'ordinateur, un circuit intégré, un contrôleur électronique, un réseau, Internet et un réseau de communications mondial.

**6.** Procédé selon l'une quelconque des revendications précédentes comprenant :

le calcul électronique de résultats financiers dudit procédé pour ledit traitement desdits patients dans lesdites installations à l'aide de ladite CPU ;
lesdits résultats financiers sont sélectionnés dans le groupe consistant en : des bénéfices, un revenu d'exploitation, un revenu brut, un revenu net, une marge brute, une marge nette, des profits, un EBITA et un EBITDA ;
ledit EBITA comprenant des bénéfices avant intérêt, impôts et amortissement ; et
ledit EBITDA comprenant des bénéfices avant intérêt, impôts, dépréciation et amortissement.

**7.** Procédé selon l'une quelconque des revendications précédentes, dans lequel :

lesdites données comprennent, en outre, des données supplémentaires sélectionnées dans le groupe consistant en des données d'installations, des données de patients et des données de coûts ;
lesdites données d'installations comprenant des informations démographiques sélectionnées dans le groupe consistant en : une zone statistique métropolitaine de chaque installation définissant une ZSM urbaine, la propriété de chaque installation, le type.de propriété de chaque installation comprenant des installations détenues par une société et en participation, la durée de service de chaque installation, l'hospitalisation de patients, la division et la rotation des employés (T/O) dans chaque installation ;
lesdites données de patients comprenant des informations de patients déterminées à partir de chaque patient, sélectionnées dans le groupe consistant en :

un type de traitement par patient, la durée (mois) de traitements par patient, le pourcentage de patients recevant ledit traitement en tant que cure principale pour leurs affections, la race des patients, le contexte ethnique de chaque patient, l'hémoglobine par patient, l'albumine par patient, l'utilisation de cathéters par patient, l'utilisation d'équipements par patient, les conditions de température pendant un traitement, les conditions d'humidité pendant un traitement, le type d'équipements et de fournitures, la composition de fluides de dialyse, une non-observance par patient, une utilisation d'un supplément de fer par patient, une utilisation d'epogen par patient, un taux de mortalité brut (CMR) de patients dans ladite installation, des mois moyens en dialyse (MOD) par patient, un indice de comorbidité de charleson modifié (MCCI) ; et

lesdites données de coûts comprenant des données financières sélectionnées dans le groupe consistant en :

des coûts d'équipements par traitement, des coûts de dialyseur par traitement, des coûts de fournitures par traitement, des coûts dé stérilisation d'équipement de dialyse pour lesdits traitements, dés économies et des coûts de réutilisation d'équipement pour ledit traitement, des coûts de main-d'oeuvre par traitement, des frais généraux par installation, un pourcentage de patients couverts par une assurance commerciale, un remboursement d'assurance maladie pour lesdits traitements, des remboursements d'agences gouvernementales pour lesdits traitements et des remboursements de compagnies d'assurance pour lesdits traitements.

**8.** Procédé selon l'une quelconque des revendications précédentes, comprenant le mappage dudit procédé.

**9.** Procédé selon l'une quelconque des revendications précédentes, comprenant la mise en oeuvre dudit procédé avec un écart type (sigma) d'environ un.

**10.** Procédé selon l'une quelconque des revendications précédentes, dans lequel ledit traitement est sélectionné dans le groupe consistant en une hémodialyse et une dialyse.

**11.** Dispositif pour fournir une mesure des performances d'une installation médicale qui traite des patients devant subir des traitements sanguins extracorporels répétitifs, ledit dispositif comprenant :

des moyens d'entrée adaptés pour permettre l'entrée de données comprenant :
une efficacité et un rendement mesurés desdits traitements par patient en utilisant un paramètre indicatif d'une purification du sang, une fréquence de traitement par patient, des coûts dudit traitement par patient, des variations d'efficacité, de rendement et de coûts dudit traitement par patient, des caractéristiques de patients et des données démographiques de chaque installation ;
des moyens de mémorisation adaptés pour mémoriser lesdites données ;
des moyens processeurs adaptés pour analyser statistiquement lesdites données mémorisées pour calculer un sigma comprenant un écart type autour d'une moyenne desdites données et pour corréler lesdites données afin de déterminer les performances de ladite installation.

**12.** Dispositif selon la revendication 11, dans lequel ledit dispositif est .sélectionné dans le groupe consistant en un microprocesseur, un ordinateur, un ordinateur central, un ordinateur serveur, un ordinateur de bureau, une ordinateur de poste de travail, un ordinateur bloc-notes, un ordinateur portable, un ordinateur de type pilote de palm, une puce d'ordinateur, un circuit intégré, un contrôleur électronique, un réseau, Internet et un réseau de communications mondial.

**13.** Dispositif selon la revendication 11 ou 12, dans lequel l'efficacité de chaque traitement est mesurée selon le modèle mathématique KT/V, où K = épuration, T = temps dudit traitement, V = volume de répartition dans le corps d'urée ou de créatinine, et dans lequel, en outre, ledit processeur est agencé pour calculer un écart type pour ladite efficacité dudit traitement par un calcul statistique, sélectionné dans le groupe consistant en le calcul d'un écart type pour KT/V pour chaque patient, le calcul d'un écart type pour KT/V inter-patient et le calcul d'un écart type pour KT/V intra-patient.

**14.** Dispositif selon la revendication 13, dans lequel ledit processeur est agencé, en outre, pour effectuer le calcul KT/V pour déterminer l'efficacité de chaque traitement.

**15.** Programme informatique qui, lorsqu'il est chargé sur un ordinateur, donne instruction à l'ordinateur d'effectuer les étapes du procédé selon l'une quelconque des revendications 1 à 10.

**16.** Programme informatique selon la revendication 15, mis en oeuvre sur un support lisible par un ordinateur.

## *Histogram: Kt/V Distribution count for 3 months*

**1 monthly measurement per patient**

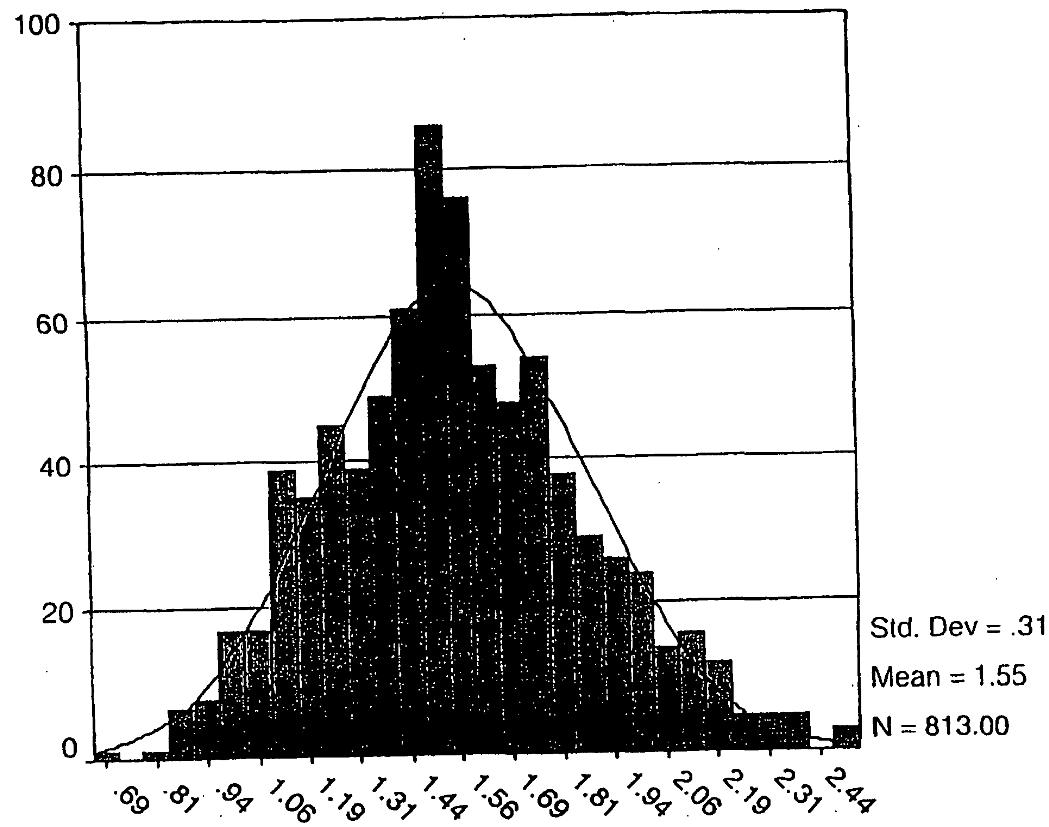


FRANCE 1301

**Fig. 1**

## *Histogram: Kt/V Distribution count for 3 months*

**1 monthly measurement per patient**

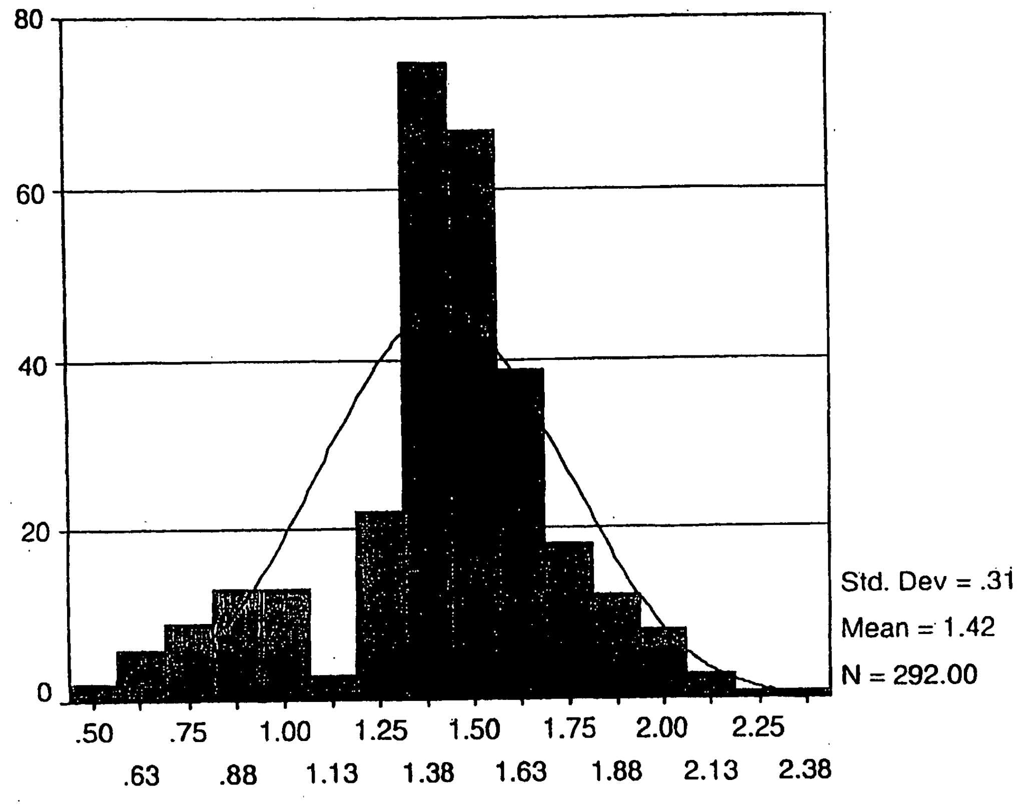


PENNSYLVANIA AVE (USA)

**Fig. 2**

## Histogram: Kt/V Distribution count for 3 months

**1 monthly measurement per patient**

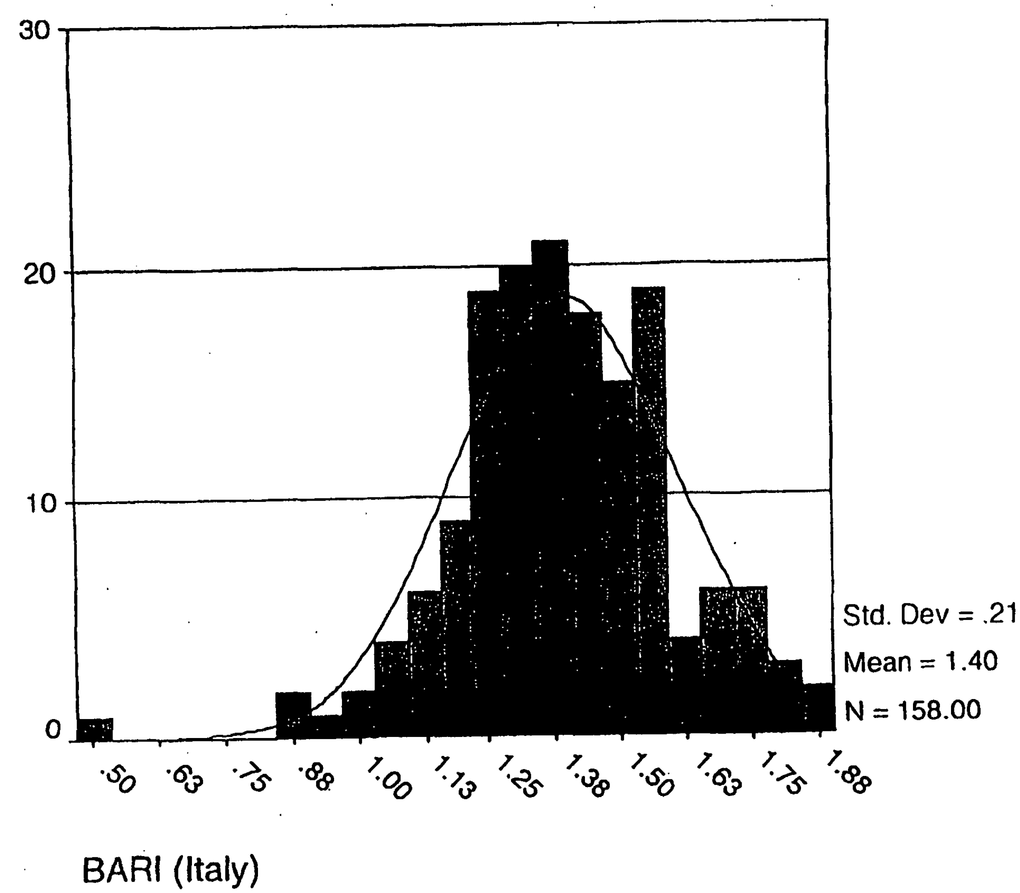


**Fig. 3**

## *Histogram: Kt/V Distribution count for 3 months*

**1 monthly measurement per patient**

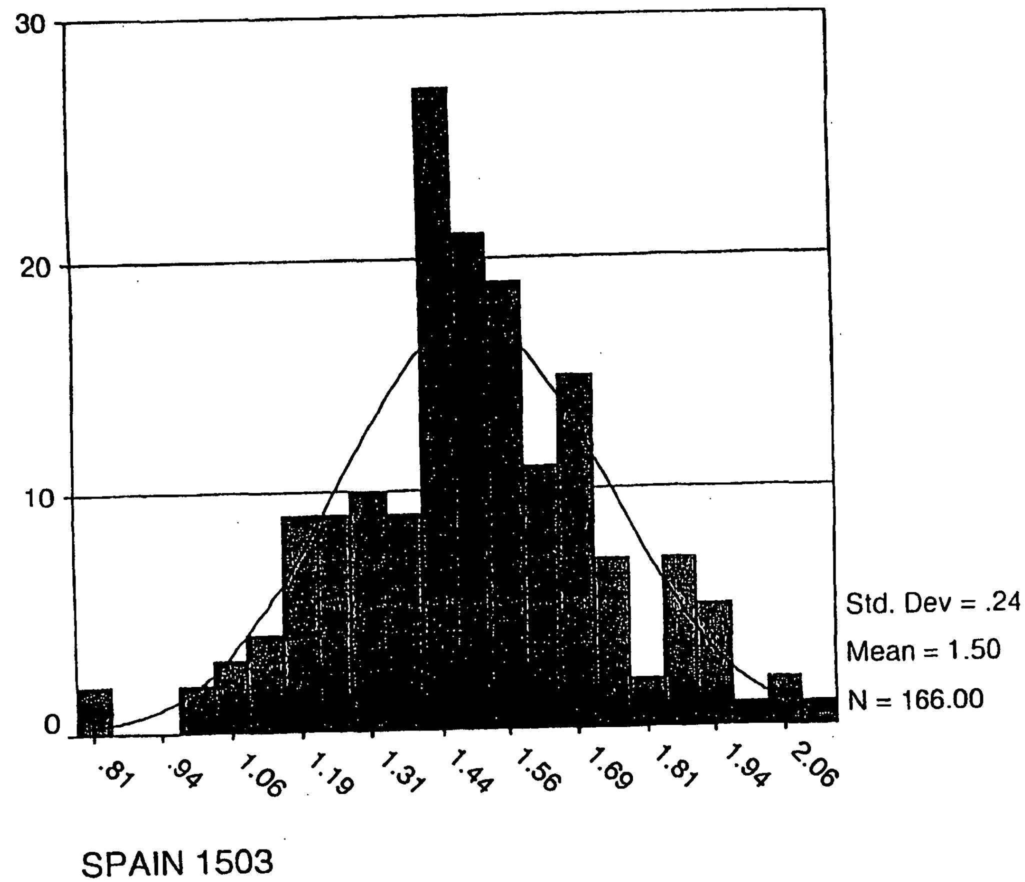


**Fig. 4**

# *Histogram: Kt/V Distribution count for 3 months*

**1 monthly measurement per patient**

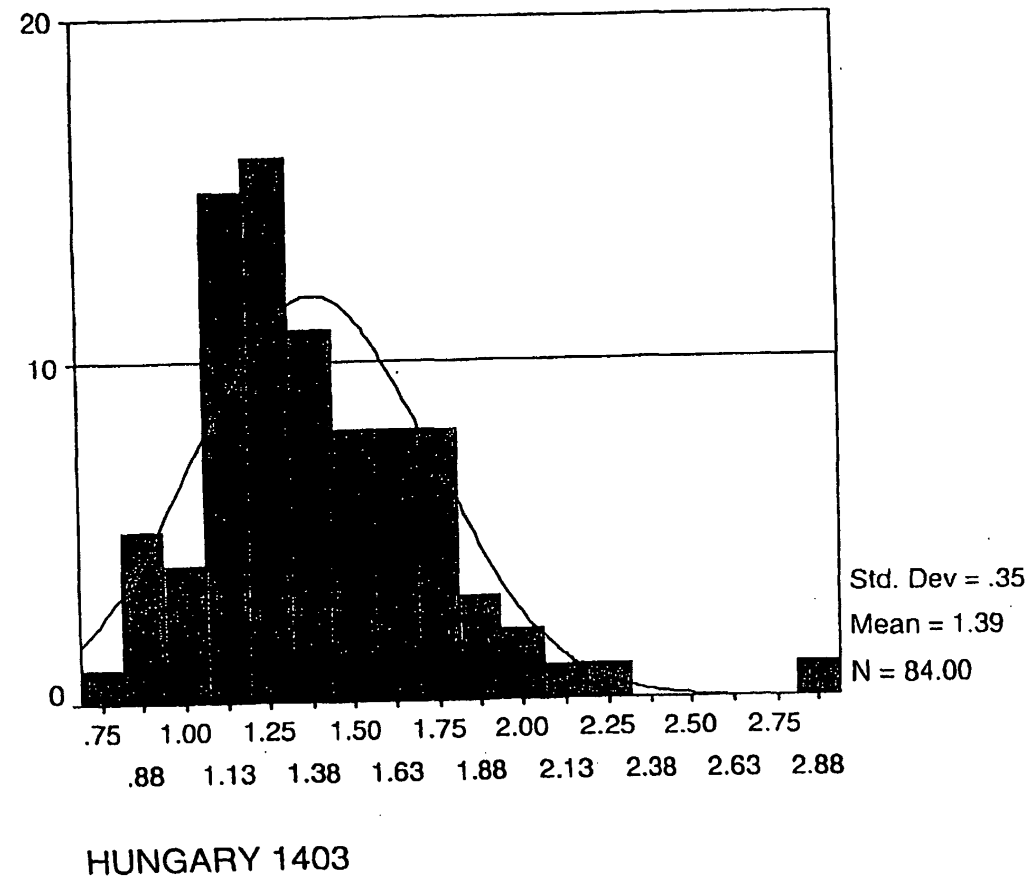


**Fig. 5**

# Overall Clinic SD

"Defines the ability of the clinic to deliver Rxs within the specs"

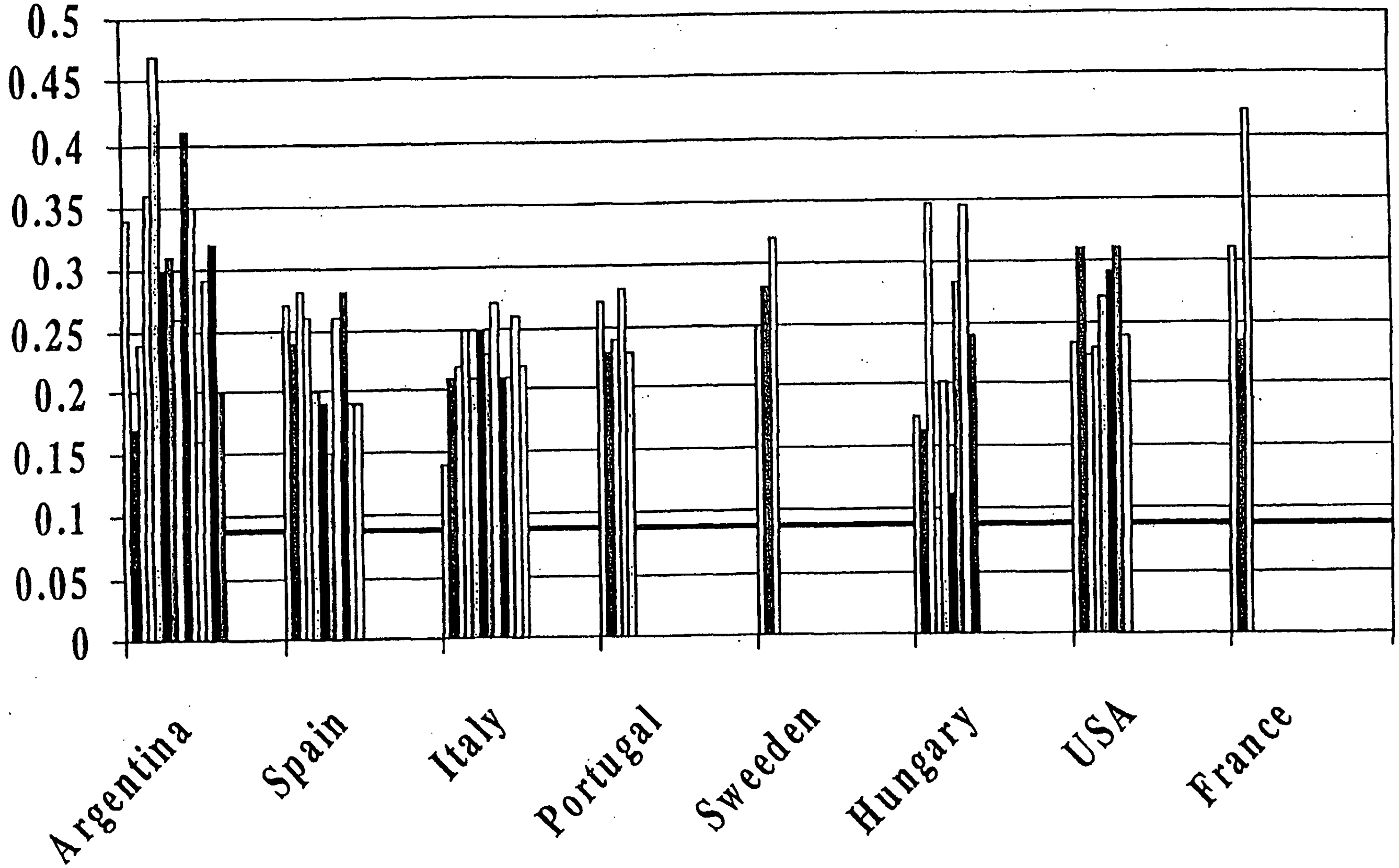


Fig. 6

**Inter Patient Variation**

12 Pts With BW Between 60-70 Kg

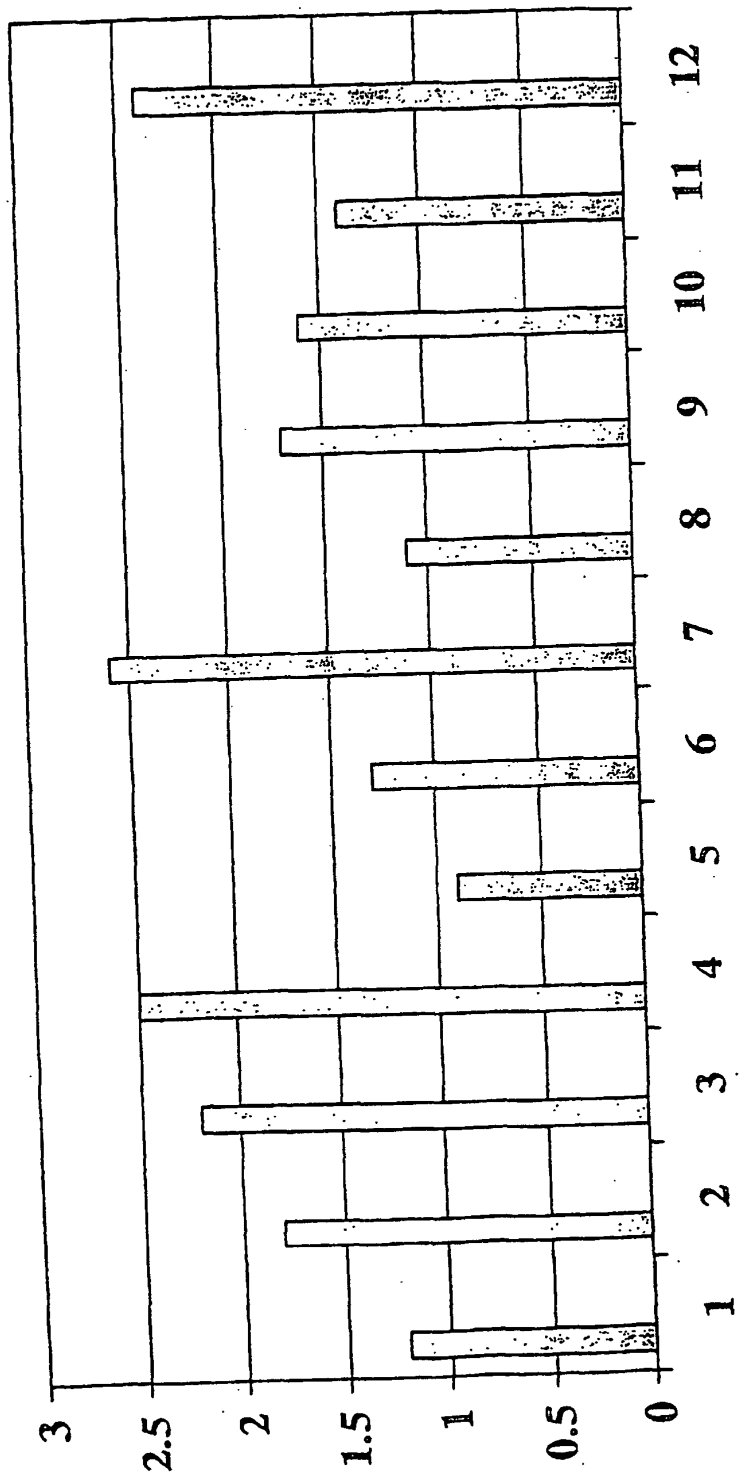


**Fig. 7**

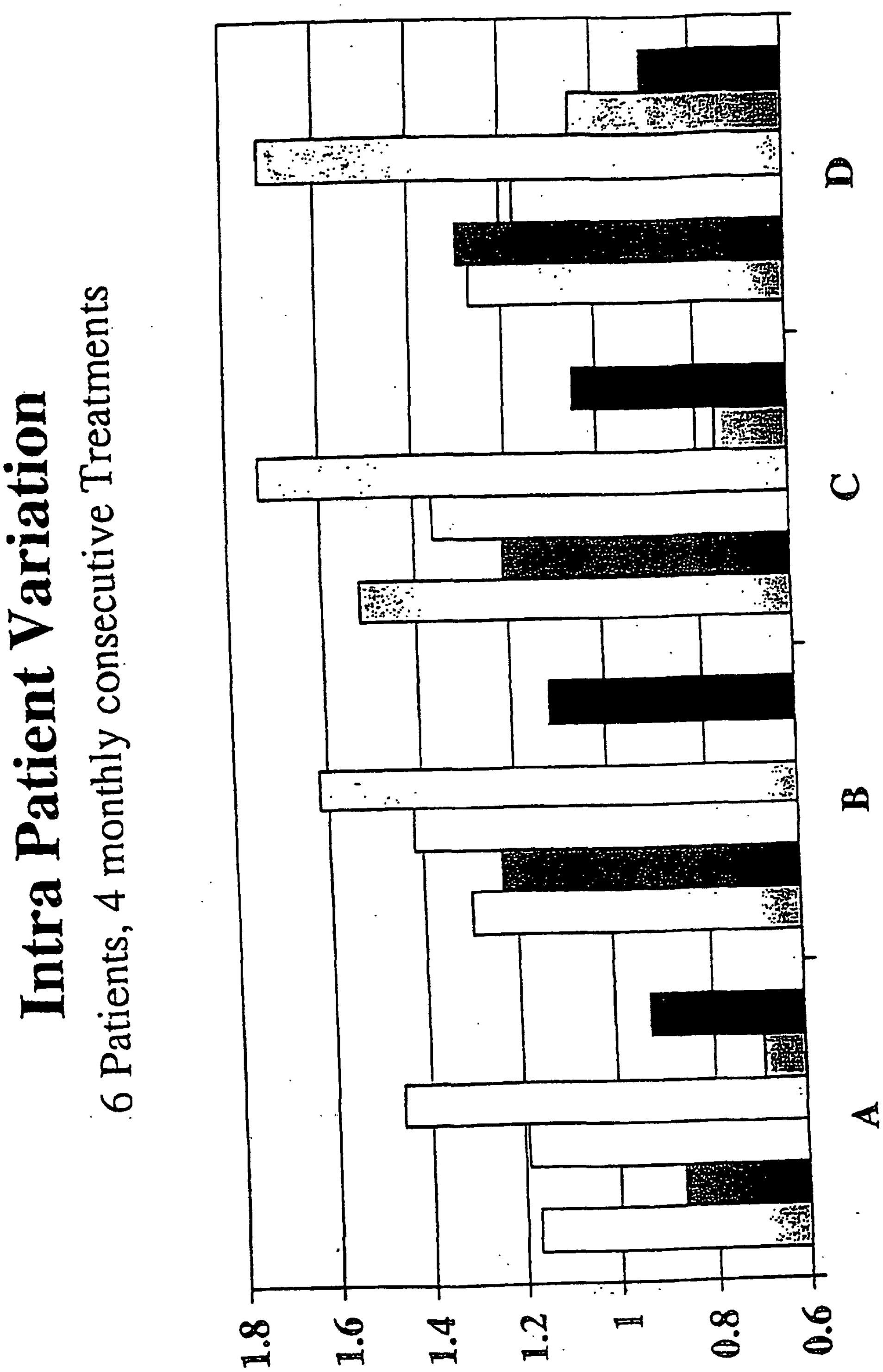
Intra Patient Variation
6 Patients, 4 monthly consecutive Treatments
1.8
1.6
1.4
1.2
1
0.8
0.6
A
B
C
D

Fig. 8

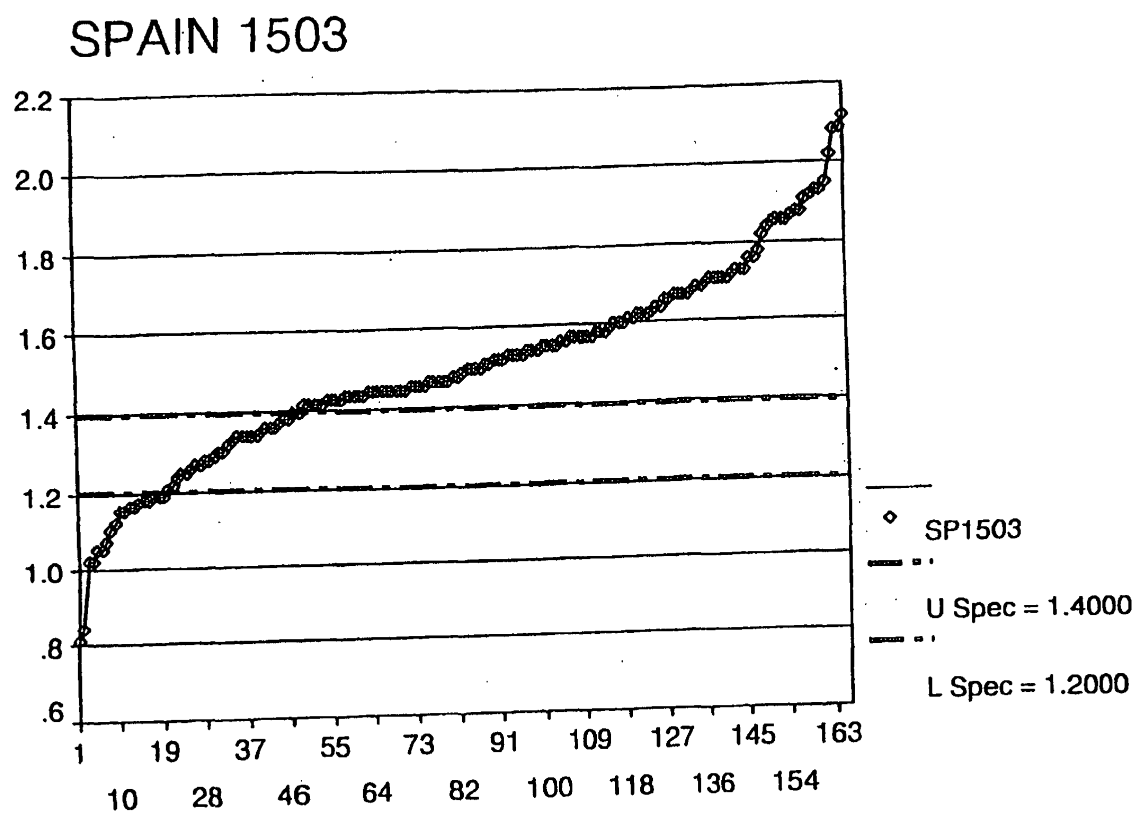


**Fig. 9**

**Process Statistics**

| | | |
|---|---|---|
| | Act. % Outside SL | 82.5% |
| Capability Indices | CP[a] | 4.737 |
| | CpU[a] | -4.740 |
| | CpK[a] | -4.740 |

The normal distribution is assumed. LSL = 1.2 and USL = 1.4.

a. The estimated capability sigma is based on the mean of the sample moving ranges.

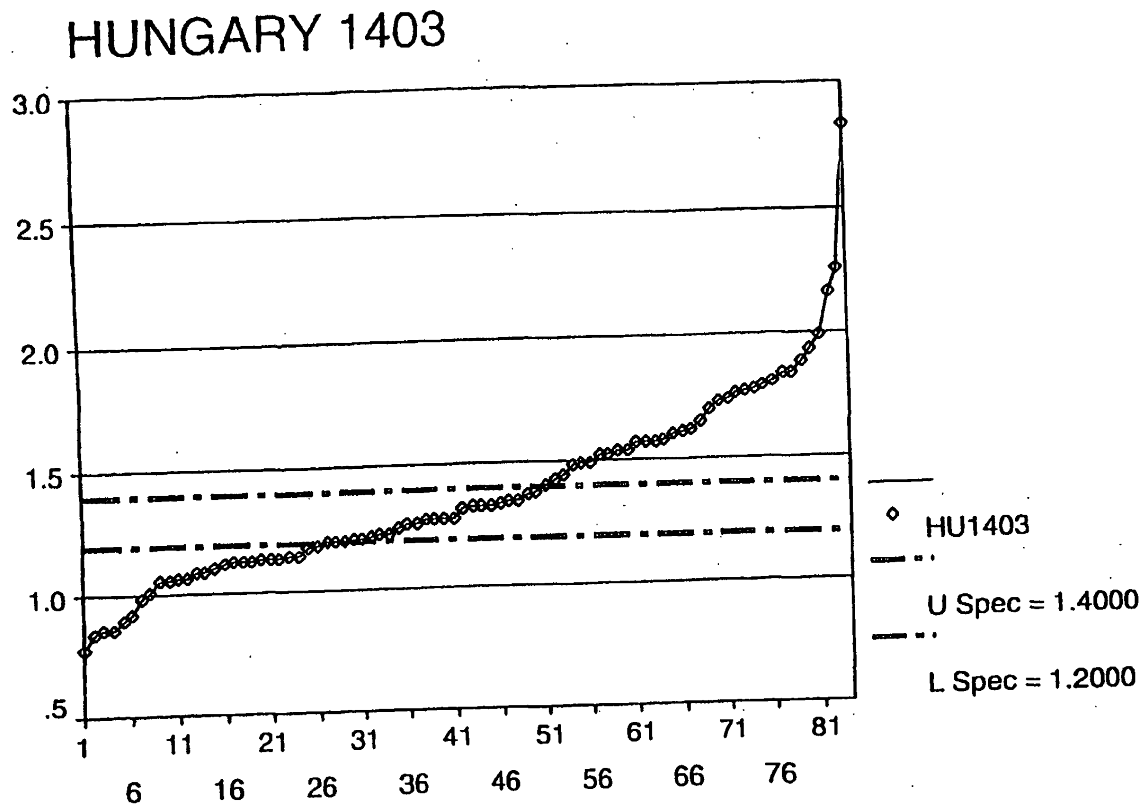


Fig. 10

**Process Statistics**

| | | |
|---|---|---|
| | Act. % Outside SL | 70.2% |
| Capability Indices | CP[a] | 1.523 |
| | CpU[a] | .205 |
| | CpK[a] | .205 |

The normal distribution is assumed. LSL = 1.2 and USL = 1.4.

a. The estimated capability sigma is based on the mean of the sample moving ranges.

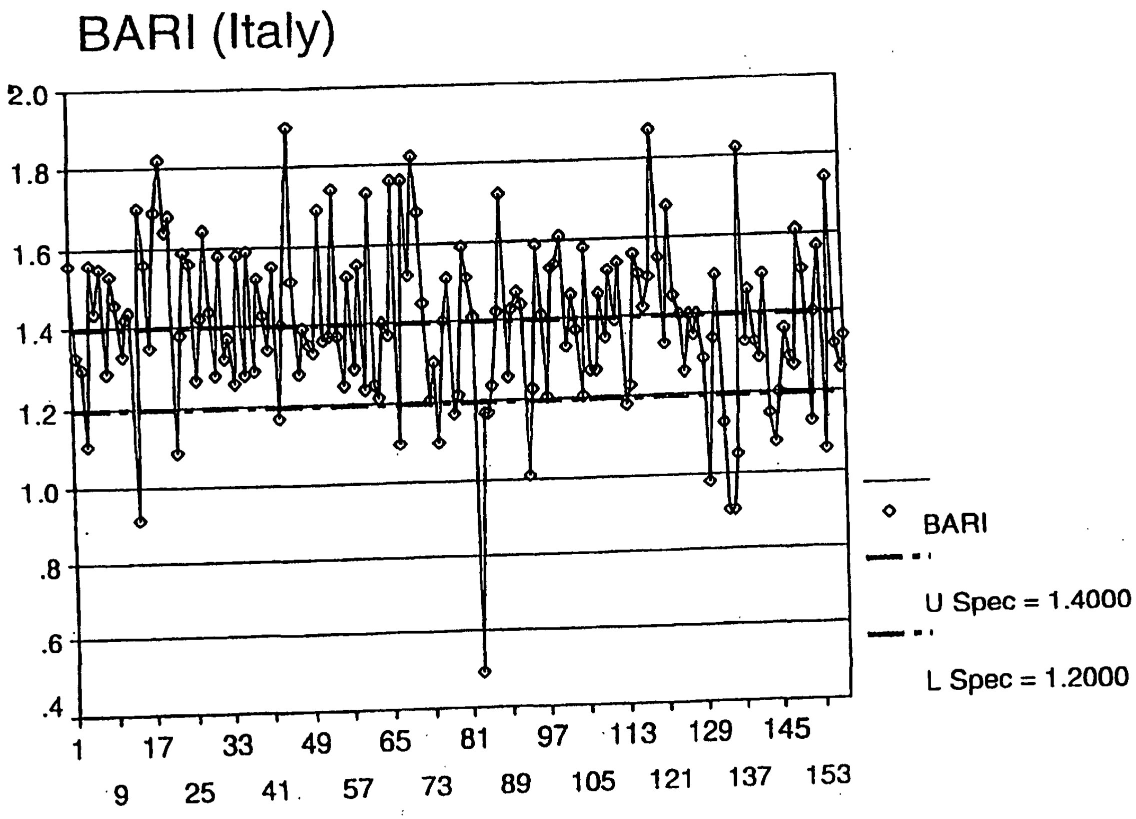


**Fig. 11**

**Process Statistics**

| | | |
|---|---|---|
| | Act. % Outside SL | 59.5% |
| Capability Indices | CP[a] | .163 |
| | CpU[a] | .007 |
| | CpK[a] | .007 |

The normal distribution is assumed. LSL = 1.2 and USL = 1.4.

a. The estimated capability sigma is based on the mean of the sample moving ranges.

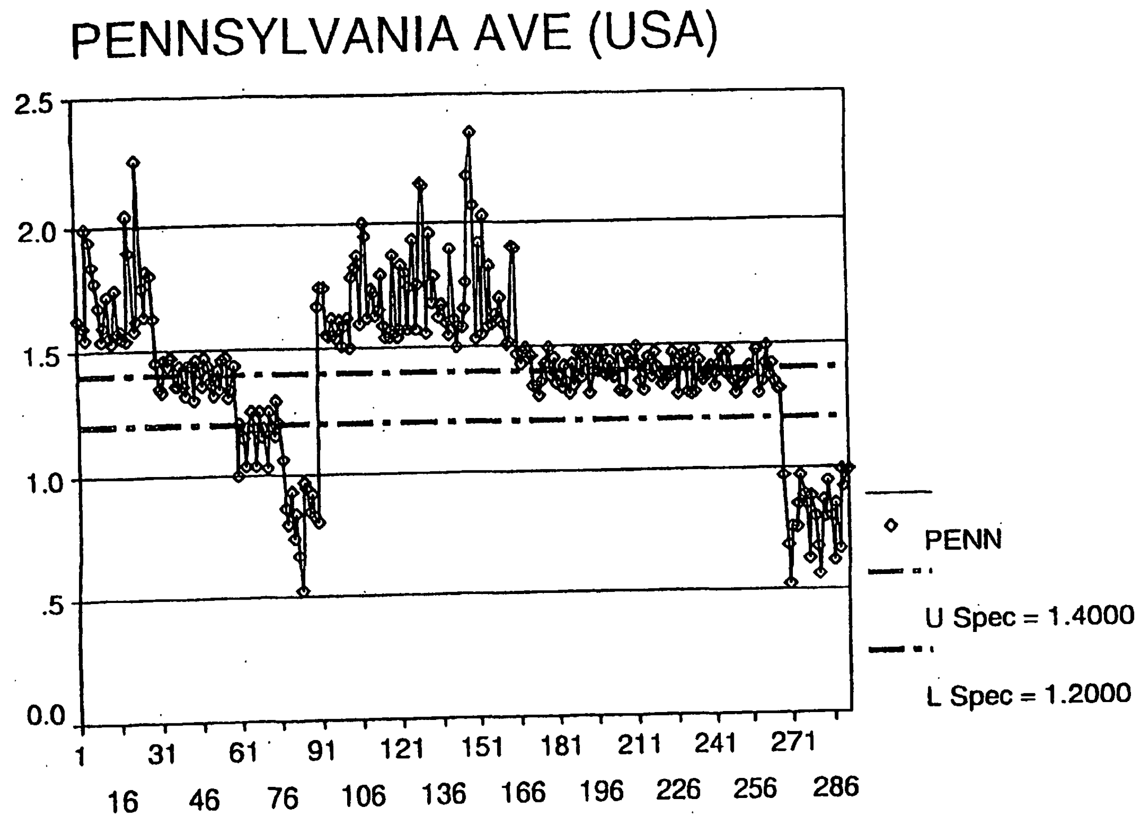


Fig. 12

**Process Statistics**

| | | |
|---|---|---|
| Capability Indices | Act. % Outside SL | 73.3% |
| | CP[a] | .320 |
| | CpU[a] | -.057 |
| | CpK[a] | -.057 |

The normal distribution is assumed. LSL = 1.2 and USL = 1.4.

a. The estimated capability sigma is based on the mean of the sample moving ranges.

Kt/V USA (1 Month average all clinics)

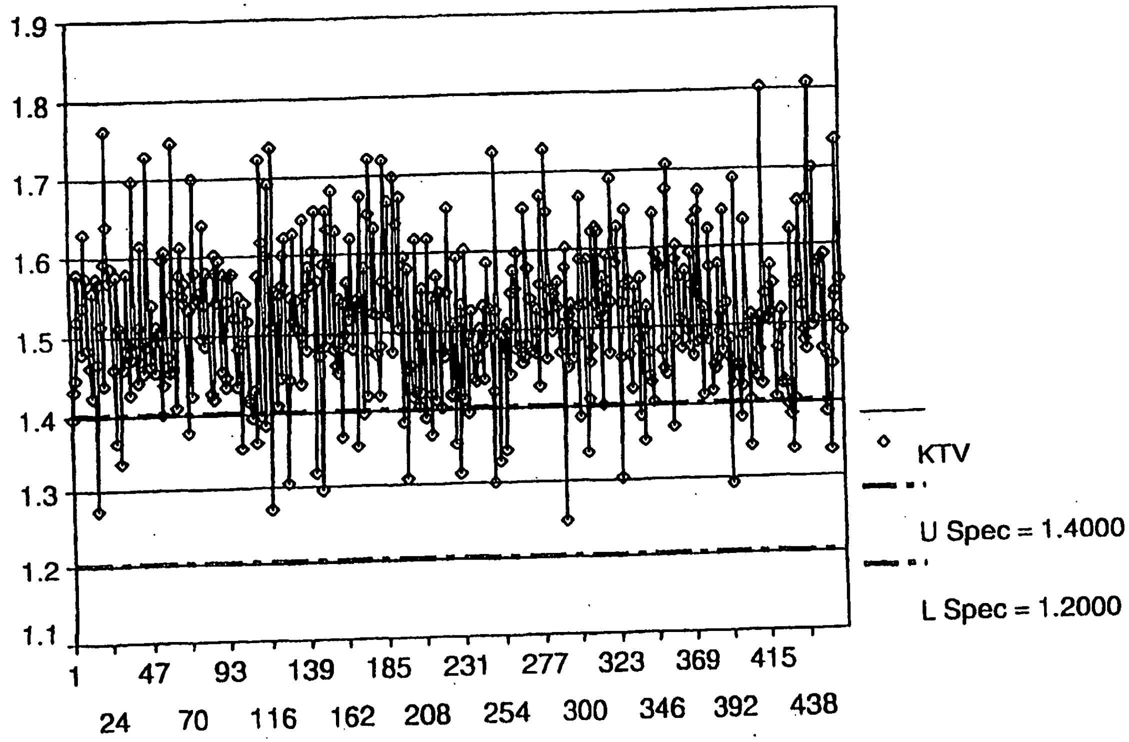


Fig. 13

Process Statistics

| | | |
|---|---|---|
| Capability Indices | Act. % Outside SL | 90.8% |
| | CP[a] | .354 |
| | CpU[a] | -.405 |
| | CpK[a] | -.405 |

The normal distribution is assumed. LSL = 1.2 and USL = 1.4.

a. The estimated capability sigma is based on the mean of the sample moving ranges.

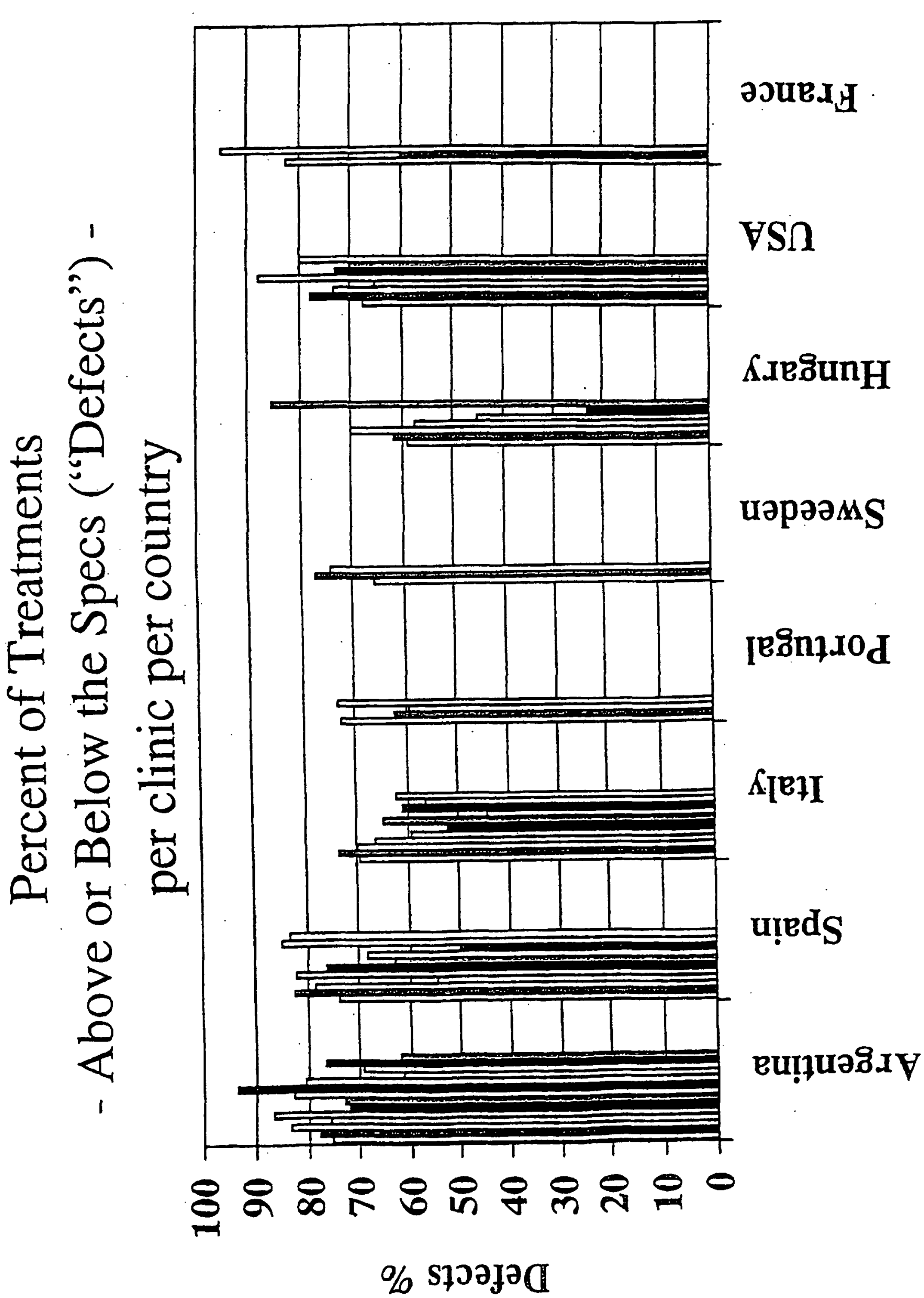
Percent of Treatments
- Above or Below the Specs ("Defects") -
per clinic per country
Defects %
100
90
80
70
60
50
40
30
20
10
0
Argentina
Spain
Italy
Portugal
Sweeden
Hungary
USA
France

Fig. 14

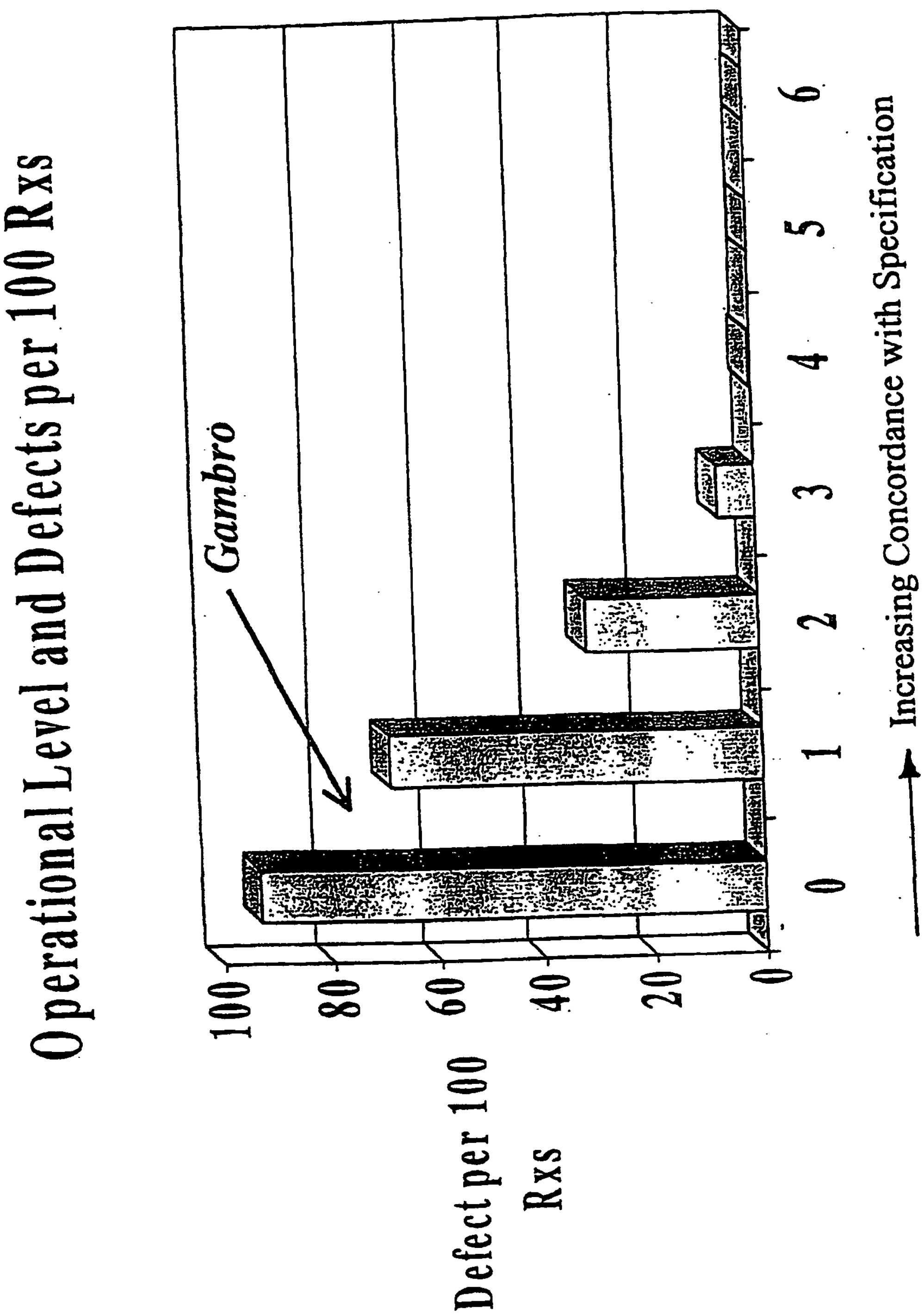

Operational Level and Defects per 100 Rxs
Gambro
Defect per 100 Rxs
100
80
60
40
20
0
0
1
2
3
4
5
6
Increasing Concordance with Specification


**Fig. 15**

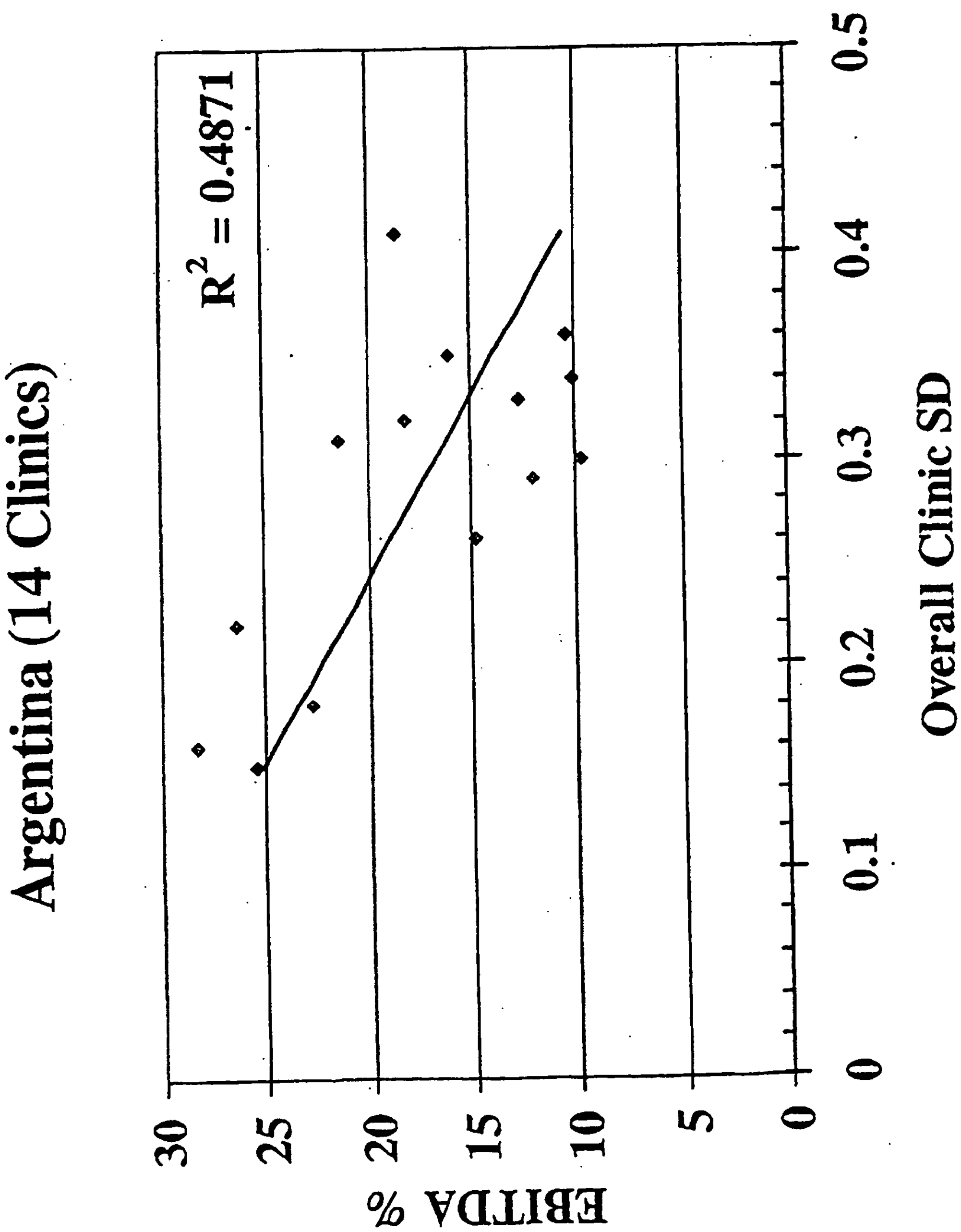
Argentina (14 Clinics)
$R^2 = 0.4871$
EBITDA %
30
25
20
15
10
5
0
0
0.1
0.2
0.3
0.4
0.5
Overall Clinic SD

Fig. 16

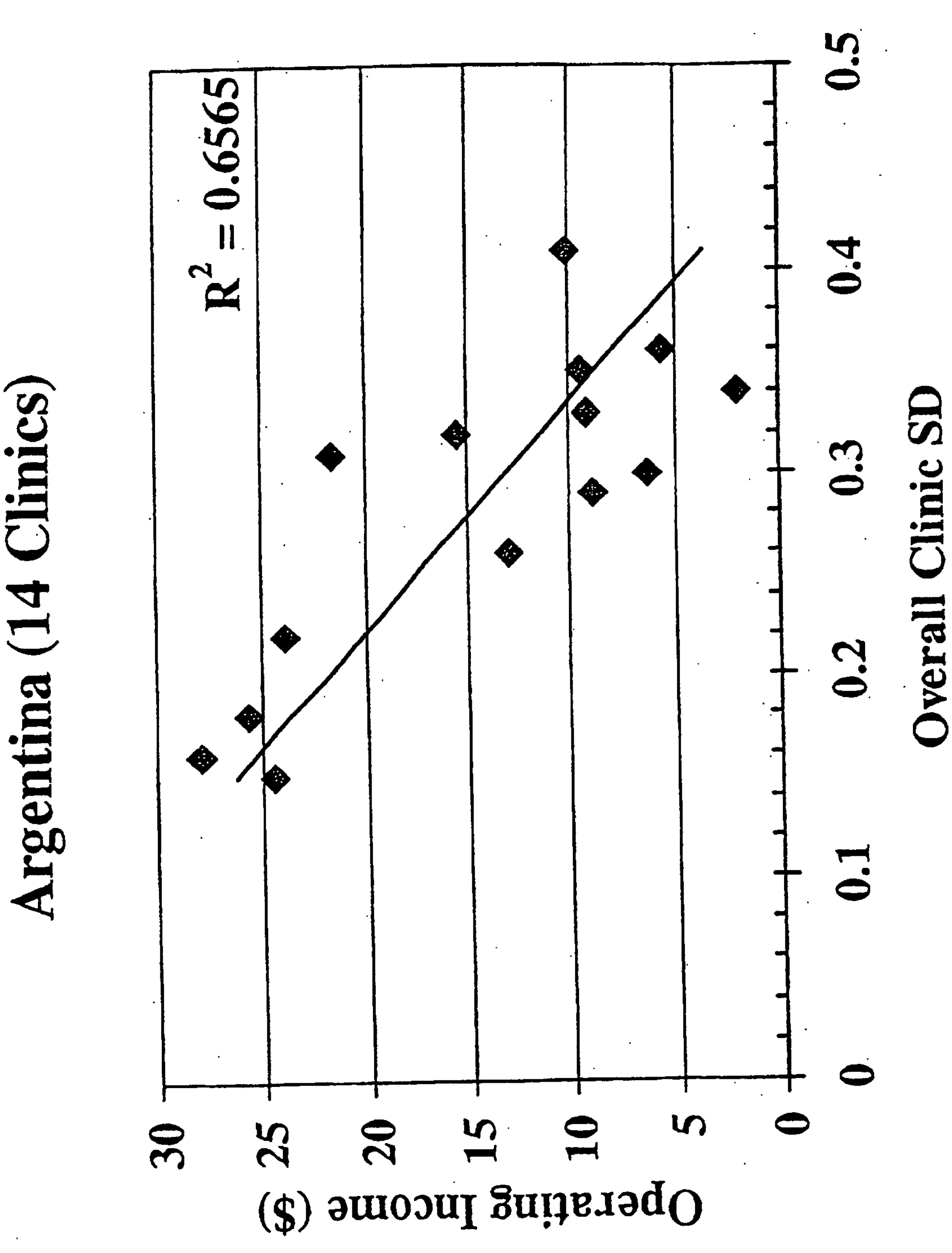
Argentina (14 Clinics)
$R^2 = 0.6565$
Operating Income ($)
30
25
20
15
10
5
0
0
0.1
0.2
0.3
0.4
0.5
Overall Clinic SD

Fig. 17

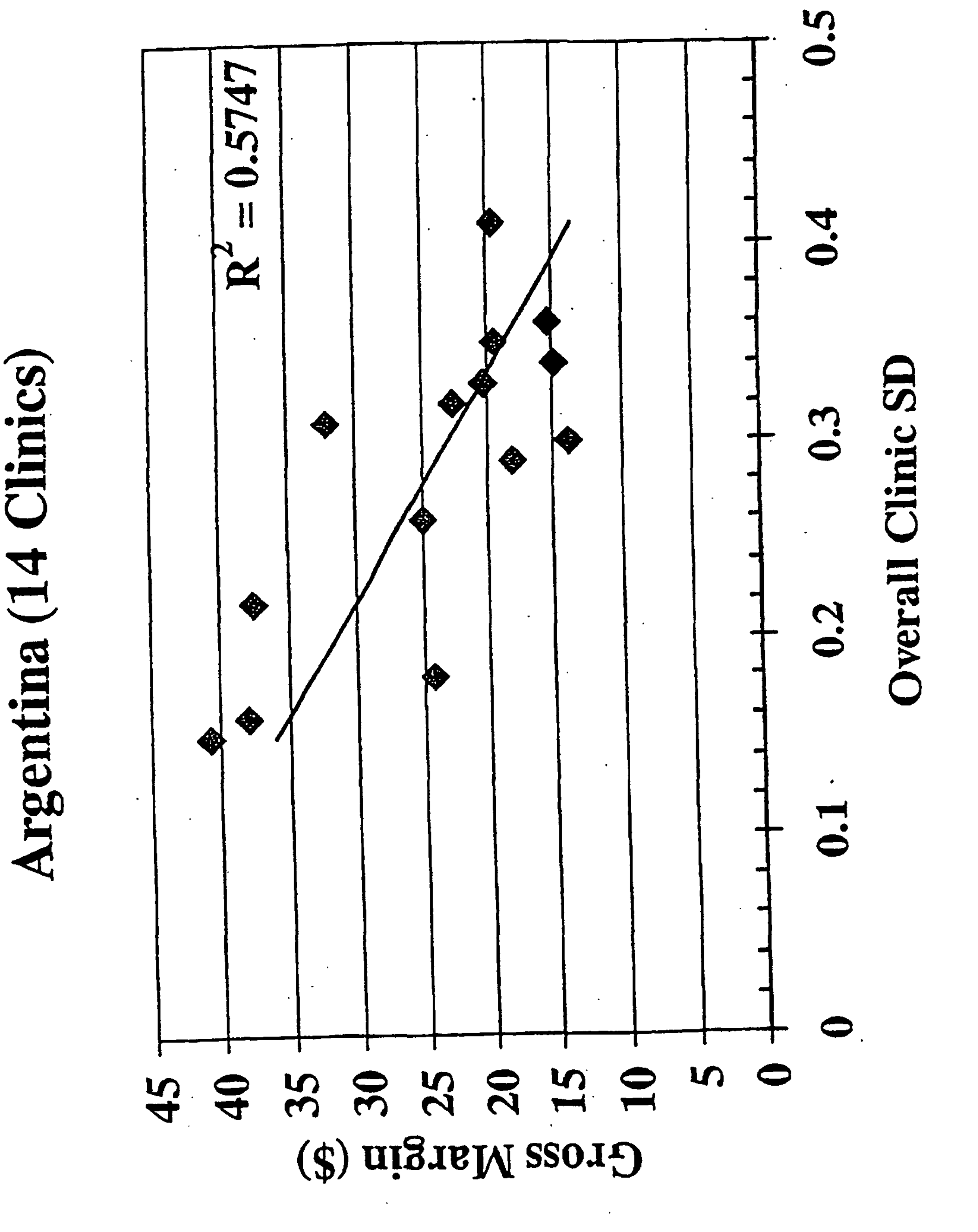
Argentina (14 Clinics)
$R^2 = 0.5747$
Gross Margin ($)
45
40
35
30
25
20
15
10
5
0
0
0.1
0.2
0.3
0.4
0.5
Overall Clinic SD

Fig. 18

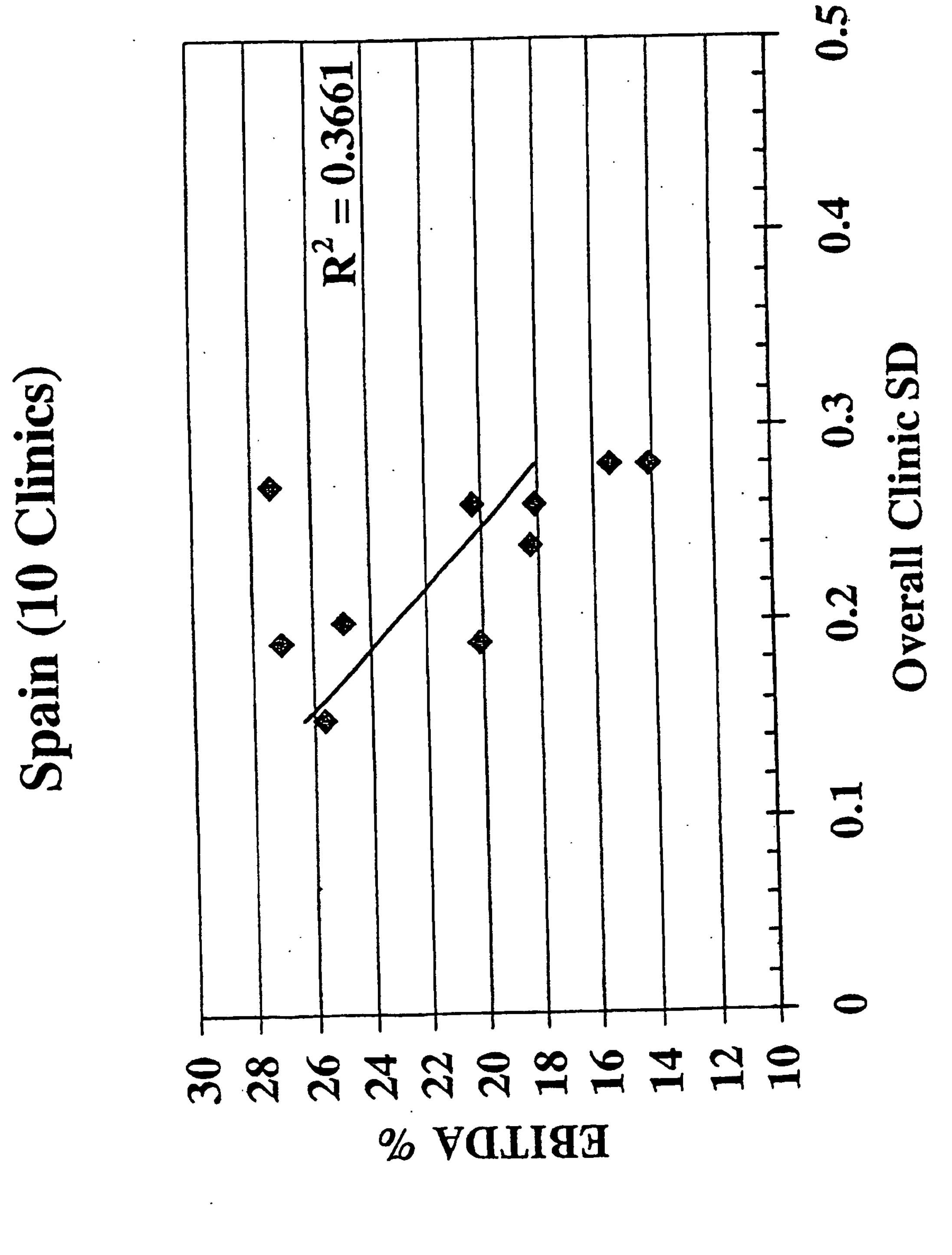
Spain (10 Clinics)
$R^2$ = 0.3661
EBITDA %
30
28
26
24
22
20
18
16
14
12
10
0
0.1
0.2
0.3
0.4
0.5
Overall Clinic SD

Fig. 19

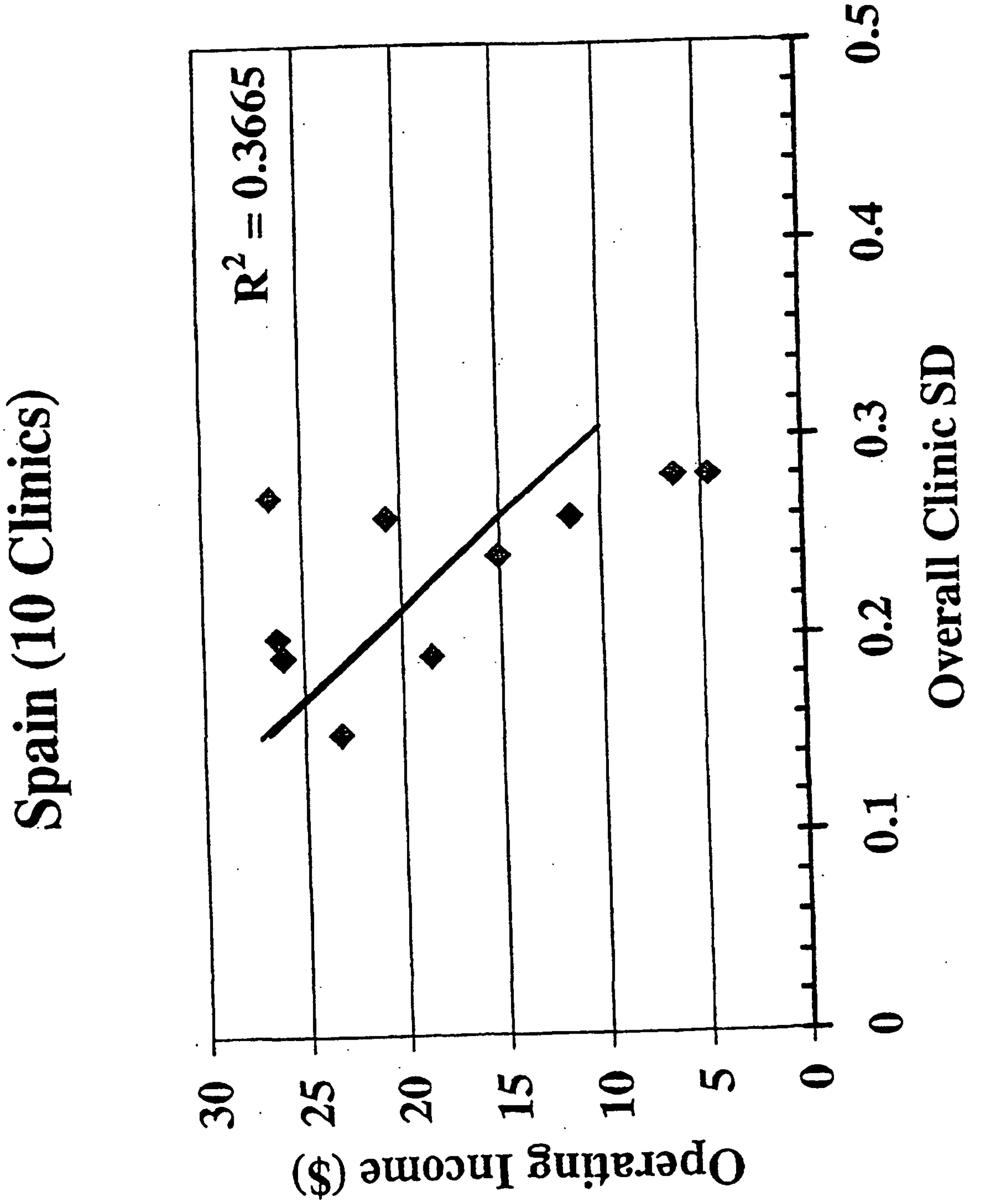
Spain (10 Clinics)
$R^2 = 0.3665$
Operating Income ($)
30
25
20
15
10
5
0
0
0.1
0.2
0.3
0.4
0.5
Overall Clinic SD

Fig. 20

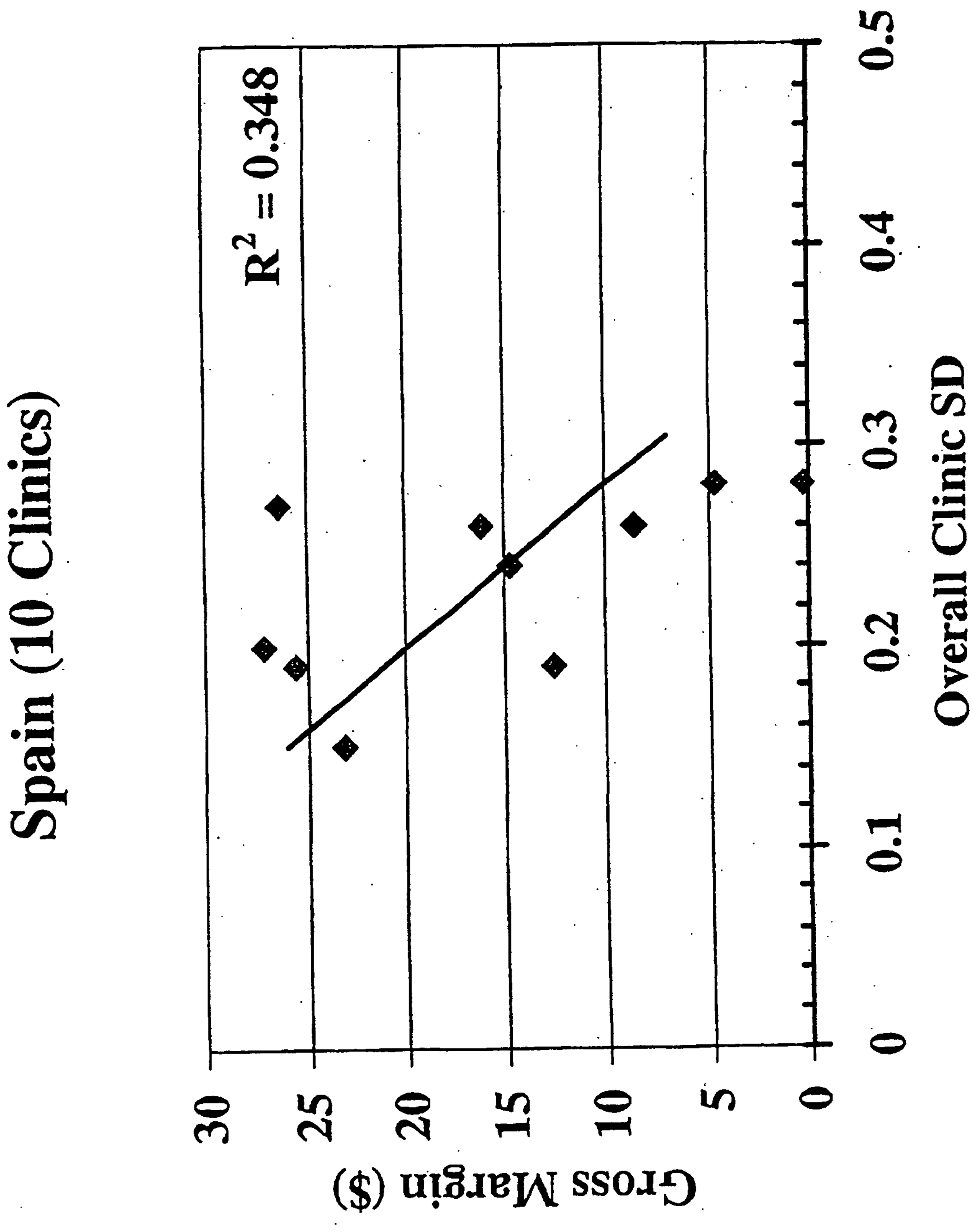
Spain (10 Clinics)
$R^2 = 0.348$
Gross Margin ($)
30
25
20
15
10
5
0
0
0.1
0.2
0.3
0.4
0.5
Overall Clinic SD

Fig. 21

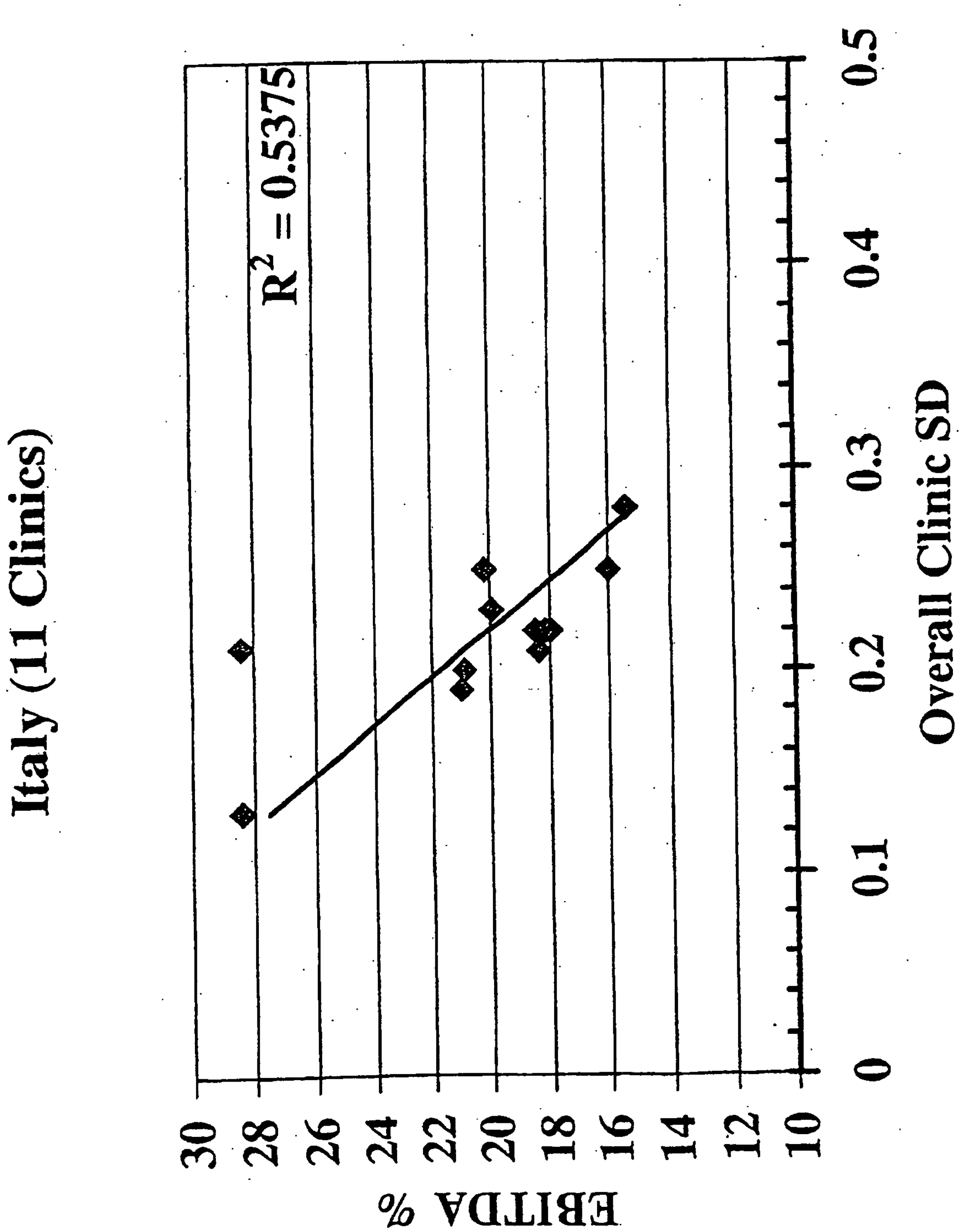
Italy (11 Clinics)
R² = 0.5375
EBITDA %
30
28
26
24
22
20
18
16
14
12
10
0
0.1
0.2
0.3
0.4
0.5
Overall Clinic SD

Fig. 22

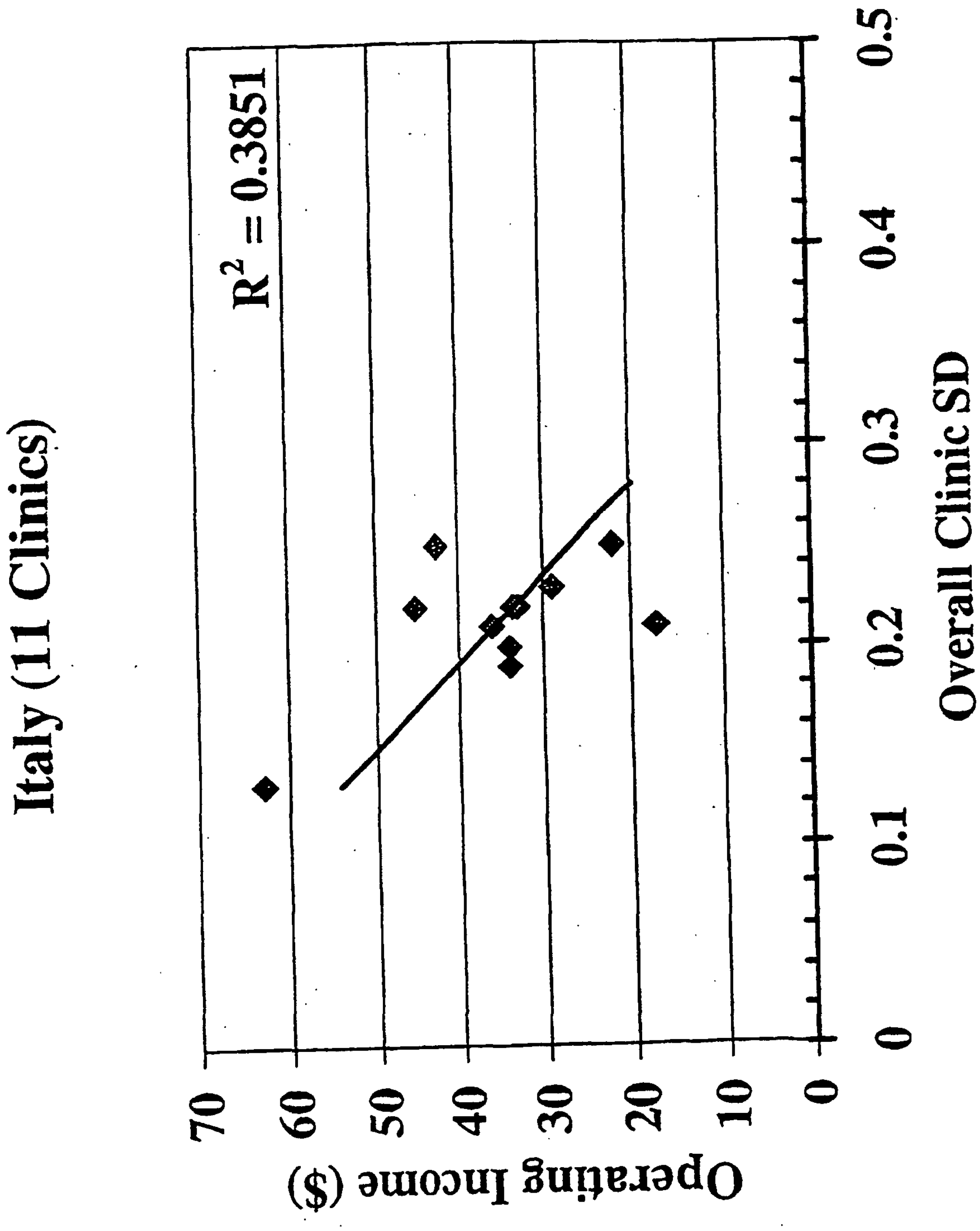
Italy (11 Clinics)
$R^2 = 0.3851$
Operating Income ($)
70
60
50
40
30
20
10
0
0
0.1
0.2
0.3
0.4
0.5
Overall Clinic SD

Fig. 23

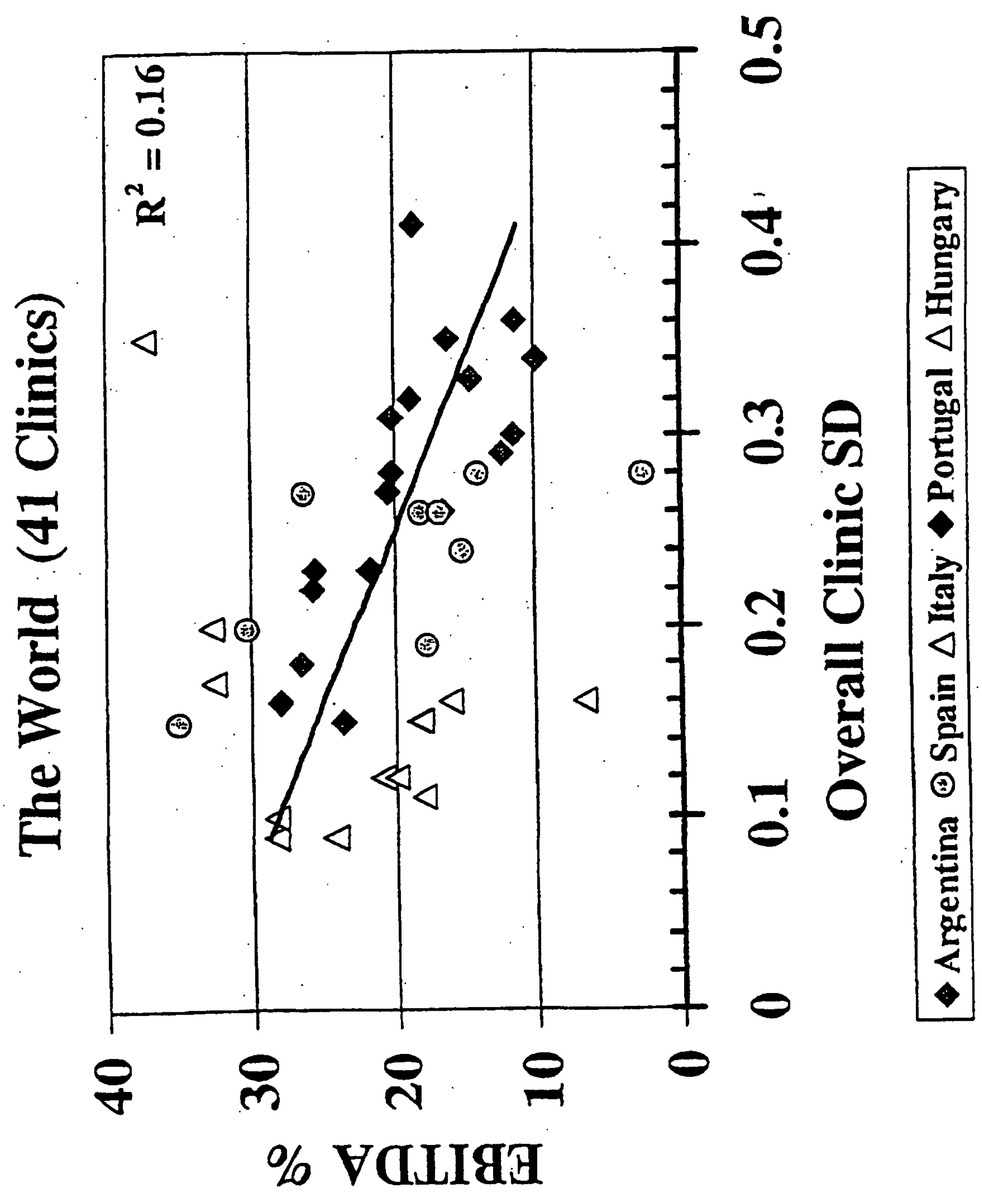
The World (41 Clinics)
$R^2 = 0.16$
EBITDA %
40
30
20
10
0
0
0.1
0.2
0.3
0.4
0.5
Overall Clinic SD
Argentina
Spain
Italy
Portugal
Hungary

Fig. 24

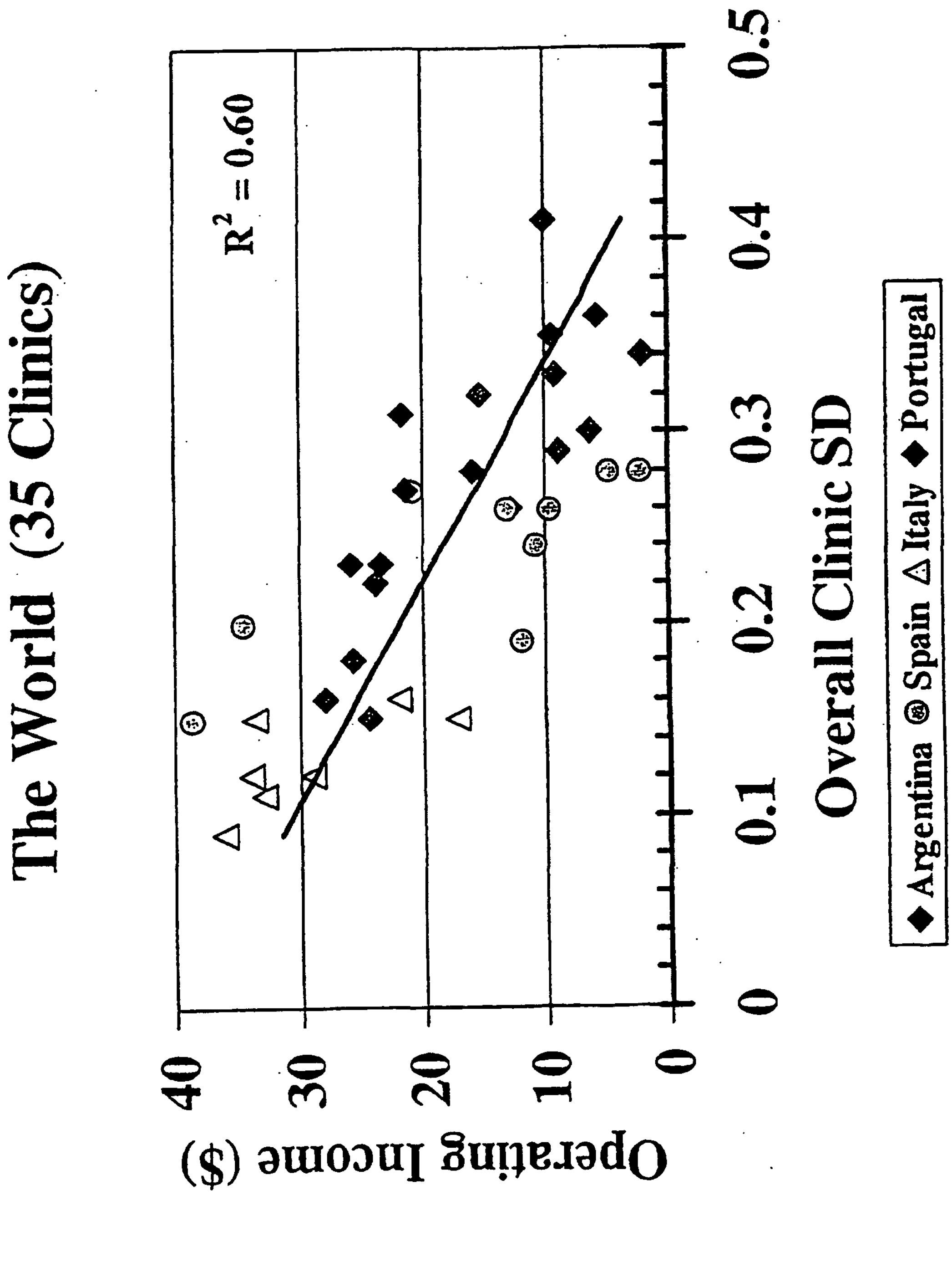
The World (35 Clinics)
$R^2 = 0.60$
Operating Income ($)
0
10
20
30
40
0
0.1
0.2
0.3
0.4
0.5
Overall Clinic SD
Argentina
Spain
Italy
Portugal

Fig. 25

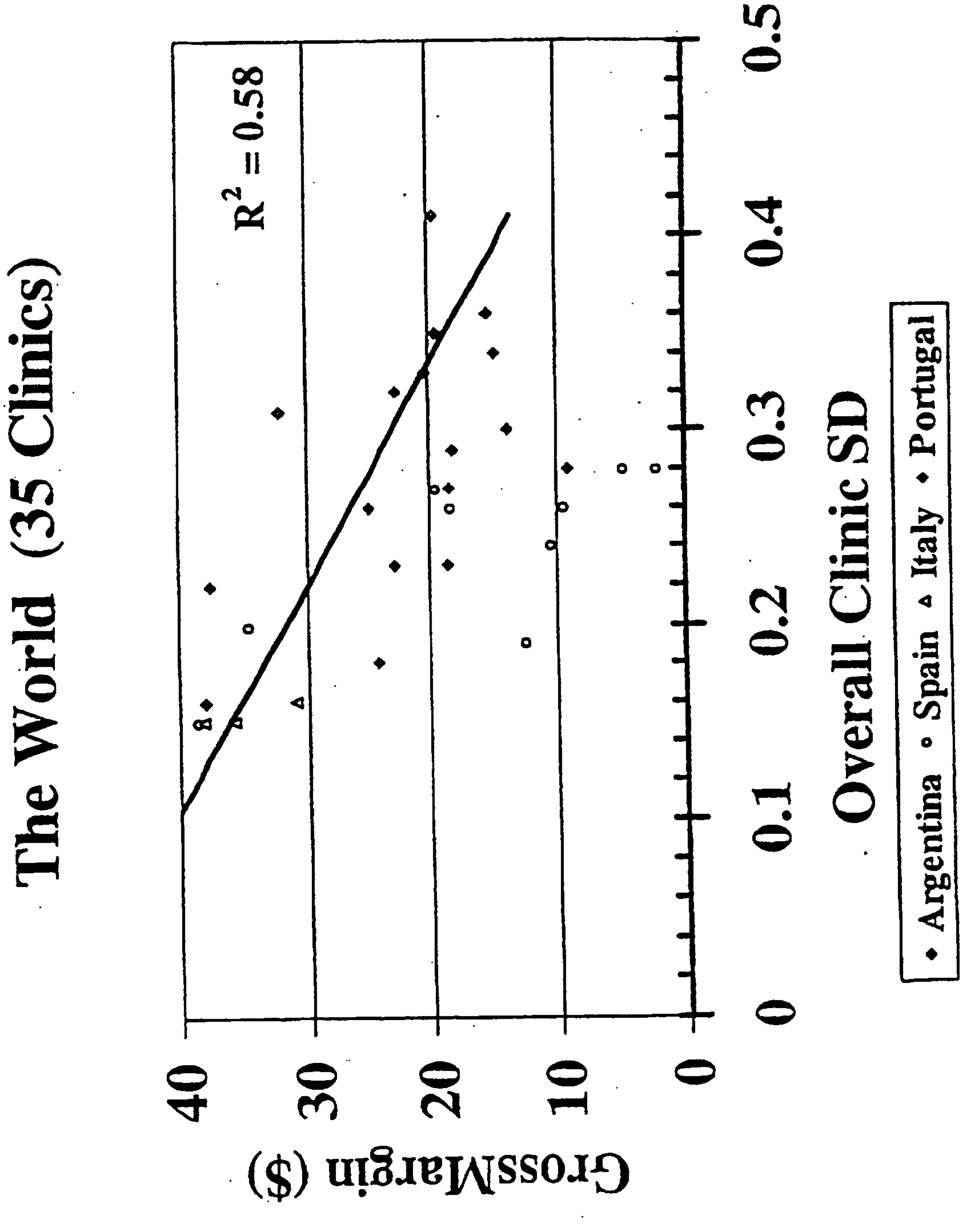

The World (35 Clinics)
$R^2 = 0.58$
GrossMargin ($)
40
30
20
10
0
0
0.1
0.2
0.3
0.4
0.5
Overall Clinic SD
Argentina
Spain
Italy
Portugal


Fig. 26

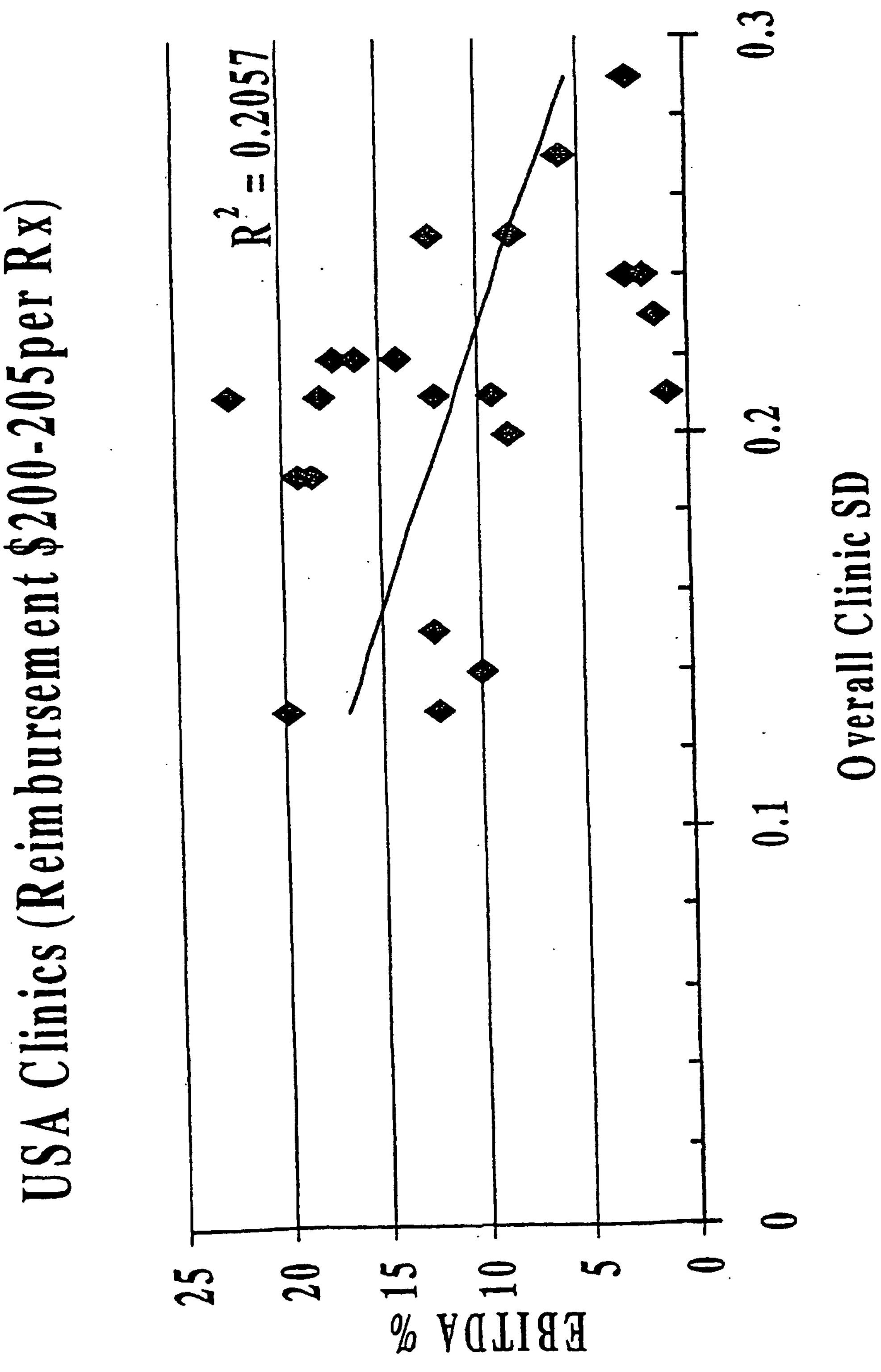
USA Clinics (Reimbursement $200-205per Rx)
$R^2$ = 0.2057
25
20
15
10
5
0
EBITDA %
0
0.1
0.2
0.3
Overall Clinic SD

Fig. 27

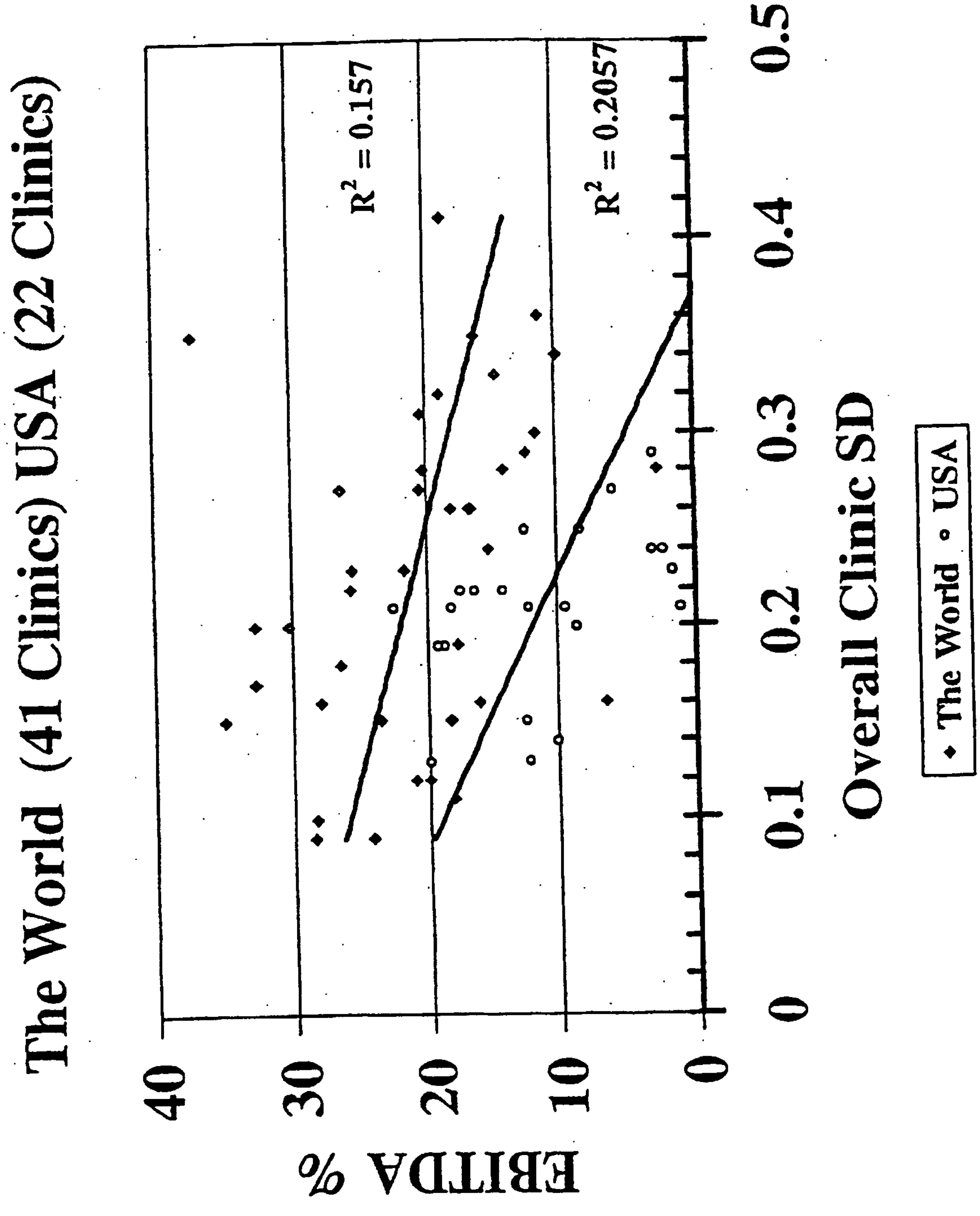
The World (41 Clinics) USA (22 Clinics)
EBITDA %
40
30
20
10
0
0
0.1
0.2
0.3
0.4
0.5
Overall Clinic SD
R2 = 0.157
R2 = 0.2057
The World
USA

Fig. 28

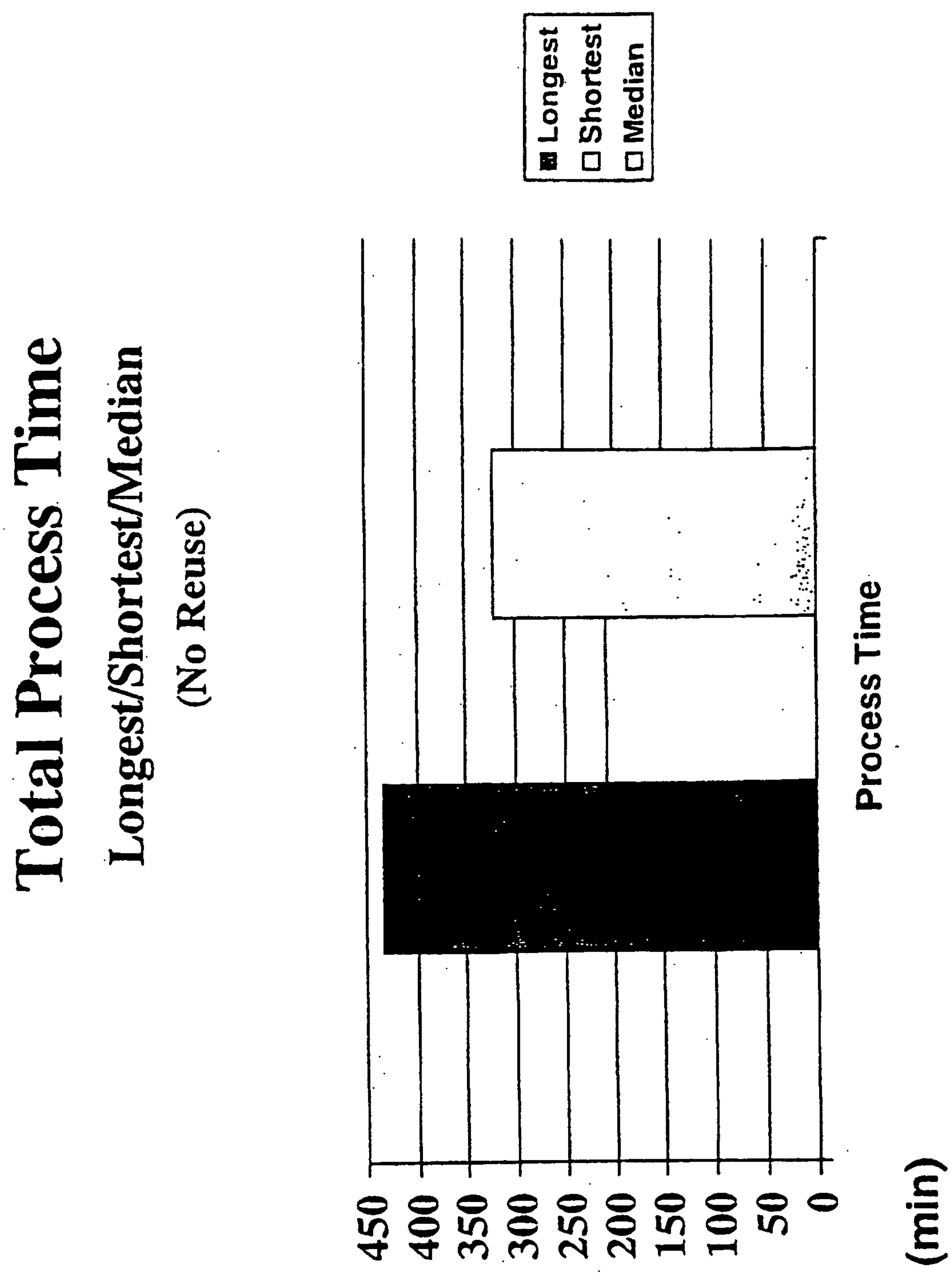
Total Process Time
Longest/Shortest/Median
(No Reuse)
Longest
Shortest
Median
450
400
350
300
250
200
150
100
50
0
(min)
Process Time

Fig. 29

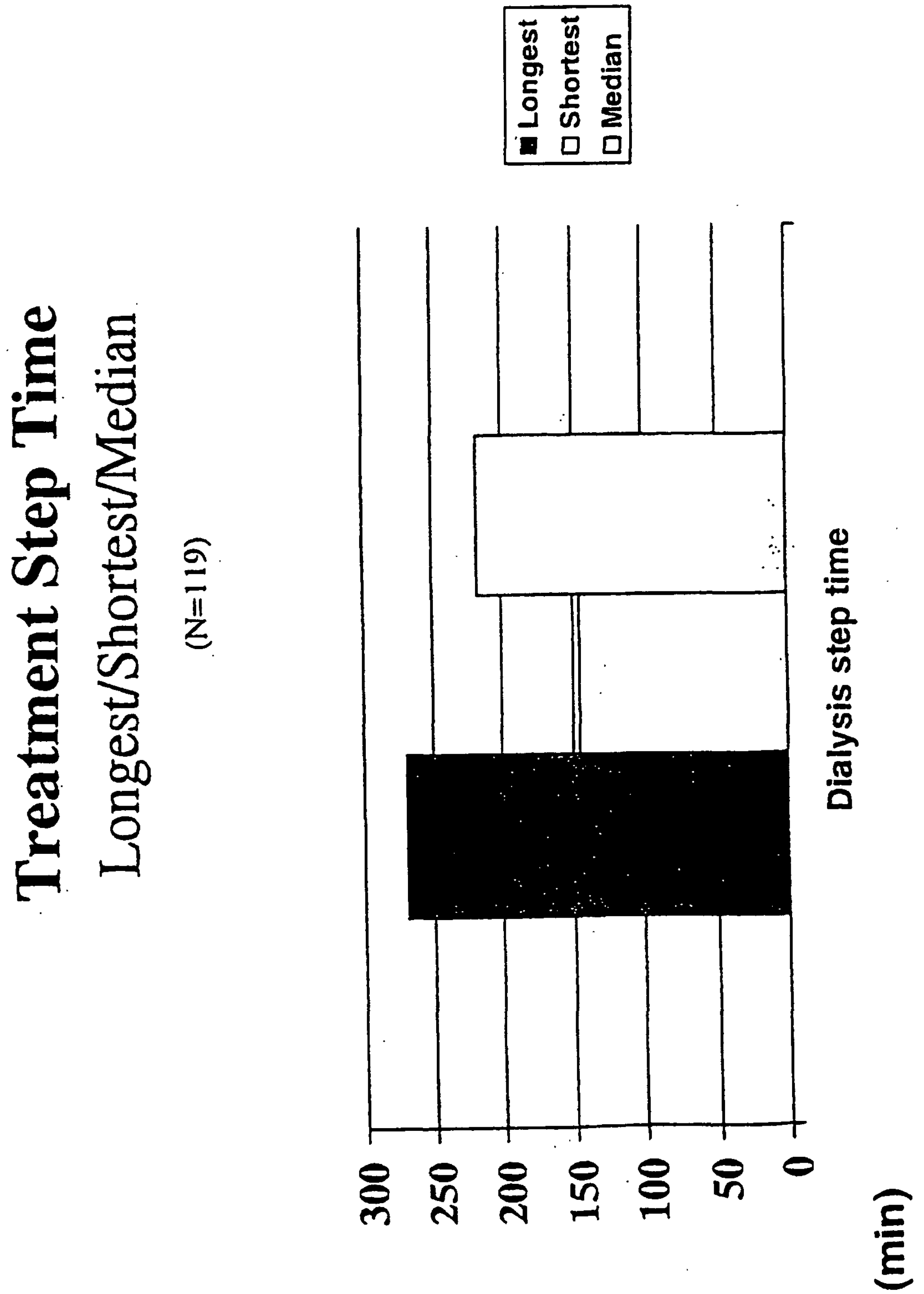

Treatment Step Time
Longest/Shortest/Median
(N=119)
Longest
Shortest
Median
300
250
200
150
100
50
0
(min)
Dialysis step time


Fig. 30

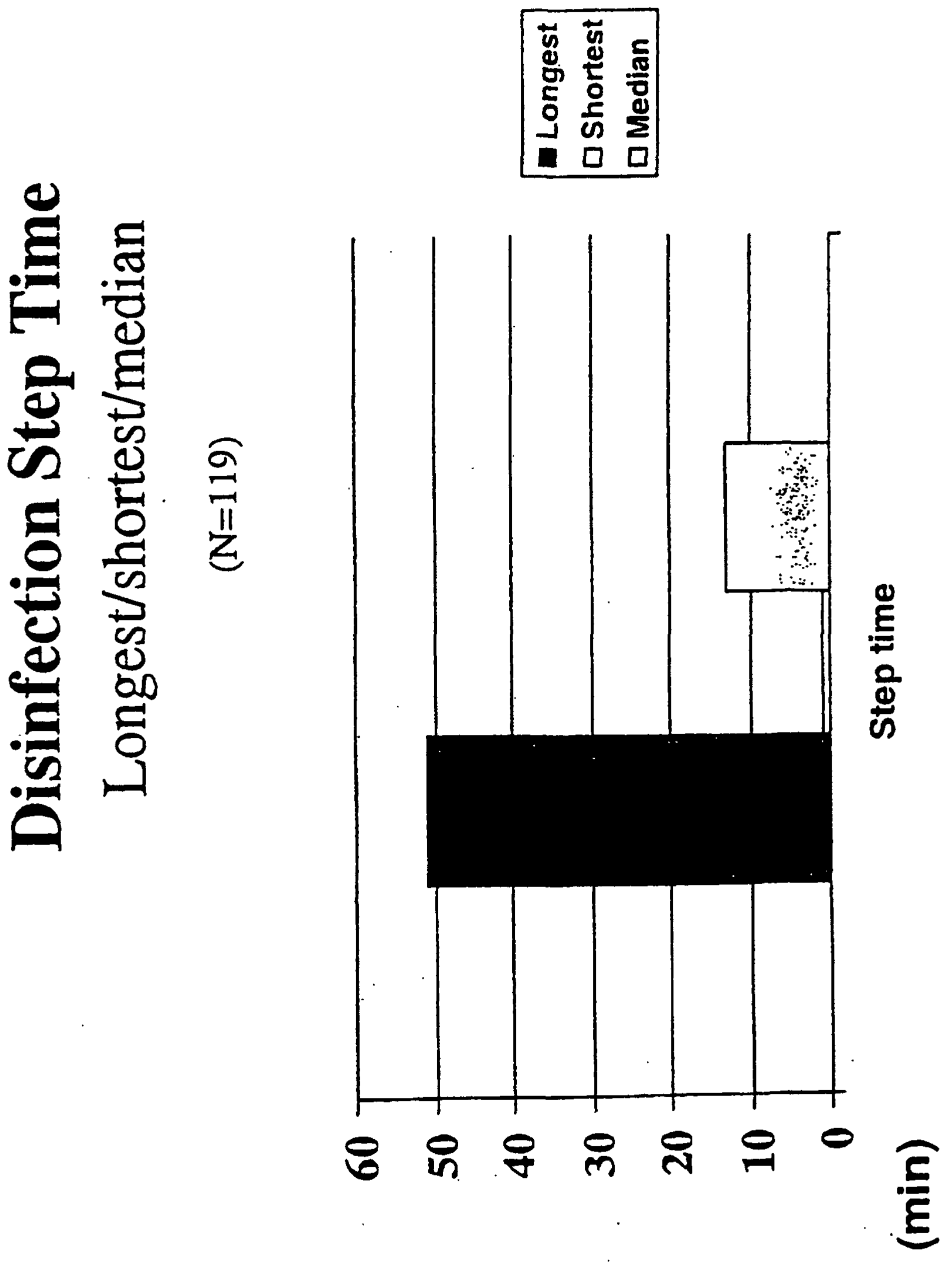
Disinfection Step Time
Longest/shortest/median
(N=119)
Longest
Shortest
Median
60
50
40
30
20
10
0
(min)
Step time

Fig. 31

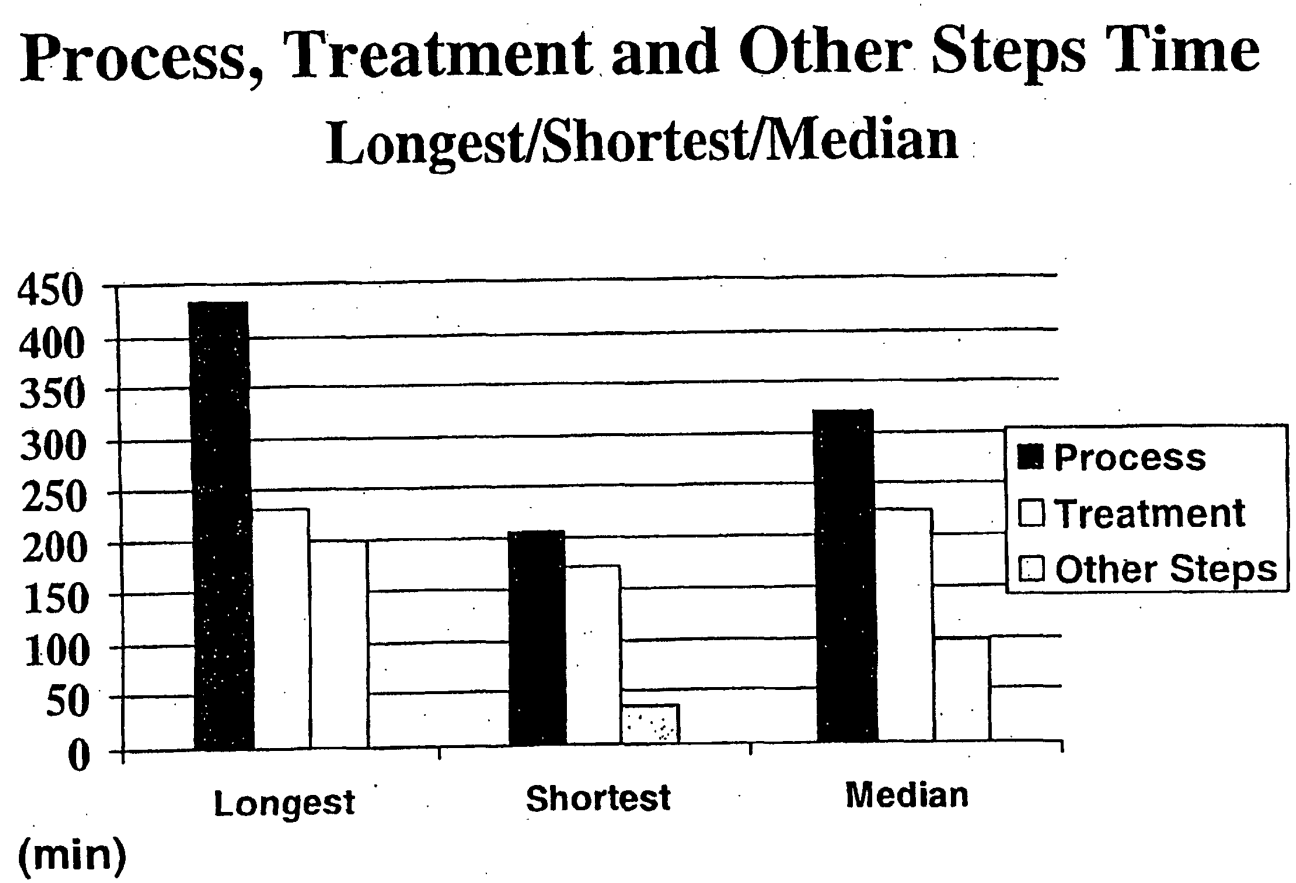
Process, Treatment and Other Steps Time
Longest/Shortest/Median
450
400
350
300
250
200
150
100
50
0
(min)
Longest
Shortest
Median
Process
Treatment
Other Steps

Fig. 32

# Total Process Staff Time

## Longest/shortest/median

(No Reuse)

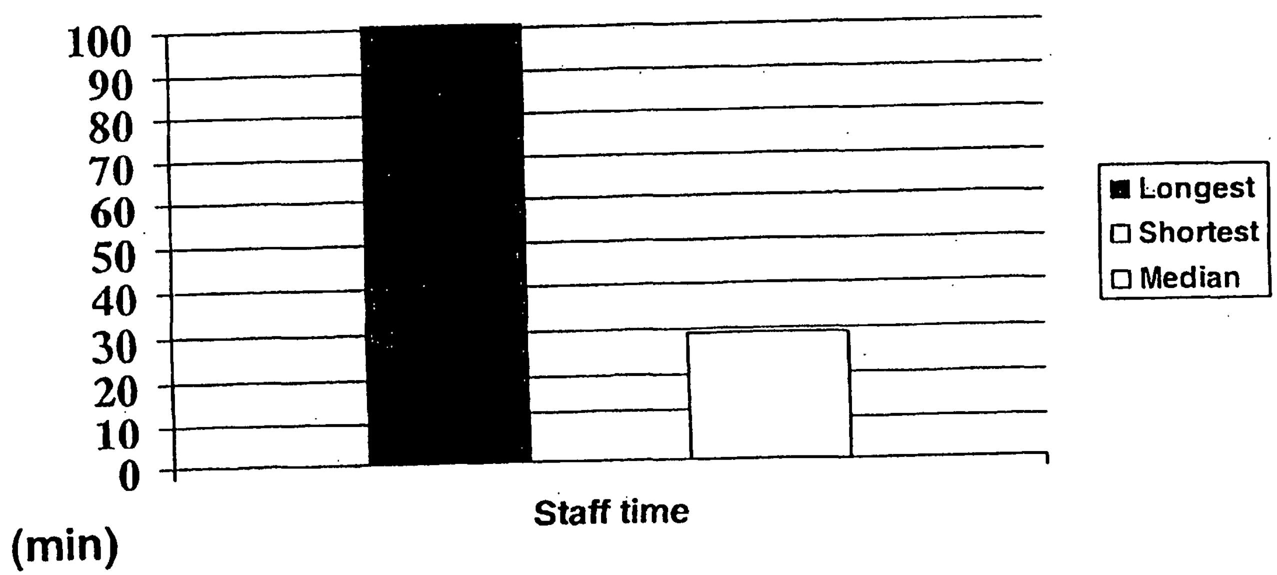


Fig. 33

# Staff Time Treatment Step

Longest/shortest/median

(N=119)

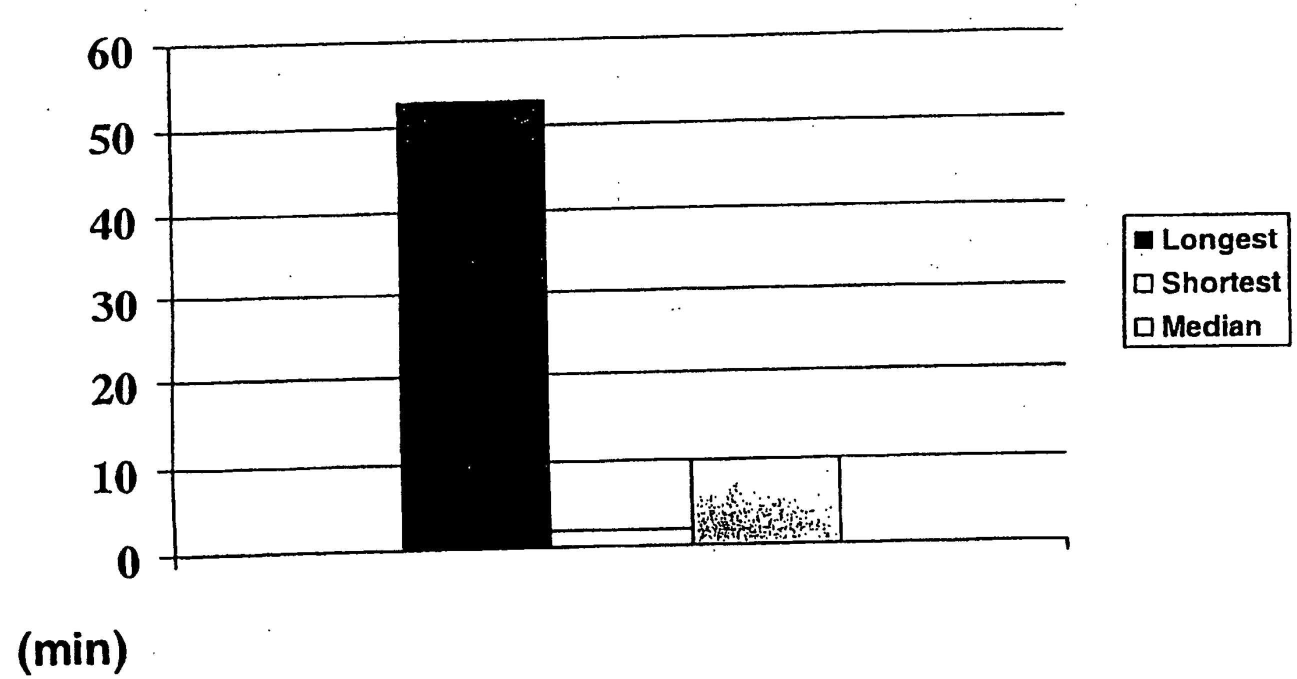


Fig. 34

## Staff Time

## Total Process, Treatment and Other Steps Time

### Longest/Shortest

(No Reuse)

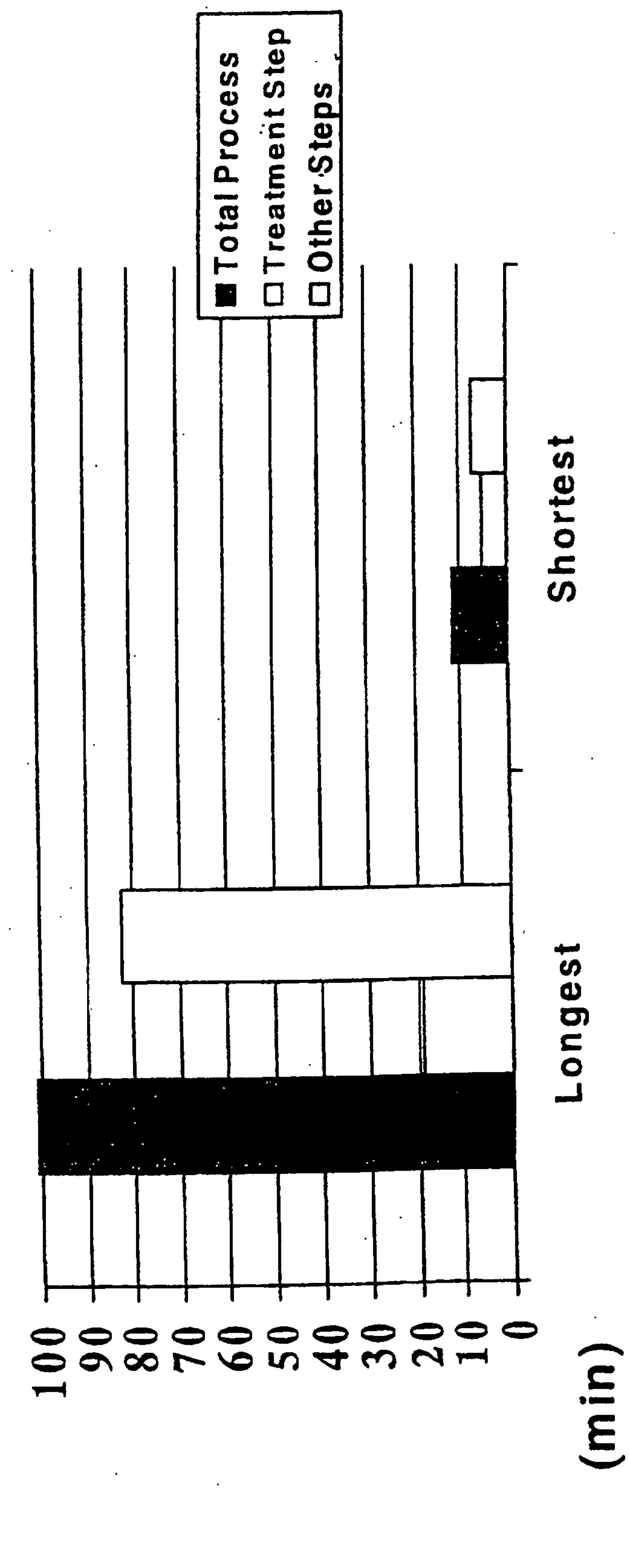


**Fig. 35**

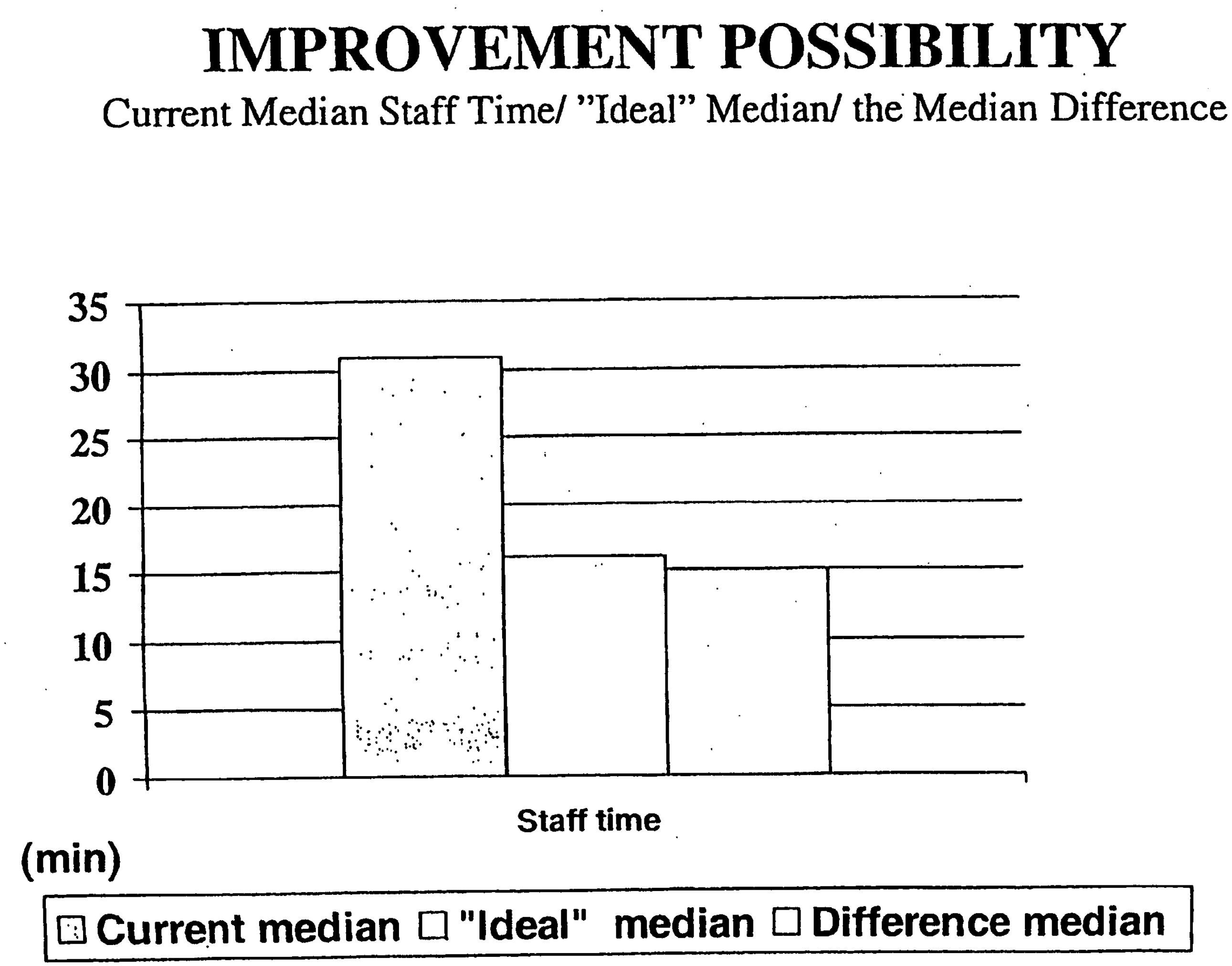
IMPROVEMENT POSSIBILITY
Current Median Staff Time/ "Ideal" Median/ the Median Difference
35
30
25
20
15
10
5
0
(min)
Staff time
Current median
"Ideal" median
Difference median

Fig. 36

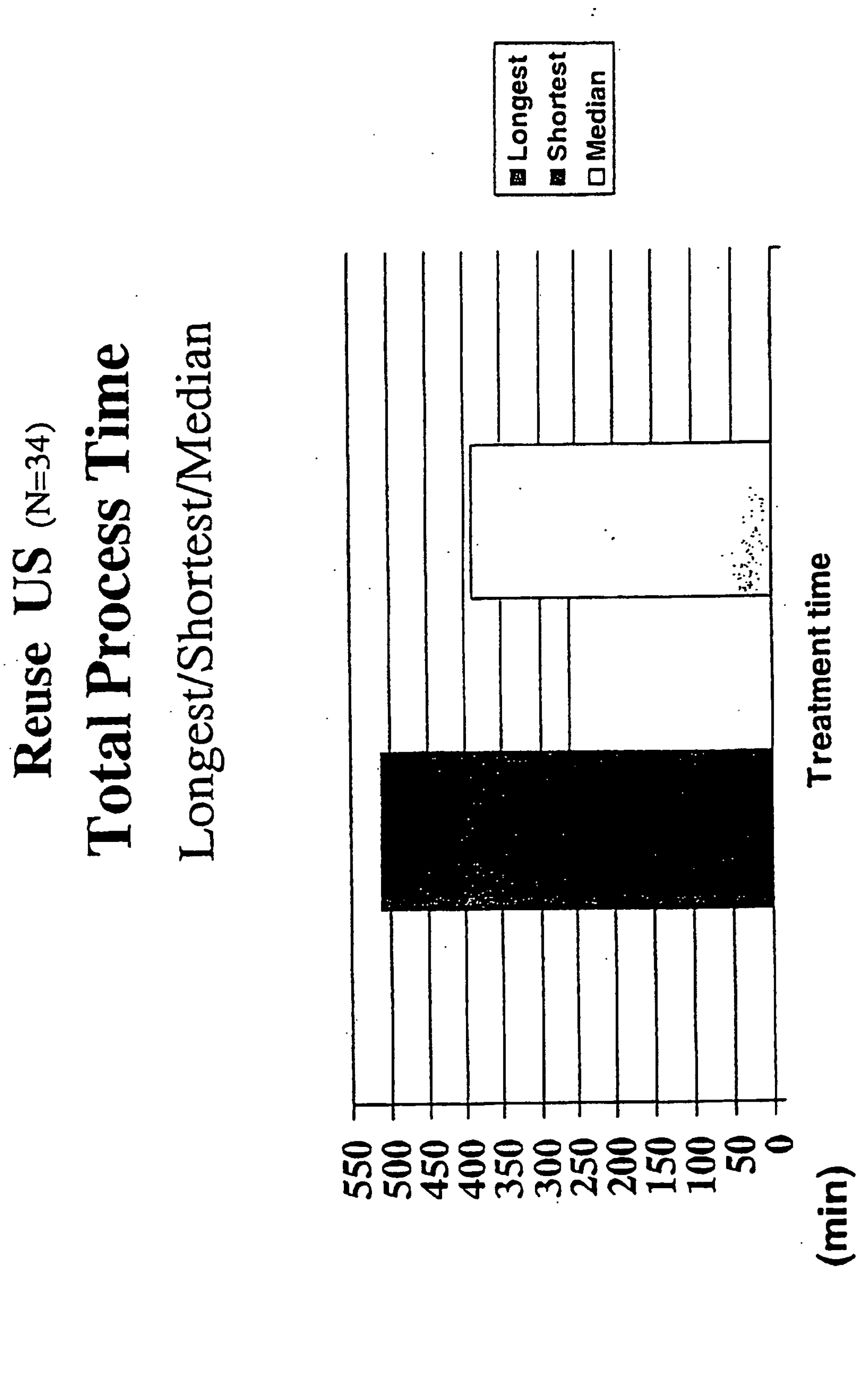

Reuse US (N=34)
Total Process Time
Longest/Shortest/Median
Longest
Shortest
Median
550
500
450
400
350
300
250
200
150
100
50
0
(min)
Treatment time


Fig. 37

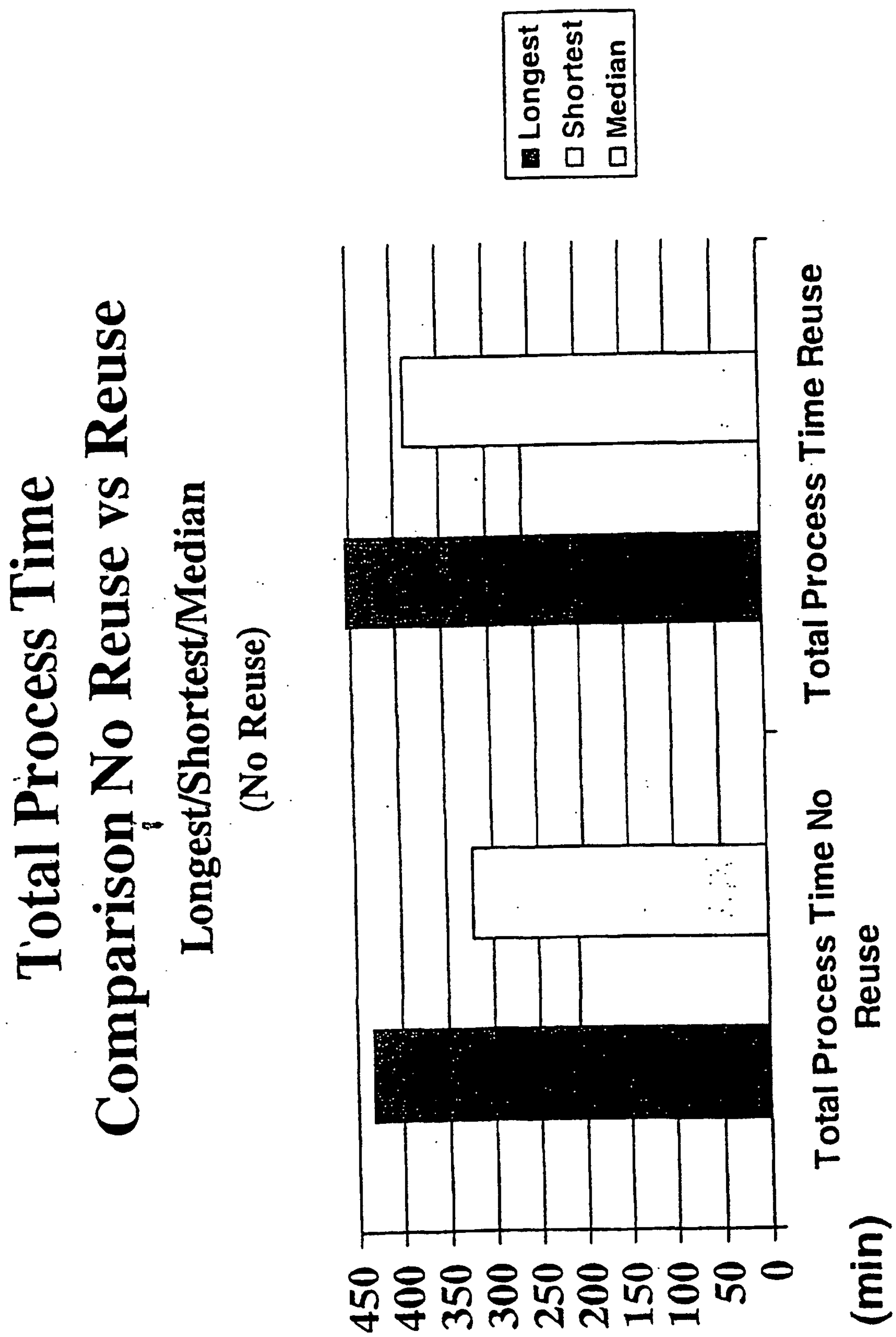
Total Process Time
Comparison No Reuse vs Reuse
Longest/Shortest/Median
(No Reuse)
Longest
Shortest
Median
450
400
350
300
250
200
150
100
50
0
(min)
Total Process Time No Reuse
Total Process Time Reuse

Fig. 38

Reuse US

**Median staff time reuse/ without the reuse step/no reuse**

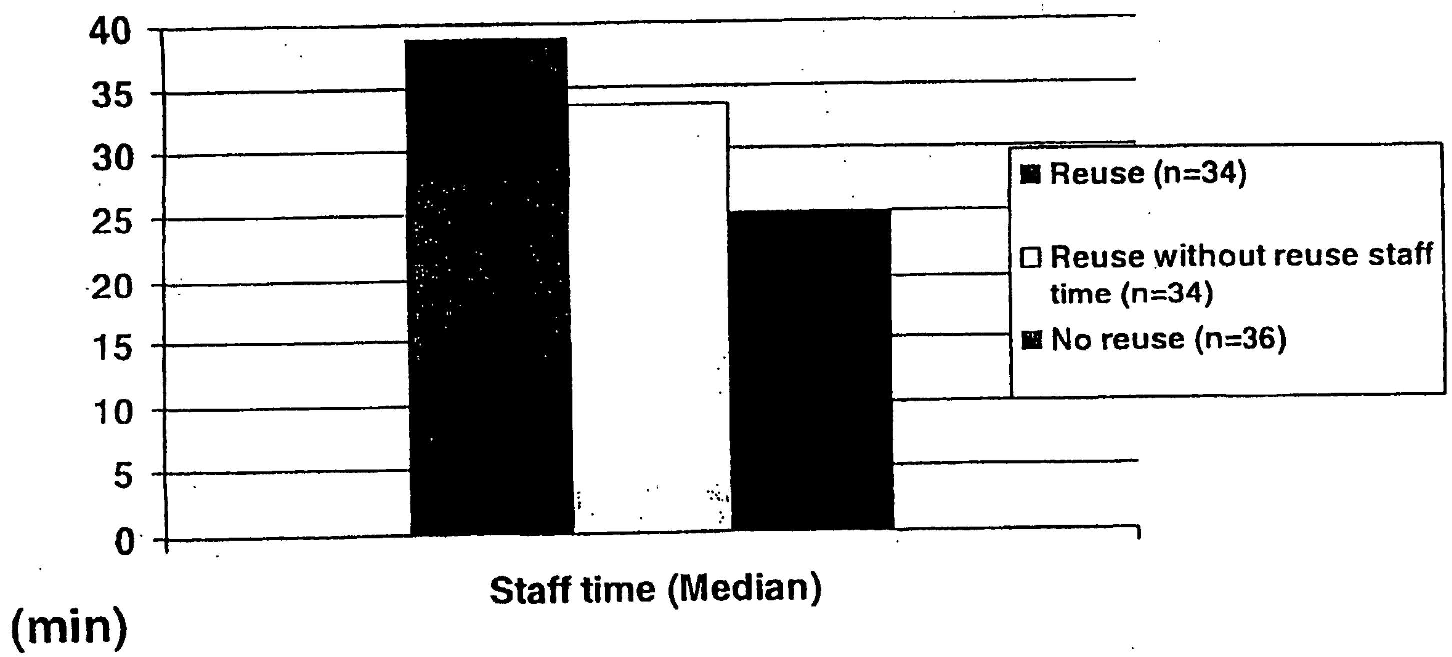


**Fig. 39**

US clinics

Median staff time reuse treatments without the reuse step/staff time no reuse treatments and the difference

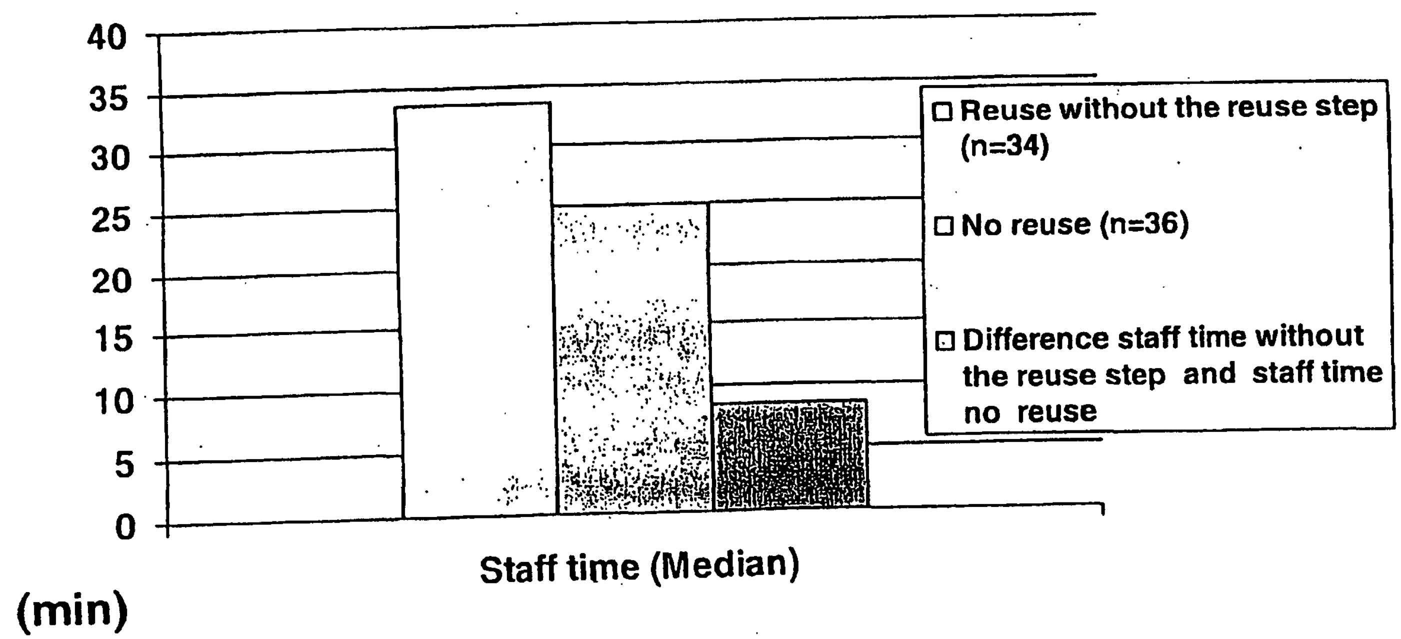


Fig. 40

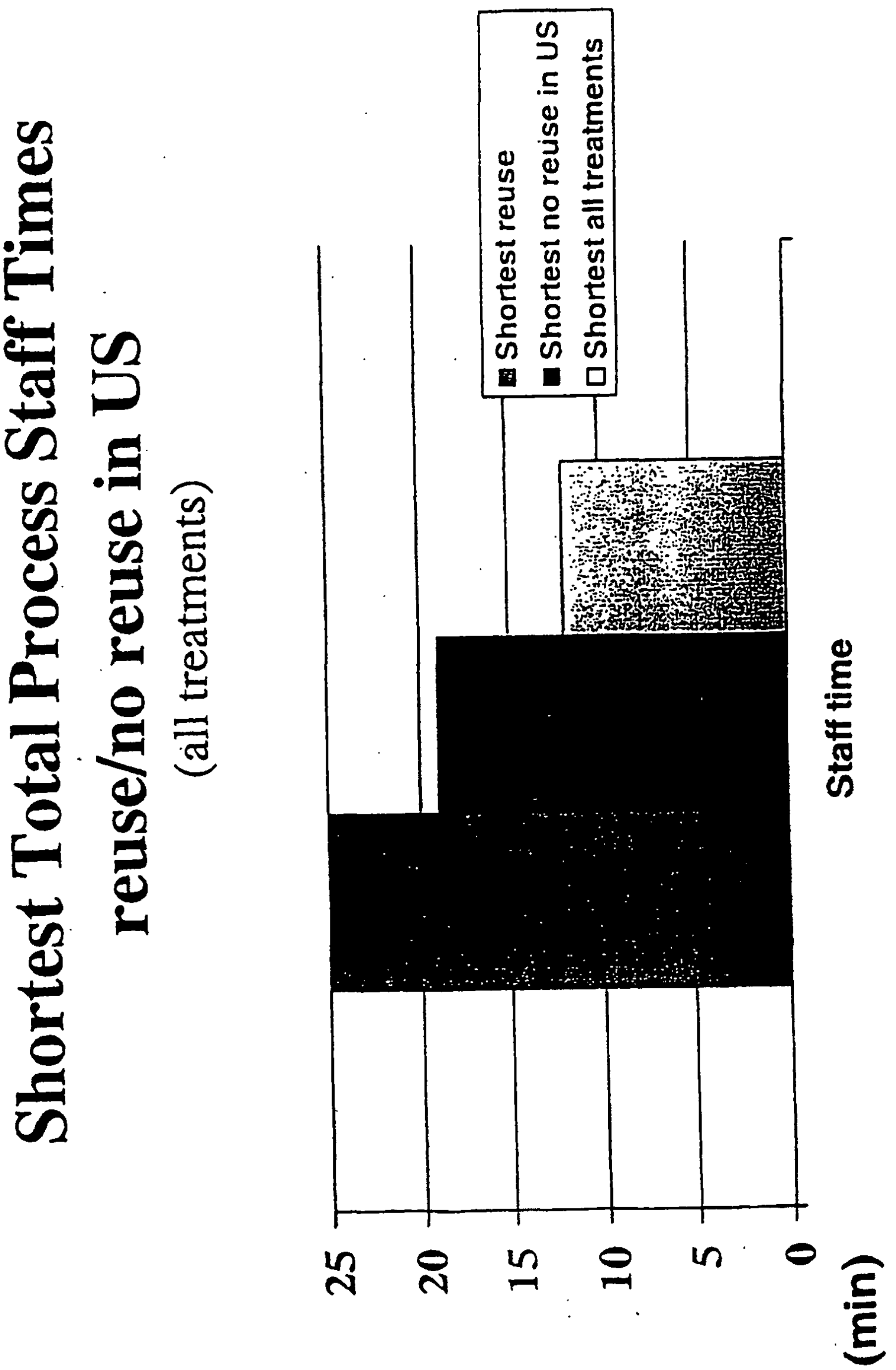
Shortest Total Process Staff Times
reuse/no reuse in US
(all treatments)
25
20
15
10
5
0
(min)
Staff time
Shortest reuse
Shortest no reuse in US
Shortest all treatments

Fig. 41

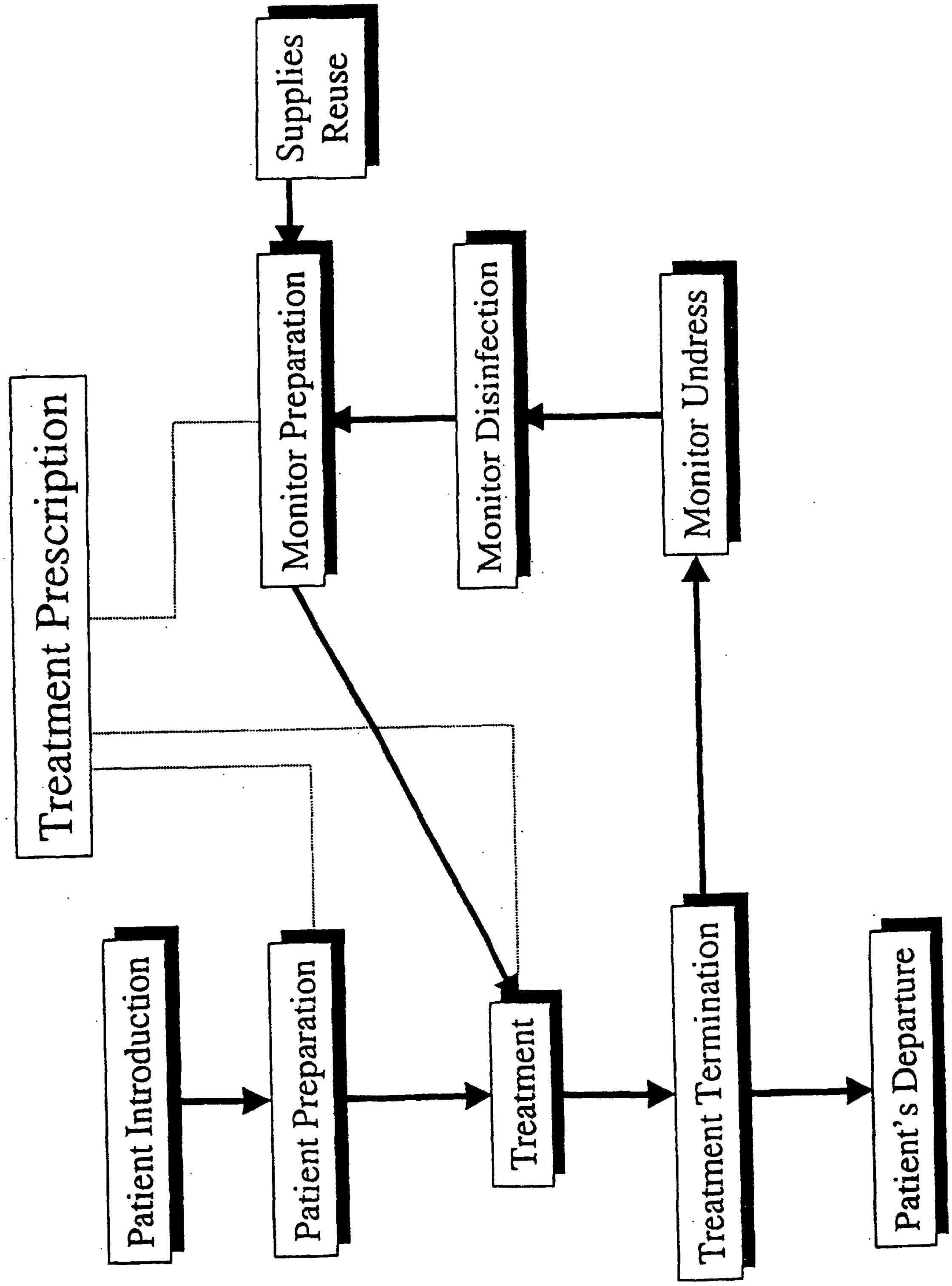
Treatment Prescription
Supplies Reuse
Monitor Preparation
Monitor Disinfection
Monitor Undress
Patient Introduction
Patient Preparation
Treatment
Treatment Termination
Patient's Departure

Fig. 42

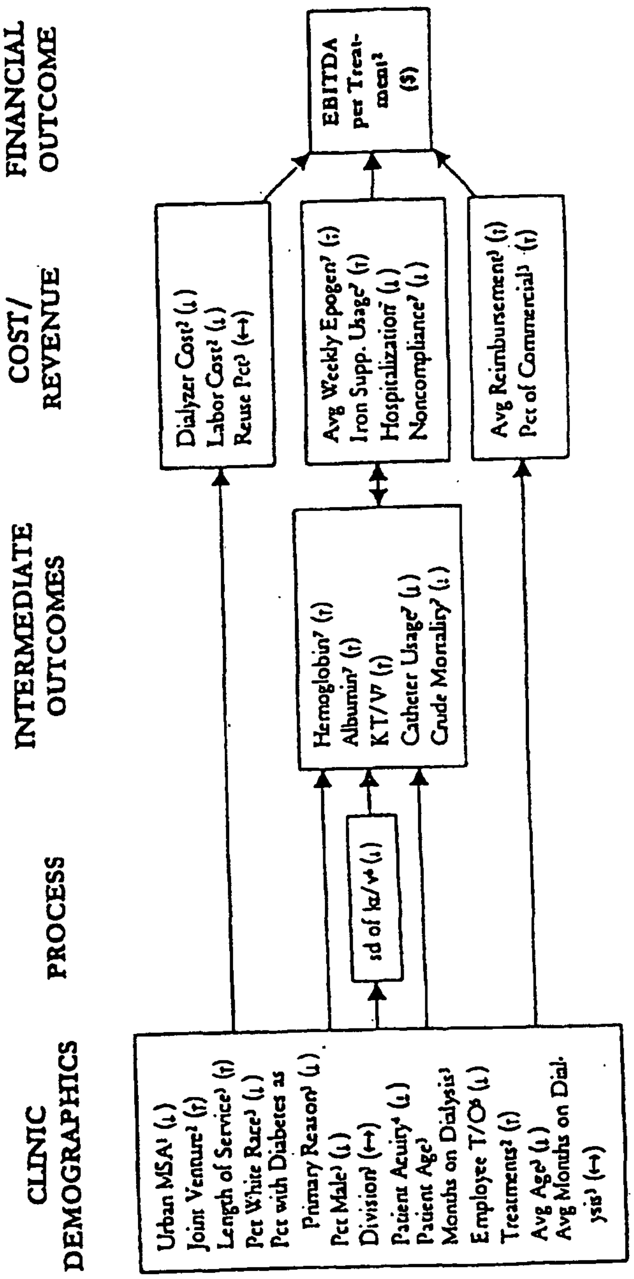
CLINIC DEMOGRAPHICS
PROCESS
INTERMEDIATE OUTCOMES
COST/ REVENUE
FINANCIAL OUTCOME
Urban MSA1 (↓)
Joint Venture2 (↑)
Length of Service2 (↑)
Pct White Race2 (↓)
Pct with Diabetes as Primary Reason2 (↓)
Pct Male2 (↓)
Division2 (↔)
Patient Acuity4 (↓)
Patient Age2
Months on Dialysis2
Employee T/O3 (↓)
Treatments2 (↑)
Avg Age2 (↓)
Avg Months on Dialysis3 (↔)
sd of kt/v4 (↓)
Hemoglobin7 (↑)
Albumin7 (↑)
KT/V7 (↑)
Catheter Usage7 (↓)
Crude Mortality7 (↓)
Dialyzer Cost2 (↓)
Labor Cost2 (↓)
Reuse Pct3 (↔)
Avg Weekly Epogen7 (↓)
Iron Supp. Usage7 (↑)
Hospitalization7 (↓)
Noncompliance7 (↓)
Avg Reimbursement3 (↑)
Pct of Commercial3 (↑)
EBITDA per Treatment2 ($)

Fig. 43

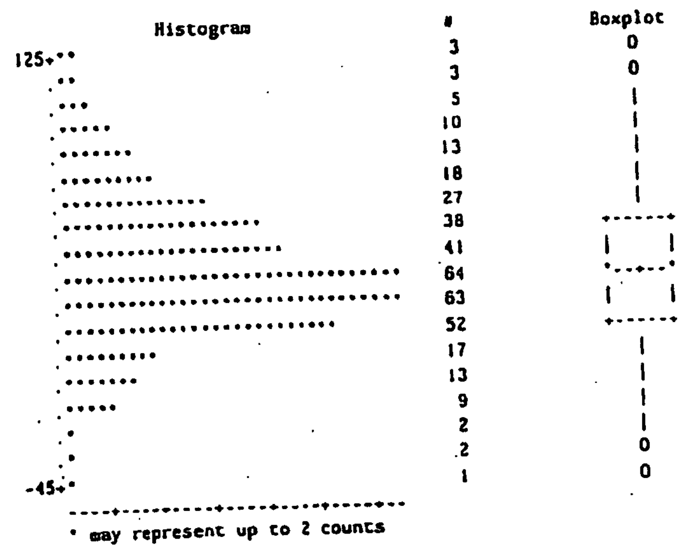


Histogram and Box and Whisker Plot of EBITDA per treatment

**Fig. 44**